(19) Europäisches Patentamt
European Patent Office
Office européen des brevets

(11) EP 1 623 721 A1

(12) # EUROPEAN PATENT APPLICATION
published in accordance with Art. 158(3) EPC

(43) Date of publication:
**08.02.2006 Bulletin 2006/06**

(21) Application number: **04730075.1**

(22) Date of filing: **28.04.2004**

(51) Int Cl.:
***A61K 45/00*** (1985.01) ***A61K 45/06*** (1985.01)
***A61K 31/499*** (2000.01) ***A61P 37/06*** (2000.01)
***A61P 37/08*** (2000.01) ***A61P 9/10*** (2000.01)
***A61P 35/00*** (2000.01) ***C07D 487/10*** (1985.01)

(86) International application number:
**PCT/JP2004/006197**

(87) International publication number:
**WO 2004/098638 (18.11.2004 Gazette 2004/47)**

(84) Designated Contracting States:
**AT BE BG CH CY CZ DE DK EE ES FI FR GB GR HU IE IT LI LU MC NL PL PT RO SE SI SK TR**

(30) Priority: **06.05.2003 JP 2003128193**

(71) Applicant: **ONO PHARMACEUTICAL CO., LTD.**
**Osaka-shi 541-8526 (JP)**

(72) Inventors:
- **SHIBAYAMA, Shiro,**
**c/o Ono Pharmaceutical Co., Ltd.**
**Tsukuba-shi,**
**Ibaraki 300-4247 (JP)**
- **SUGIYAMA, Tetsuya,**
**c/o Ono Pharmaceutical Co., Ltd**
**Tsukuba-shi,**
**Ibaraki 300-4247 (JP)**
- **SAGAWA, Kenji,**
**c/o Ono Pharmaceutical Co., Ltd.**
**Mishima-gun, Osaka 618-8585 (JP)**
- **KASANO, Miki,**
**c/o Ono Pharmaceutical Co., Ltd.**
**Tsukuba-shi,**
**Ibaraki 300-4247 (JP)**

(74) Representative: **Henkel, Feiler & Hänzel**
**Möhlstrasse 37**
**81675 München (DE)**

(54) **EFFECTOR CELL FUNCTION INHIBITOR**

(57) The present invention relates to a function inhibitor of an effector cell comprising a CCR5 antagonist. Since the function inhibitor of an effector cell comprising a CCR5 antagonist inhibits the function of effector cells which play an important role in formation of diseases and the like, it is effective for the prevention and/or treatment of, for example, a transplant rejection (e.g., rejection of a solid organ graft, rejection of islet cell transplantation in diabetes mellitus, graft-versus-host disease (GVHD), *etc.),* an autoimmune disease (e.g., arthritis, rheumatoid arthritis, multiple sclerosis, ulcerative colitis, *etc.*), an allergic disease (e.g., asthma, *etc.),* an ischemic disease (e.g., ischemia-reperfusion injury, *etc.*), cancer or a metastatic disease or the like.

EP 1 623 721 A1

```
SEQUENCE LISTING

<110> ONO PHARMACEUTICAL CO., LTD.

<120> FUNCTION INHIBITOR FOR EFFECTOR CELL

<130> ONF-4970PCT

<140> EP 04730075
<141> 2004-04-28

<150> PCT/JP2004/006197
<151> 2004-04-28

<150> JP2003-128193
<151> 2003-05-06

<160> 2

<170> PatentIn Ver. 2.1

<210> 1
<211> 37
<212> DNA
<213> Artificial Sequence

<220>
<223> Description of Artificial Sequence:Forword primer
      hCCR5Xbal

<400> 1
agctagtcta gatccgttcc cctacaagaa actctcc                          37

<210> 2
<211> 37
<212> DNA
<213> Artificial Sequence

<220>
<223> Description of Artificial Sequence:Revese primer
      hCCR5Xbal

<400> 2
agctagtcta gagtgcacaa ctctgactgg gtcacca                          37
```

## Description

TECHNICAL FIELD

The present invention relates to a function inhibitor of an effector cell comprising a CCR5 antagonist. More specifically, it relates to the use of a function inhibitor of an effector cell comprising a CCR5 antagonist for the prevention and/or treatment of a disease in which an effector cell is concerned, such as a transplant rejection, an autoimmune disease, an ischemic disease, an allergic disease, a cancer or a metastatic disease.

BACKGROUND ART

Currently, maintenance immunosuppressive therapy which uses an immune system, namely an agent capable of inhibiting activation of immune system cells, is mainly carried out for diseases of the transplantation field. In this therapy, a calcineurin inhibitor cyclosporin or tacrolimus (FK506) is used in combination with one or two or more immunosuppressant(s). As the immunosuppressants to be combined, a TOR (target of rapamycin) inhibitor sirolimus (rapamycin), a nonspecific anti-inflammatory agent corticosteroid, growth inhibitors azathioprine and mycophenolate mofetil and the like can be exemplified. In the multiple drug therapy which includes agents such as cyclosporin, tacrolimus and sirolimus, the one-year survival ratio of grafts is about 90%, but on the other hand, it is also a fact that they produce chronic rejection reactions and serious side effects. As the side effects produced by the administration of these agents, for example, side effects such as nephropathy, hypertension and hyperlipemia have been reported in the case of cyclosporin, and nephropathy, diabetes mellitus, neuropathy and the like in the case of tacrolimus, and hyperlipemia, thrombopenia and the like in the case of sirolimus. Since these side effects are serious ones, there is a demand for an immunosuppressant which can effect long-term survival of grafts and alleviate side effects, in comparison with the already present drugs. Taking such situations into consideration, attempts have been made on the development of neutralizing antibodies capable of inhibiting co-stimulation and function of adhesion molecule or the like, which are necessary for activating T cell that performs central role of the immune system. Since these neutralizing antibodies seem to specifically inhibit the process of T cell activation which is a cause of inducing organ injury, it is considered that they will become drugs having high safety in comparison with the already present drugs. However, since antibody preparations are not aimed at mass production, in addition to problems peculiar to biological preparations, e.g., a problem from the viewpoint of their supply, and expensiveness or the like, there are many problems to be resolved, for example, a problem in that their effects are attenuated by production of antibodies in the living body for the antibodies as the drugs.

In addition, anti-inflammatory agents and immune function-controlling agents are mainly used for the treatment of autoimmune diseases and allergic diseases. For example, non-steroidal anti-inflammatory drugs (NSAIDs) having cyclooxigenase (COX) inhibitory action, disease modifying anti-rheumatoid drugs (DMARDs), steroid drugs and the like are used in the case of rheumatoid arthritis. Though the drug therapy which uses these drugs is effective for controlling the inflammation itself, it has been suggested that those which have strong effects also show strong side effects, and this is merely a symptomatic therapy and cannot be used for the fundamental treatment of the disease. For example, when NSAIDs are applied to patients of rheumatoid arthritis, it has been shown that inflammation, pain and the like of joints are disappeared, but destruction of bones considered to be most serious case of rheumatoid arthritis advances without undergoing influence of the drugs. With the aim of avoiding such problems, protein preparations for anti-cytokine, such as an anti-TNF antibody and anti-IL-6 antibody, have also been developed and used, but the above-described problems of antibody preparations have not been dissolved. In the case of asthma on the other hand, when controller and reliever are included, inhalation or oral steroid preparations, sustained-release or short period acting theophylline preparations, long time or short time acting β-2 stimulants, anti-allergy preparations, anti-choline preparations and the like are used. It can be said that, for asthma patients, the inhalation steroid preparations are very excellent medicaments in view of the effect, and there are almost no serious cases in view of side effects. However, those which can be regarded as side effects of inhalations, such as infection of the mouth with *Candida* (fungus) and foreign matter feeling of the mouth, have been reported. Though these side effects are healed with the lapse of time when use of the drug is discontinued, the inhalation steroid preparations cannot be used during the period as a matter of course, and there are no substitutive drugs having both efficacy and safety.

At present, chemotherapy which uses alkylating agent, nitrosourea agent, metabolic antagonist, carcinostatic antibiotics and the like and radiotherapy are carried out for the treatment of cancer and metastasis. However, side effects such as nausea, vomiting, diarrhea, pyrexia, bone marrow inhibition, serious infection, liver function disorder, nephropathy, blood coagulation disorder and neuropathy have also been reported. It is known that avoidance from immunological surveillance of host is concerned in the proliferation and metastasis of cancer. On the contrary, it has been suggested that the host cell is also concerned in the proliferation and metastasis of cancer.

Because of the reasons described in the above, concern has been directed toward the development of new drugs which are excellent in terms of effects and have high safety, commonly in these diseases.

On the other hand, as another common point in these diseases, a point can be exemplified in which concern of T cell and myeloid cell in the formation, advance and/or continuation of the diseases has been suggested. That is, there is a possibility that an agent which can specifically inhibit activation of the T cell and myeloid cell can become a drug useful in these diseases.

As well as B cell, T cell is known as a main cell which controls the immune system. T cell is a lymphocyte concerned in the immune system, namely, among cellular immunity and humoral immunity, mainly in the cellular immunity, and is classified into several subclasses depending on its functions or its surface antigens. Expression of CD4 and CD8 molecules as surface antigens of T cell is used as an index showing the degree of differentiation of T cell in the thymus, and it is generally considered that CD4-positive T cell is mainly concerned in the assistance of antibody production and induction of various immune responses, while CD8-positive T cell mainly has cytotoxic activity. Among these CD4-positive T cell and CD8-positive T cell, those cells which did not receive antigen stimulation yet are called CD4-positive naive T cell and CD8-positive naive T cell, respectively, and these cells differentiate into T cell derived effector cells having various functions by receiving an antigen-specific activation signal and a co-stimulation signal from an antigen presenting cell. As such effector cells, Th1 cell and Th2 cell differentiated from the CD4-positive naive T cell and cytotoxic T cell (CTL) differentiated from the CD8-positive naive T cell are known. The assistance of antibody production, induction of various immune responses and cytotoxic activity generally known as functions of T cell are not those of naive T cells but represent functions of these effector cells.

In addition, the T cell expresses two or more surface antigens other than the CD4 and CD8 surface antigens. Also included in these surface antigens are a large number of those which are functioning as receptors that receive stimulation from an antigen presenting cell. It is known that there are certain chemokine receptors which perform a function, not only as receptors that mediate cell migration in response to chemokine, but also as receptors that receive stimulation from such an antigen presenting cell (cf *J. Immunol.,* 1996, Mar. 15; 156 (6): 2095-2103), and CCR5 is one of them, too.

In recent years, some experiments on animal models using CCR5 knockout mice, suggesting that a chemokine receptor CCR5 is taking an important role in transplant rejection, autoimmune diseases and the like, have been reported (cf. *Transplantation,* 2001, vol. 72, no. 7, pp. 1199-1205, *Diabetes,* 2002, vol. 51, no. 8, p. 2489-2495, *Journal of Virology,* 2003, vol. 77, no. 1, pp. 191 to 198, *Journal of Immunology,* 2000, vol. 164, no. 12, pp. 6303-6312). These reports which used knockout mice suggest concern of CCR5 in diseases, but there is no description or suggestion that a drug capable of inhibiting the action of CCR5 inhibited functions of the T cell and its effector cells.

In addition, regarding reports with human, there are reports in which risk of contracting diseases, survival period of grafts by transplantation and the like in human expressing inactive type CCR5 and in human expressing wild type CCR5 are compared (cf. *The Lancet,* 2001, vol. 357, pp. 1758-1761, *Arthritis & Rheumatism,* 1999, vol. 42, no. 5, pp. 989-992, *The Lancet,* 1999, vol. 3 54, pp. 1264-1265, *European Journal of Immunogenetics,* 2002, vol. 29, no. 6, pp. 525-528). However, these reports also suggest concern of CCR5 in diseases, but similar to the above-described reports of knockout mice, they do not show effect of a drug which inhibit the action of CCR5.

On the other hand, there is a description stating that a triazabispiro[5.5]undecane derivative compound represented by formula (X)

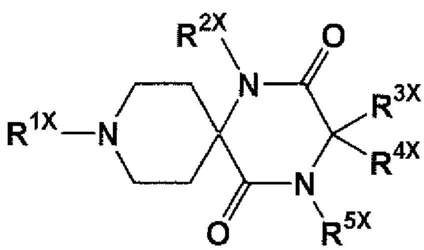

(X)

(in the formula, $R^{1X}$ represents formula (X-2)

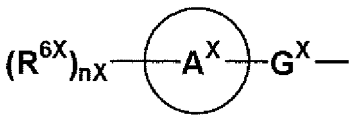

(X-2)

or formula (X-3)

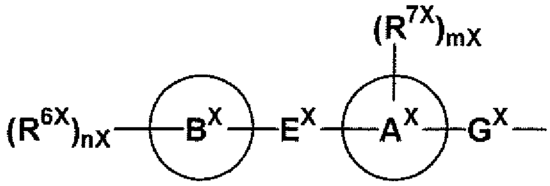

(X-3)

$R^{2X}$ represents alkyl, alkynyl or the like, $R^{3X}$ and $R^{4X}$ represents H, (substituted) alkyl or the like, or $R^{3X}$ and $R^{4X}$ are taken together to represent formula (X-4)

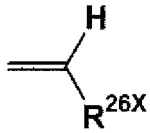

(X-4)

and $R^{5X}$ represents H or alkyl) is useful for the prevention and/or treatment of asthma, atopic dermatitis, nettle rash, allergic bronchopulmonary aspergillosis, allergic eosinophilic gastroenteritis, glomerulonephritis, nephropathy, hepatitis, arthritis, rheumatoid arthritis, psoriasis, rhinitis, conjunctivitis, suppression of ischemia-reperfusion injury, multiple sclerosis, ulcerative colitis, acute respiratory distress syndrome, shock accompanied by bacterial infection, diabetes mellitus, treatment of autoimmune disease, transplanted organ rejection reaction, immunosuppression, metastasis prevention, or acquired immunodeficiency syndrome, effected by controlling mutual action of chemokine/chemokine receptor (cf. WO 02/74770).

However, there is no description stating that the compound represented by general formula (X) showed an effect on the function of effector cells and function of T cells, and what is more, there is no evidence that this is effective for transplant rejection, autoimmune disease, allergic disease, ischemic disease, or cancer or metastasis or the like.

DISCLOSURE OF THE INVENTION

Accordingly, the object of the present invention is to provide a drug which specifically inhibits function of effector cell, is useful for the prevention and/or treatment of a disease in which effector cell is concerned in the formation, advance and/or continuation of the disease, such as a transplant rejection, an autoimmune disease, an ischemic disease, an allergic disease, a cancer or a metastasis, and can also become a drug having high safety.

As a result of intensive studies carried out in order to solve the above-described problems, the present inventors have found that an antagonist of a chemokine receptor CCR5 inhibits the function of effector cells, and thereby accomplished the present invention.

The present invention relates to:

1. a function inhibitor of an effector cell, which comprises a CCR5 antagonist,
2. the function inhibitor of an effector cell according to the above 1, wherein the function is cell migration, cell proliferation or cell activation,
3. the function inhibitor of an effector cell according to the above 1, wherein the effector cell is a CCR5-positive effector cell,
4. the function inhibitor of an effector cell according to the above 1, which is an agent for prevention and/or treatment of a disease caused by effector cell function,
5. the function inhibitor of an effector cell according to the above 1, which is an agent for prevention and/or treatment of a T cell-mediated disease,
6. the function inhibitor of an effector cell according to the above 1, which is an agent for prevention and/or treatment of a myeloid cell-mediated disease,
7. the function inhibitor of an effector cell according to the above 5, wherein the T cell-mediated disease is transplant rejection, autoimmune disease, allergic disease or ischemic disease;
8. the function inhibitor of an effector cell according to the above 6, wherein the myeloid cell-mediated disease is cancer or cancer metastasis,
9. the function inhibitor of an effector cell according to the above 1, wherein the CCR5 antagonist is a non-peptide substance,
10. the function inhibitor of an effector cell according to the above 1, wherein the CCR5 antagonist is a compound of formula (I)

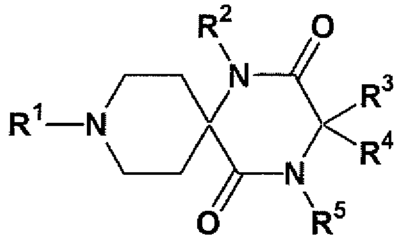

(I)

wherein $R^1$ represents (1) a hydrogen atom, (2) C1-18 alkyl, (3) C2-18 alkenyl, (4) C2-18 alkynyl, (5) $-COR^6$, (6) $-CONR^7R^8$, (7) $-COOR^9$, (8) $-SO_2R^{10}$, (9) $-COCOOR^{11}$, (10) $-CONR^{12}COR^{13}$, (11) Cyc1 or (12) C1-18 alkyl, C2-18 alkenyl or C2-18 alkynyl substituted with 1-5 substituent(s) selected from (a) halogen, (b) $-CONR^7R^8$, (c) $-COOR^9$, (d) $-OR^{14}$, (e) $-SR^{15}$, (f) $-NR^{16}R^{17}$, (g) $-NR^{18}COR^{19}$, (h) $-SO_2NR^{20}R^{21}$, (i) $-OCOR^{22}$, (j) $-NR^{23}SO_2R^{24}$, (k) $-NR^{25}COOR^{26}$, (1) $-NR^{27}CONR^{28}R^{29}$, (m) Cyc1, (n) keto and (o) $-N(SO_2R^{24})_2$;

$R^6$-$R^9$, $R^{11}$-$R^{21}$, $R^{23}$, $R^{25}$ and $R^{27}$-$R^{29}$ each independently represents (1) a hydrogen atom, (2) C1-8 alkyl, (3) C2-8 alkenyl, (4) C2-8 alkynyl, (5) Cycl or (6) C1-8 alkyl, C2-8 alkenyl or C2-8 alkynyl substituted with 1-5 substituent(s) selected from (a) Cycl, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}COR^{38}$, (i) $-NR^{39}SO_2R^{40}$ and (j) $-N(SO_2R^{40})_2$, or

$R^7$ and $R^8$, $R^{20}$ and $R^{21}$, or $R^{28}$ and $R^{29}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{195}$-(C2-6 alkylene)-, wherein $R^{195}$ is a hydrogen atom, C1-8 alkyl, phenyl, or C1-8 alkyl substituted with phenyl;

$R^{10}$, $R^{22}$, $R^{24}$ and $R^{26}$ each independently represents (1) C1-8 alkyl, (2) C2-8 alkenyl, (3) C2-8 alkynyl, (4) Cycl or (5) C1-8 alkyl, C2-8 alkenyl or C2-8 alkynyl substituted with 1-5 substituent(s) selected from (a) Cycl, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}COR^{38}$, (i) $-NR^{39}SO_2R^{40}$ and (j) $-N(SO_2R^{40})_2$;

$R^{30}$-$R^{37}$ and $R^{39}$ each independently represents a hydrogen atom, C1-8 alkyl, Cycl or C1-8 alkyl substituted with Cycl, or

$R^{35}$ and $R^{36}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{196}$-(C2-6 alkylene)-, wherein $R^{196}$ represents a hydrogen atom, C1-8 alkyl, phenyl or C1-8 alkyl substituted with phenyl;

$R^{38}$ and $R^{40}$ each independently represents C1-8 alkyl, Cyc1 or C1-8 alkyl substituted with Cycl;

Cyc1 represents a C3-15 mono-, bi- or tri-(fused or spiro)carbocyclic ring or a 3-15 membered mono-, bi- or tri-(fused or spiro)cyclic hetero ring containing 1-4 nitrogen atom(s), 1-3 oxygen atom(s) and/or 1-3 sulfur atom(s), and Cyc1 may be substituted with 1-5 of $R^{51}$;

$R^{51}$ represents (l) C1-8 alkyl, (2) C2-8 alkenyl, (3) C2-8 alkynyl, (4) halogen, (5) nitro, (6) trifluoromethyl, (7) trifluoromethoxy, (8) nitrile, (9) keto, (10) Cyc2, (11) $-OR^{52}$, (12) $-SR^{53}$, (13) $-NR^{54}R^{55}$, (14) $-COOR^{56}$, (15) $-CONR^{57}R^{58}$, (16) $-NR^{59}COR^{60}$, (17) $-SO_2NR^{61}R^{62}$, (18) $-OCOR^{63}$, (19) $-NR^{64}SO_2R^{65}$, (20) $-NR^{66}COOR^{67}$, (21) $-NR^{68}CONR^{69}R^{70}$, (22) $-B(OR^{71})_2$, (23) $-SO_2R^{72}$, (24) $-N(SO_2R^{72})_2$, or (25) C1-8 alkyl, C2-8 alkenyl or C2-8 alkynyl substituted with 1-5 substituent(s) selected from (a) halogen, (b) Cyc2, (c) $-OR^{52}$, (d) $-SR^{53}$, (e) $-NR^{54}R^{55}$, (f) $-COOR^{56}$, (g) $-CONR^{57}R^{58}$, (h) $-NR^{59}COR^{60}$, (i) $-SO_2NR^{61}R^{62}$, (j) $-OCOR^{63}$, (k) $-NR^{64}SO_2R^{65}$, (1) $-NR^{66}COOR^{67}$, (m) $-NR^{68}CONR^{69}R^{70}$, (n) $-B(OR^{71})_2$, (o) $-SO_2R^{72}$ and (p) $-N(SO_2R^{72})_2$;

$R^{52}$-$R^{62}$, $R^{64}$, $R^{66}$ and $R^{68}$-$R^{71}$ each independently represents (1) a hydrogen atom, (2) C1-8 alkyl, (3) C2-8 alkenyl, (4) C2-8 alkynyl, (5) Cyc2 or (6) C1-8 alkyl, C2-8 alkenyl or C2-8 alkynyl substituted with Cyc2, $-OR^{73}$, $-COOR^{74}$ or $-NR^{75}R^{76}$, or

$R^{57}$ and $R^{58}$ $R^{61}$ and $R^{62}$, or $R^{69}$ and $R^{70}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{197}$-(C2-6 alkylene)-, wherein $R^{197}$ represents a hydrogen atom, C1-8 alkyl, phenyl or C1-8 alkyl substituted with phenyl;

$R^{63}$, $R^{65}$, $R^{67}$ and $R^{72}$ each independently represents (1) C1-8 alkyl, (2) C2-8 alkenyl, (3) C2-8 alkynyl, (4) Cyc2 or (5) C1-8 alkyl, C2-8 alkenyl or C2-8 alkynyl substituted with Cyc2, $-OR^{73}$, $-COOR^{74}$ or $-NR^{75}R^{76}$;

$R^{73}$-$R^{76}$ each independently represents a hydrogen atom, C1-8 alkyl, Cyc2 or C1-8 alkyl substituted with Cyc2;

Cyc2 has the same meaning as Cyc1, and Cyc2 may be substituted with 1-5 of $R^{77}$;

$R^{77}$ represents (1) C1-8 alkyl, (2) halogen, (3) nitro, (4) trifluoromethyl, (5) trifluoromethoxy, (6) nitrile, (7) $-OR^{78}$, (8) $-NR^{79}R^{80}$, (9) $-COOR^{81}$, (10) $-SR^{82}$, (11) $-CONR^{83}R^{84}$, (12) C2-8 alkenyl, (13) C2-8 alkynyl, (14) keto, (15) Cyc6, (16) $-NR^{161}COR^{162}$, (17) $-SO_2NR^{163}R^{164}$, (18) $-OCOR^{165}$, (19) $-NR^{166}SO_2R^{167}$, (20) $-NR^{168}COOR^{169}$, (21) $-NR^{170}CONR^{171}R^{172}$, (22) $-SO_2R^{173}$, (23) $-N(SO_2R^{167})_2$ or (24) C1-8 alkyl, C2-8 alkenyl or C2-8 alkynyl substituted with 1-5 substituent(s) selected from (a) halogen, (b) $-OR^{78}$, (c) $-NR^{79}R^{80}$, (d) $-COOR^{81}$, (e) $-SR^{82}$, (f) $-CONR^{83}R^{84}$, (g) keto, (h) Cyc6, (i) $-NR^{161}COR^{162}$, (j) $-SO_2NR^{163}R^{164}$, (k) $-OCOR^{165}$, (1) $-NR^{166}SO_2R^{167}$,

(m) $-NR^{168}COOR^{169}$, (n) $-NR^{170}CONR^{171}R^{172}$, (o) $-SO_2R^{173}$, and (p) $-N(SO_2R^{167})_2$;

$R^{78}$-$R^{84}$, $R^{161}$-$R^{164}$, $R^{166}$, $R^{168}$ and $R^{170}$-$R^{172}$ each independently represents (a) a hydrogen atom, (b) C1-8 alkyl, (c) C2-8 alkenyl, (d) C2-8 alkynyl, (e) Cyc6, (f) C1-8 alkyl, C2-8 alkenyl or C2-8 alkynyl substituted with Cyc6, $-OR^{174}$, $-COOR^{175}$, $-NR^{176}R^{177}$ or $-CONR^{178}R^{179}$, or

$R^{83}$ and $R^{84}$, $R^{163}$ and $R^{164}$, or $R^{171}$ and $R^{172}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{198}$-(C2-6 alkylene)-, wherein $R^{198}$ represents a hydrogen atom, C1-8 alkyl, phenyl or C1-8 alkyl substituted with phenyl;

$R^{165}$, $R^{167}$, $R^{169}$ and $R^{173}$ each independently represents (a) C1-8 alkyl, (b) C2-8 alkenyl, (c) C2-8 alkynyl, (d) Cyc6 or (e) C1-8 alkyl, C2-8 alkenyl or C2-8 alkynyl substituted with Cyc6, $-OR^{174}$, $-COOR^{175}$, $-NR^{176}R^{177}$ or $-CONR^{178}R^{179}$;

$R^{174}$-$R^{177}$ each independently represents (1) a hydrogen atom, (2) C1-8 alkyl, (3) Cyc6 or (4) C1-8 alkyl substituted with Cyc6, or

$R^{178}$ and $R^{179}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{199}$-(C2-6 alkylene)-, wherein $R^{199}$ represents a hydrogen atom, C1-8 alkyl, phenyl or C1-8 alkyl substituted with phenyl;

Cyc6 represents a C3-8 mono-carbocyclic ring or a 3-8 membered monocyclic hetero ring containing 1-4 nitrogen atom(s), 1-2 oxygen atom(s) and/or 1-2 sulfur atom(s), with the proviso that, Cyc6 may be substituted with 1-5 of $R^{180}$;

$R^{180}$ represents (1) C1-8 alkyl, (2) halogen, (3) nitro, (4) trifluoromethyl, (5) trifluoromethoxy, (6) nitrile, (7) $-OR^{181}$, (8) $-NR^{182}R^{183}$, (9) $-COOR^{184}$, (10) $-SR^{185}$ or (11) $-CONR^{186}R^{187}$;

$R^{181}$-$R^{187}$ each independently represents (1) a hydrogen atom, (2) C1-8 alkyl, (3) phenyl or (4) C1-8 alkyl substituted with phenyl, or

$R^{182}$ and $R^{183}$, or $R^{186}$ and $R^{187}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{200}$-(C2-6 alkylene)-, wherein $R^{200}$ represents a hydrogen atom, C1-8 alkyl, phenyl, C1-8 alkyl substituted with phenyl;

$R^2$ represents (1) a hydrogen atom, (2) C1-8 alkyl, (3) C2-8 alkenyl, (4) C2-8 alkynyl, (5) $-OR^{90}$, (6) Cyc3 or (7) C1-8 alkyl, C2-8 alkenyl or C2-8 alkynyl substituted with 1-5 substituent(s) selected from (a) halogen, (b) $-OR^{90}$, (c) $-SR^{91}$, (d) $-NR^{92}R^{93}$, (e) $-COOR^{94}$, (f) $-CONR^{95}R^{96}$, (g) $-NR^{97}COR^{98}$, (h) $-SO_2NR^{99}R^{100}$, (i) $-OCOR^{101}$, (j) $-NR^{102}SO_2R^{103}$, (k) $-NR^{104}COOR^{105}$, (1) $-NR^{106}CONR^{107}R^{108}$, (m) Cyc3, (n) keto and (o) $-N(SO_2R^{103})_2$;

$R^{90}$-$R^{100}$, $R^{102}$ , $R^{104}$ and $R^{106}$-$R^{108}$ each independently represents (1) a hydrogen atom, (2) C1-8 alkyl, (3) C2-8 alkenyl, (4) C2-8 alkynyl, (5) Cyc3 or (6) C1-8 alkyl, C2-8 alkenyl or C2-8 alkynyl substituted with Cyc3, or

$R^{95}$ and $R^{96}$, $R^{99}$ and $R^{100}$, or $R^{107}$ and $R^{108}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{201}$-(C2-6 alkylene)-, wherein $R^{201}$ is a hydrogen atom, C1-8 alkyl, phenyl or C1-8 alkyl substituted with phenyl;

$R^{101}$, $R^{103}$ and $R^{105}$ are each independently (1) C1-8 alkyl, (2) C2-8 alkenyl, (3) C2-8 alkynyl or (4) Cyc3, or C1-8 alkyl, C2-8 alkenyl or C2-8 alkynyl substituted with Cyc3;

Cyc3 has the same meaning as Cyc1, and Cyc3 may be substituted with 1-5 of $R^{109}$;

$R^{109}$ has the same meaning as $R^{51}$;

$R^3$ and $R^4$ each independently represents (1) a hydrogen atom, (2) C1-8 alkyl, (3) C2-8 alkenyl, (4) C2-8 alkynyl, (5) $-COOR^{120}$, (6) $-CONR^{121}R^{122}$, (7) Cyc4 or (8) C1-8 alkyl, C2-8 alkenyl or C2-8 alkynyl substituted with 1-5 substituent(s) selected from (a) halogen, (b) nitrile, (c) Cyc4, (d) $-COOR^{120}$, (e) $-CONR^{121}R^{122}$, (f) $-OR^{123}$, (g) $-SR^{124}$, (h) $-NR^{125}R^{126}$, (i) $-NR^{127}COR^{128}$, (j) $-SO_2NR^{129}R^{130}$, (k) $-OCOR^{131}$, (1) $-NR^{132}SO_2R^{133}$, (m) $-NR^{134}COOR^{135}$, (n) $-NR^{136}CONR^{137}R^{138}$, (O)-S-$SR^{139}$, (p) -NHC(=NH)$NHR^{140}$, (q) keto, (r) $-NR^{145}CONR^{146}COR^{147}$ and (s) $-N(SO_2R^{133})_2$;

$R^{120}$-$R^{130}$, $R^{132}$, $R^{134}$, $R^{136}$ -$R^{138}$, $R^{145}$ and $R^{146}$ each independently represents (1) a hydrogen atom, (2) C1-8 alkyl, (3) C2-8 alkenyl, (4) C2-8 alkynyl, (5) Cyc4 or (6) C1-8 alkyl, C2-8 alkenyl or C2-8 alkynyl substituted with Cyc4, halogen,$-OR^{148}$, $-SR^{149}$, $-COOR^{150}$ or $-NHCOR^{141}$, or

$R^{121}$ and $R^{122}$, $R^{129}$ and $R^{130}$ or $R^{137}$ and $R^{138}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{201}$-(C2-6 alkylene)-, wherein $R^{201}$ represents a hydrogen atom, C1-8 alkyl, phenyl, C1-8 alkyl substituted with phenyl;

$R^{131}$, $R^{133}$, $R^{135}$, $R^{139}$ and $R^{147}$ each independently represents (1) C1-8 alkyl, (2) C2-8 alkenyl, (3) C2-8 alkynyl, (4) Cyc4 or (5) C1-8 alkyl, C2-8 alkenyl or C2-8 alkynyl substituted with Cyc4, halogen, $-OR^{148}$, $-SR^{149}$, $-COOR^{150}$ or $-NHCOR^{141}$;

$R^{140}$ represents a hydrogen atom, $-COOR^{142}$ or $-SO_2R^{143}$;

$R^{141}$-$R^{143}$ each independently represents (1) C1-8 alkyl, (2) C2-8 alkenyl, (3) C2-8 alkynyl, (4) Cyc4 or (5) C1-8 alkyl, C2-8 alkenyl or C2-8 alkynyl substituted with Cyc4;

$R^{148}$-$R^{150}$ each independently represents (1) a hydrogen atom, (2) C1-8 alkyl, (3) C2-8 alkenyl, (4) C2-8 alkynyl, (5) Cyc4 or (6) C1-8 alkyl, C2-8 alkenyl or C2-8 alkynyl substituted with Cyc4;

Cyc4 has the same meaning as Cyc1, and Cyc4 may be substituted with 1-5 of $R^{144}$;
$R^{144}$ has the same meaning as $R^{51}$, or
$R^3$ and $R^4$ are taken together to represent

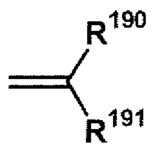

wherein $R^{190}$ and $R^{191}$ each independently represents (1) a hydrogen atom, (2) C1-8 alkyl, (3) C2-8 alkenyl, (4) C2-8 alkynyl, (5) $-COOR^{120}$, (6) $-CONR^{121}R^{122}$, (7) Cyc4 or (8) C1-8 alkyl, C2-8 alkenyl or C2-8 alkynyl substituted with 1-5 substituent(s) selected from (a) halogen, (b) nitrile, (c) Cyc4, (d) $-COOR^{120}$, (e) $-CONR^{121}R^{122}$, (f) $-OR^{123}$, (g) $-SR^{124}$, (h) $-NR^{125}R^{126}$, (i) $-NR^{127}COR^{128}$, (j) $-SO_2NR^{129}R^{130}$, (k) $-OCOR^{131}$, (1) $-NR^{132}SO_2R^{133}$, (m) $-NR^{134}COOR^{135}$, (n) $-NR^{136}CONR^{137}R^{138}$, (o) $-S-SR^{139}$, (p) $-NHC(=NH)NHR^{140}$, (q) keto, (r) $-NR^{145}CONR^{146}COR^{147}$ and (s) $-N(SO_2R^{133})_2$;
$R^{120}$-$R^{140}$ and $R^{145}$- $R^{147}$ have the same meanings as described above;
$R^5$ represents (1) a hydrogen atom, (2) C1-8 alkyl, (3) Cyc5 or (4) C1-8 alkyl substituted with Cyc5;
Cyc5 has the same meaning as Cyc1, and Cyc5 may be substituted with 1-5 of $R^{150}$;
$R^{150}$ has the same meaning as $R^{51}$;
an N-oxide thereof, a salt thereof, or a prodrug thereof,

11. a medicament which comprises the function inhibitor of an effector cell comprising the CCR5 antagonist, in combination with one, two or more immunosuppressive drog(s),
12. the medicament according to the above 11, wherein the one, two or more immunosuppressive drug(s) are selected from the group of tacrolimus, cyclosporine, sirolimus, corticosteroid, azathioprine, mycophenolate mofetil, FTY-720 and cyclophosphamide,
13. a method for prevention and/or treatment of a disease caused by effector cell function, which comprises administering to a mammal an effective amount of the compound of formula (I)

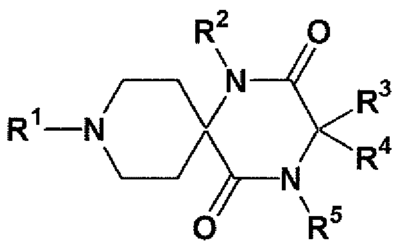

(I)

wherein all symbols have the same meanings as those defined in the above 10,
an N-oxide thereof, a salt thereof, or a prodrug thereof,
14. use of a compound of formula (I)

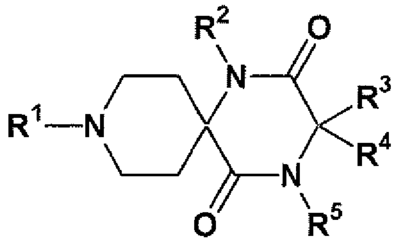

(I)

wherein all symbols have the same meanings as those defined in the above 10,
an N-oxide thereof, a salt thereof, or a prodrug thereof for the manufacture of an agent for prevention and/or treatment of a disease caused by effector cell function, and the like.

In this specification, all of the RA, RO, CD4, CD8, HLA-DR, HLA-ABC, CD11c, CD83, CD80, CD86 and CD3

represent cell surface antigens.

The term "negative" as used in the specification means that a surface antigen cannot be detected, and "positive" means that the surface antigen can be detected. In this connection, all of the surface antigen detection methods so far known are included in the method to be used for the detection of surface antigens. For example, conventionally known techniques which are used by those skilled in the art for detecting protein (e.g., flow cytometry (FACS), immunostaining, western blotting, fluorescent antibody technique, *etc.),* techniques equivalent thereto and the like can be cited.

In the specification, the effector cells are T cells and myeloid cells, and regarding the T cells, all but excluding the following naive T cells are included therein. Examples of the effector cells include RA-negative and/or RO-positive T cells, macrophages and the like, and examples of the "RA-negative and/or RO-positive T cells" include Th1 cell, Th2 cell, cytotoxic T cell (CTL), central memory T cell (TCM), effector memory T cell (TEM) and the like. In this connection, the TCM and TEM are those which are defined by the method described in a reference (*Nature,* 1999, Oct. 14; 401 (6754): 708-12). Preferred as the effector cells are Th1 cell, Th2 cell, CTL, TEM, macrophage and the like, and more preferred are Th1 cell, TEM and the like.

In the specification, the T cell includes all of the cells which express T cell receptor (TCR). As the "cells which express TCR", for example, a CD4-positive CD8-negative T cell (namely, a CD4-positive T cell), a CD4-negative CD8-positive T cell (namely, a CD8-positive T cell), a CD4-negative CD8-negative T cell, a CD4-positive CD8-positive T cell and the like can be cited.

In the specification, the naive T cell represents a T cell which have never received antigen stimulation, and for example, an RA-positive T cell and the like can be cited.

In the specification, the memory T cell represents the same meaning of the effector cell.

In the specification, the CCR5-positive effector cell includes all of the cells which are effector cells and express CCR5. Examples of the CCR5-positive effector cell include a CCR5-positive Th1 cell, a CCR5-positive Th2 cell, a CCR5-positive CTL, a CCR5-positive TCM, a CCR5-positive TEM, a CCR5-positive macrophage and the like. Preferred CCR5-positive effector cells are a CCR5-positive Th1 cell, a CCR5-positive CTL, a CCR5-positive TEM, a CCR5-positive macrophage and the like, and more preferred are a CCR5-positive Th1 cell, a CCR5-positive TEM, a CCR5-positive macrophage and the like.

In the specification, regarding the function of effector cells, it includes all of those which are mediated by CCR5 and concerned in the formation, advance and/or continuation of the T cell-mediated diseases or myeloid cell (macrophage cell)-mediated diseases which are described later. Examples of the function of effector cells include cell migrating cell growth, cell activation and the like. In this connection, the migrating cell, growing cell and activating cell are not limited to the effector cells. That is, not only the case in which the effector cell migrations, grows or activates, but also a case in which the effector cell effects migration of other cell, a case in which the effector cell effects growth of other cell and a case in which the effector cell effects activation of other cell are included therein. Examples of the preferred function of the effector cell include migration of the effector cell, growth of the effector cell, activation of the effector cell and the like. In addition, the term "mediated by CCR5" as used herein means that a step in which CCR5 and its ligand are linked to each other is contained until the effector cell expresses its function, and this is not limited to a case in which the cell which expresses the ligand-binding CCR5 and the function-expressing effector cell are the same. In this connection, the ligand which binds to CCR5 includes all of those which binds to CCR5 and also includes CCR5 ligands so far known and CCR5 ligands which will be found in the future. Preferred examples of the ligand which binds to CCR5 include RANTES, MIP-1$\alpha$, MIP-1$\beta$ and the like. In this connection, RANTES, MIP-1$\alpha$, MIP-1$\beta$ and the like may be in the form of being bound onto the cell membrane. As the pathway from the binding of a ligand with CCR5 to the expression of a function by the effector cell, the cases exemplified in the following can, for example, be cited.

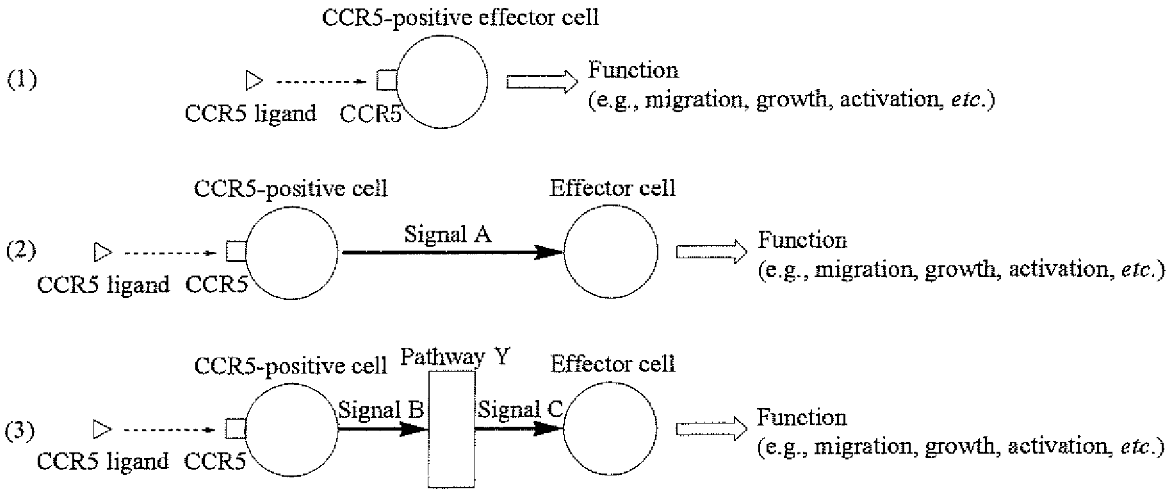


In the above, (1) represents a case in which a CCR5-positive effector cell to which a ligand was bound expresses a function, (2) represents a case in which a CCR5-positive cell to which a ligand was bound transmits a signal A, and an effector cell to which the signal A was transmitted expresses a function, and (3) represents a case in which a CCR5-positive cell to which a ligand was bound transmits a signal B, a pathway Y to which the signal B was transmitted transmits a signal C, and an effector cell to which the signal C was transmitted expresses a function. In this connection, the CCR5-positive cell includes all of the cells which express CCR5. Also, the signals A, B and C include all of those which are transmitted even when a cell which transmits respective signal is not contacted with to which the signal is transmitted, namely humoral factors (e.g., a cytokine, a chemokine, a stimulus via other mediator released from a cell, *etc.),* and/or those which are transmitted through their contact (e.g., an adhesion molecule, a surface antigen, a stimulus via a humoral factor in the state of being bound onto the cell membrane, *etc.*), and the signals A, B and C may be the binding of the above-described ligands which binds to CCR5 with CCR5, or one of the signals may be constructed from two or more factors. In addition, the signals A, B and C may be the same or different from one another. The pathway Y includes all of those which can transmit the signal B to the effector cell as the signal C, and it may contain at least one cell which can receive signal B and at least one cell which can transmit signal C and is not limited by the signal transduction method between them. In this connection, the "cell which can receive signal B" and the "cell which can transmit signal C" may be the same.

As the above-described "pathway from the binding of a ligand with CCR5 to the expression of a function by the effector cell", the one shown by (1) in the foregoing and the like can be preferably exemplified.

In the specification, the cell migration means migration of a cell, and examples include movement of a cell due to concentration gradient of a chemotactic factor, *etc.,* and the like.

In the specification, the cell activation means activation of a cell, and it includes all of the acceleration of cell function, expression of a new function by the cell and the like. As the cell activation, assistance of antibody production and induction of immune response by a cell, expression of action such as cytotoxic activity, and the like.

In the specification, the cell growth means growth of a cell, and examples include increase of the number of cells by cell division.

In the specification, the T cell-mediated disease may be any disease in which T cell is concerned in any of the steps of the formation, advance and/or continuation of the disease, and it may be a disease in which concern of T cell is conventionally known, a disease in which concern of T cell is conventionally known by an animal model of said disease, or a disease in which concern of T cell will be found in the future. Examples of the T cell-mediated disease include a transplant rejection (e.g., rejection of a solid organ graft, rejection of islet cell transplantation in diabetes mellitus, graft-versus-host disease (GVHD), *etc.),* an autoimmune disease (e.g., arthritis, rheumatoid arthritis, multiple sclerosis, ulcerative colitis, *etc.*), an allergic disease (e.g., asthma, *etc.),* an ischemic disease (e.g., ischemia-reperfusion injury, *etc.*) and the like. Preferred examples of the T cell-mediated disease include a transplant rejection, an autoimmune disease and the like.

In the specification, the myeloid cell (macrophage cell)-mediated disease may be any disease in which a myeloid cell is concerned in any of the steps of the formation, advance and continuation of the disease, and it may be a disease in which concern of a myeloid cell is conventionally known, a disease in which concern of a myeloid cell is conventionally known by an animal model of said disease, or a disease in which concern of a myeloid cell will be found in the future. Examples of the macrophage cell-mediated disease include cancer, metastasis and the like.

In the specification, all of the substances which have the CCR5 antagonistic activity are included in the CCR5

antagonist, and all of the CCR5 antagonists so far known and the CCR5 antagonists which will be found in the future are included therein. As the CCR5 antagonist, a peptide CCR5 antagonist and a non-peptide CCR5 antagonist can, for example, be cited. In this connection, the peptide CCR5 antagonist includes all of those which are CCR5 antagonists and consist of peptides, and for example, an anti-CCR5 antibody and the like are also included therein. Also, the non-peptide CCR5 antagonist includes all of those which are CCR5 antagonists and consist of non-peptide substances. Examples of the non-peptide CCR5 antagonist include a low molecular CCR5 antagonist and the like. Preferred as the CCR5 antagonist is a non-peptide CCR5 antagonist, more preferred is a low molecular CCR5 antagonist, and most preferred are the compounds described in WO 02/74770.

The compounds represented by formula (I) are the compounds described in WO 02/74770, and those which are related to the compounds represented by formula (I), such as the terms used in the description of the compounds represented by formula (I), production method of the compounds represented by formula (I), and salts of the compounds represented by formula (I), represent the same meanings as described in the above-described official gazette.

Among the compounds represented by formula (I) to be used as the CCR5 antagonists, the compounds described in Examples of the above-described official gazette can be preferably exemplified. Among these, more preferred compounds include:

(1) 4-[4-({(3R)-1-butyl-3-[(R)-cyclohexyl(hydroxy)methyl]-2,5-dioxo-1,4,9-triazaspiro[5.5] undec-9-yl}methyl)benzyl]-3-methoxybenzoic acid hydrochloride,
(2) 4-[4-({(3R)-1-butyl-3-[(R)-cyclohexyl(hydroxy)methyl]-2,5-dioxo-1,4,9-triazaspiro[5.5]undec-9-yl}methyl)phenoxy]-3-ethoxybenzoic acid hydrochloride,
(3) 4-[4-({(3R)-1-butyl-3-[(1R)-2-ethyl-1-hydroxybutyl]-2,5-dioxo-1,4,9-triazaspiro[5.5]undec-9-yl}methyl)phenoxy]-3-methoxybenzoic acid hydrochloride,
(4) 4-[4-({(3R)-1-butyl-3-[(R)-cyclohexyl(hydroxy)methyl]-2,5-dioxo-1,4,9-triazaspiro[5.5]undec-9-yl}methyl)phenoxy]-3,5-dimethylbenzoic acid hydrochloride,
(5) 4-[4-(1-{(3R)-1-butyl-3-[(R)-cyclohexyl(hydroxy)methyl]-2,5-dioxo-1,4,9-triazaspiro[5.5]undec-9-yl}ethyl)phenoxy]benzoic acid hydrochloride,
(6) 4-[4-({(3R)-1-butyl-3-[(R)-3-cyclopenten-1-yl(hydroxy)methyl]-2,5-dioxo-1,4,9-triazaspiro[5.5]undec-9-yl}methyl)phenoxy]-3-methoxybenzoic acid hydrochloride,
(7) 4-[4-({(3R)-1-butyl-3-[(R)-cycloheptyl(hydroxy)methyl] -2,5 -dioxo- 1, 4,9-triazaspiro[5.5]undec-9-yl}methyl)phenoxy]-3-methaxybenzoic acid hydrochloride,
(8) 4-[4-({(3R)-1-butyl-3-[(R)-3-cyclopenten-1-yl(hydroxy)methyl]-2,5-diaxo-1,4,9-triazaspiro[5.5]undec-9-yl}methyl)phenaxy]-3-ethoxybenzoic acid hydrochloride,
(9) 4-[4-({(3R)-1-butyl-3-[(R)-hydroxy(tetrahydro-2H-pyran-4-yl)methyl]-2,5-dioxo-1,4,9-triazaspiro[5.5]undec-9-yl}methyl)phenoxy]-N-isopropyl-3-methoxybenzamide hydrochloride,
(10) 4-[4-({(3R)-1-butyl-3-[(R)-hydroxy(tetrahydro-2H-pyran-4-yl)methyl]-2,5-dioxo-1,4,9-triazaspiro[5.5]undec-9-yl}methyl)benzyl]-N-(2,2-dimethylpropyl)-3-methoxybenzamide hydrochloride,
(11) N-{4-[4-({(3R)-1-butyl-3-[(1R)-1-hydroxy-2-methylpropyl]-2,5-dioxo-1,4,9-triazaspiro[5.5]undec-9-yl}methyl)-phenoxy]-3-methoxyphenyl} methanesulfonamide hydrochloride,
(12) N-{4-[4-({(3R)-1-butyl-3-[(R)-hydroxy(tetrahydro-2H-pyran-4-yl)methyl]-2,5-dioxo-1,4,9-triazaspiro[5.5]undec-9-yl}methyl)phenoxy]-3-methoxyphenyl}-2-methylpropaneamide hydrochloride,
(13) 4-(4-{[(3S)-1-butyl-3-(cyclohexylmethyl)-2,5-dioxo-1,4,9-triazaspiro[5.5]undec-9-yl]methyl}phenoxy)benzoic acid hydrochloride,
(14) N-{4-[4-({(3R)-1-butyl-3-[(1R)-1-hydroxy-2-methylpropyl]-2,5-dioxo-1,4, 9-triazaspira[5.5]undec-9-yl}methyl)-3,5-dimethyl-1H-pyrazol-1-yl]phenyl}methanesulfonamide dihydrochloride,
(15) 4-[4-({(3R)-1-butyl-3-[(1R)-hydroxy-2-methylpropyl]-2,5-diaxo-1,4,9-triazaspiro[5.5]undec-9-yl}methyl)phenoxy]benzoic acid hydrochloride,
(16) 4-[4-({(3R)-1-butyl-3-[(R)-cyclohexyl(hydroxy)methyl]-2,5-dioxo-1,4,9-triazaspiro[5.5]undec-9-yl}methyl)-3,5-dimethyl-1H-pyrazol-1-yl]-N-methylbenzamide dihydrochloride,
(17) 4-[4-({(3R)-1-butyl-3-[(R)-cyclohexyl(hydroxy)methyl]-2,5-dioxo-1,4,9-triazaspiro[5.5]undec-9-yl}methyl)phenoxy]benzoic acid hydrochloride,
(18) 4-(4-{(3R)-1-but-2-in-1-yl-3-[(R)-cyclohexyl(hydroxy)methyl]-2,5-dioxo-1,4,9-triazaspira[5.5]undec-9-yl}butoxy)-N-methylbenzamide hydrochloride,
(19) 4-(4-{[(3S)-1-butyl-3-(cyclohexylmethyl)-2,5-dioxo-1,4,9-triazaspiro[5.5]undec-9-yl]methyl}phenoxy)benzoic acid hydrochloride,
(20) N-{4-[4-({(3R)-1-butyl-3-[(1R)-1-hydroxy-2-methylpropyl]-2,5-diaxo-1,4,9-triazaspiro[5.5]undec-9-yl}methyl)-3,5-dimethyl-1H-pyrazol-1-yl]phenyl}methanesulfonamide dihydrochloride,
(21) 4-[4-({(3R)-1-butyl-3-[(1R)-1-hydroxy-2-methylpropyl]-2,5-dioxo-1,4,9-triazaspiro[5.5]undec-9-yl}methyl)phenoxy]benzoic acid hydrochloride,

(22) 4-[4-({(3R)-1-butyl-3-[(R)-cyclohexyl(hydroxy)methyl]-2,5-dioxo-1,4,9-triazaspiro[5.5]undec-9-yl}methyl)-3,5-dimethyl-1H-pyrazol-1-yl]-N-methylbenzamide dihydrochloride,
(23) 4-[4-({(3R)-1-butyl-3-[(R)-cyclohexyl(hydroxy)methyl]-2,5-diaxo-1,4,9-triazaspiro[5.5]undec-9-yl}methyl)phenoxy]benzoic acid dihydrochloride,
(24) 4-(4-{(3R)-1-but-2-ynyl-3-[(R)-cyclohexyl(hydroxy)methyl]-2,5-dioxo-1,4,9-triazaspiro[5.5]undec-9-yl}butoxy)-N-methylbenzamide hydrochloride,
(25) 4-(2-(3R)-1-butyl-3-[(R)-cyclohexyl(hydroxy)methyl]-2,5-dioxo-1,4,9-triazaspiro[5.5]undec-9-yl)ethoxy)-N-methylbenzamide hydrochloride,
(26) 4-({(3R)-1-butyl-3-[(R)-cyclohexyl(hydroxy)methyl]-2,5-dioxo-1,4,9-triazaspiro[5.5]undec-9-yl}methyl)-N-{4-[(methylamino)carbonyl]benzyl}benzamide hydrochloride,
(27) 4-[4-({(3R)-1-butyl-3-[(R)-cyclohexyl(hydroxy)methyl]-2,5-dioxo-1,4,9-triazaspiro[5.5]undec-9-yl}methyl)phenoxy]-N-(cyclopropylmethyl)benzamide hydrochloride,
(28) 4-[4-({(3R)-1-butyl-3-[(R)-cyclohexyl(hydroxy)methyl]-2,5-diaxo-1,4,9-triazaspiro[5.5]undec-9-yl}methyl)phenoxy]-3-methoxybenzoic acid hydrochloride,
(29) 4-[4-({(3R)-1-butyl-3-[(R)-cyclohex-3-en-1-yl(hydroxy)methyl]-2,5-dioxo-1,4,9-triazaspiro[5.5]undec-9-yl}methyl)phenoxy]-N-methylbenzamide hydrochloride,
(30) 4-[4-({(3R)-1-butyl-3-[(R)-cyclohexyl(hydroxy)methyl]-2,5-dioxo-1,4,9-triazaspiro[5.5]undec-9-yl}methyl)phenoxy]-3-methoxy-N-methylbenzamide hydrochloride,
(31) 4-[4-({(3R)-1-butyl-3-[(R)-cyclohexyl(hydroxy)methyl]-2,5-dioxo-1,4,9-triazaspiro[5.5]undec-9-yl}methyl)phenoxy]-3-chlorobenzoic acid hydrochloride,
(32) 4-[4-({(3R)-1-butyl-3-[(R)-cyclohexyl(hydroxy)methyl]-2,5-dioxo-1,4,9-triazaspiro[5.5]undec-9-yl}methyl)phenoxy]-3-chloro-N-methylbenzamide hydrochloride,
(33) $N^1$-[4-({(3R)-1-butyl-3-[(R)-cyclohexyl(hydroxy)methyl]-2,5-dioxo-1,4,9-triazaspiro[5.5]undec-9-yl}methyl)benzyl]-$N^4$-methylterephthalamide hydrochloride,
(34) (3R)-1-butyl-3-[(R)-cyclohexyl(hydroxy)methyl]-9-{4-[4-(5-oxo-4,5-dihydro-1,2,4-thiadiazal-3-yl)phenoxy]benzyl}-1,4,9-triazaspirn[5.5]undecane-2,5-dione hydrochloride,
(35) 4-[4-({(3R)-1-butyl-3-[(R)-cydohexyl(hydroxy)methyl]-2,5-dioxo-1,4,9-triazaspiro[5.5]undec-9-yl}methyl)benzyl]-3-methoxybenzoic acid hydrochloride,
(36) 4-[4-({(3R)-1-butyl-3-[(R)-cyclohexyl(hydroxy)methyl]-2,5-dioxo-1,4,9-triazaspiro[5.5]undec-9-yl}methyl)phenoxy]-3-methoxy-N,N-dimethylbenzamide hydrochloride,
(37) N-{4-[4-({(3R)-1-butyl-3-[(R)-cyclohexyl(hydroxy)methyl]-2,5-dioxo-1,4,9-triazaspiro[5.5]undec-9-yl}methyl)-phenoxy]-3-methoxyphenyl} methane sulfonamide hydrochloride,
(38) 4-[4-({(3R)-1-butyl-3-[(R)-cyclohexyl(hydroxy)methyl]-2,5-dioxo-1,4,9-triazaspiro[5.5]undec-9-yl}methyl)phenoxy]-3-ethoxybenzole acid hydrochloride,
(39) 4-[4-({(3R)-3-[(R)-cyclohexyl(hydroxy)methyl]-2,5-dioxo-1-pentyl-1,4,9-triazaspiro[5.5]undec-9-yl}methyl)phenoxy]-3-methoxybenzoic acid hydrochloride,
(40) N-{4-[4-({(3R)-1-butyl-3-[(R)-cyclohexyl(hydroxy)methyl]-2,5-dioxo-1,4,9-triazaspiro[5.5]undec-9-yl}methyl)-phenoxy]-3-methoxyphenyl} acetamide hydrochloride,
(41) 4-[4-({(3R)-1-butyl-3-[(lR)-2-ethyl-1-hydroxybutyl]-2,5-dioxo-1,4,9-triazaspiro[5.5]undec-9-yl}methyl)phenoxy]-3-methoxybenzoic acid hydrochloride,
(42) 4-[4-({(3R)-1-butyl-3-[(R)-hydroxy(tetrahydro-2H-pyran-4-yl)methyl]-2,5-dioxo-1,4,9-triazaspiro[5.5]undec-9-yl}methyl)phenoxy]-N-(cyclopropylmethyl)benzamide hydrochloride,
(43) 4-[4-({(3R)-1-butyl-3-[(R)-cyclohexyl(hydroxy)methyl]-2,5-dioxo-1,4,9-triazaspiro[5.5]undec-9-yl}methyl)phenoxy]-3,5-dimethylbenzoic acid hydrochloride,
(44) 4-[4-(1-{(3R)-1-butyl-3-[(R)-cyclohexyl(hydroxy)methyl]-2,5-dioxo-1,4,9-triazaspiro[5.5]undec-9-yl}ethyl)phenoxy]benzoic acid hydrochloride,
(45) 4-[4-({(3R)-1-butyl-3-[(1R)-2-ethyl-1-hydroxybutyl]-2,5-dioxa-1,4,9-triazaspiro[5.5]undec-9-yl}methyl)phenoxy]-3-methoxy-N-methylbenzamide hydrochloride,
(46) 4-[4-({(3R)-1-butyl-3-[(R)-3-cyclopenten-1-yl(hydroxy)methyl]-2,5-dioxo-1,4,9-triazaspiro[5.5]undec-9-yl}methyl)phenoxy]-3-methylbenzoic acid hydrochloride,
(47) 4-[4-({(3R)-1-butyl-3-[(R)-cycloheptyl(hydroxy)methyl]-2,5-dioxo-1,4,9-triazaspiro[5.5]undec-9-yl}methyl)phenoxy]-3-methoxybenzoic acid hydrochloride,
(48) 4-[4-({(3R)-1-butyl-3-[(R)-cyclopentyl(hydroxy)methyl]-2,5-dioxo-1,4,9-triazaspiro[5.5]undec-9-yl}methyl)phenoxy]-3-ethoxybenzoic acid hydrochloride,
(49) 4-[4-({(3R)-1-butyl-3-[(R)-cyclopent-3-en-1-yl(hydroxy)methyl]-2,5-dioxo-1,4,9-triazaspiro[5.5]undec-9-yl}methyl)phenoxy]-3-ethoxybenzoic acid hydrochloride,
(50) 4-[4-({(3R)-1-butyl-3-[(R)-cyclopentyl(hydroxy)methyl]-2,5-dioxo-1,4,9-triazaspiro[5.5]undec-9-yl}methyl)phenoxy]-3-methoxy-N,N-dimethylbenzamide hydrochloride,

(51) 4-[4-({(3R)-1-butyl-3-[(R)-3-cyclopenten-1-yl(hydroxy)methyl]-2,5-dioxo-1,4,9-triazaspiro[5.5]undec-9-yl}methyl)phenoxy]-3-methoxy-N-methylbenzamide hydrochloride,
(52) 4-[4-({(3R)-1-butyl-3-[(1R)-2-ethyl-1-hydroxybutyl]-2,5-dioxo-1,4,9-triazasp iro [5.5]undec-9-yl}methyl)phenoxy]-3-methoxy-N-(2-methoxyethyl)benzamide hydrochloride,
(53) 4-[4-({(3R)-1-butyl-3-[(R)-cyclopentyl(hydroxy)methyl]-2,5-dioxo-1,4,9-triazaspiro[5.5]undec-9-yl}methyl)phenoxy]-3-methoxy-N-methylbenzamide hydrochloride,
(54) 4-[4-({(3R)-1-butyl-3-[(R)-hydroxy(tetrahydro-2H-pyran-4-yl)methyl]-2, 5-dioxo-1,4,9-triazaspiro[5.5]undec-9-yl}methyl)phenoxy]-N-(cyclopropylmethyl)-3-methoxybenzamide hydrochloride,
(55) 4-[4-({(3R)-1-butyl-3-[(R)-hydroxy(tetrahydro-2H-pyran-4-yl)methyl]-2,5-dioxo-1,4,9-triazaspiro[5.5]undec-9-yl}methyl)benzyl]-3-methoxy-N-methylbenzamide hydrochloride,
(56) 4-[4-({(3R)-1-butyl-3-[(R)-hydroxy(tetrahydro-2H-pyran-4-yl)methyl]-2,5-diaxa-1,4,9-triazaspiro[5.5]undec-9-yl}methyl)phenoxy]-N-isopropyl-3-methoxybenzoic acid hydrochloride,
(57) 4-[4-({(3R)-1-butyl-3 -[(R)-hydroxy(tetrahydro-2H-pyran-4-yl)methyl]-2,5-dioxo-1,4,9-triazaspiro[5,5]undec-9-yl} methyl)phenoxy]-N-isobutyl-3-methoxybenzamide hydrochloride,
(58) 4-[4-({(3R)-1-butyl-3-[(R)-hydroxy(tetrahydro-2H-pyran-4-yl)methyl]-2,5-dioxo-1,4,9-triazaspiro[5.5]undec-9-yl}methyl)phenoxy]-3-methoxy-N-neopentylbenzamide hydrochloride,
(59) N-{4-[4-({(3R)-1-butyl-3-[(1R)-1-hydroxy-2-methylpropyl]-2,5-dioxo-1,4,9-triazaspiro[5.5]undec-9-yl}methyl)-phenoxy]-3-methoxyphenyl} methane sulfonamide hydrochloride,
(60) N-{4-[4-({(3R)-1-butyl-3-[(R)-hydroxy(tetrahydro-2H-pyran-4-yl)methyl]-2,5-dioxo-1,4,9-triazaspiro[5.5]undec-9-yl}methyl)phenoxy]-3-anethoxyphenyl}-2-methylpropanamide hydrochloride,
(61) N-{4-[4-({(3R)-1-butyl-3-[(R)-hydroxy(tetrahydro-2H-pyran-4-yl)methyl]-2,5-dioxo-1,4,9-triazaspiro[5.5]undec-9-yl}methyl)phenoxy]-3-methoxyphenyl}-3-methylbutanamide hydrochloride, and the like. Particularly desirable compounds include:

(1) 4-[4-({(3R)-1-butyl-3-[(R)-cyclohexyl(hydroxy)methyl]-2,5-dioxo-1,4,9-triazaspiro[5.5]undec-9-yl}methyl)-benzyl]-3-methoxybenzoic acid hydrochloride,
(2) 4-[4-({(3R)-1-butyl-3-[(R)-cyclohexyl(hydroxy)methyl]-2,5-dioxo-1,4,9-triazaspiro[5.5]undec-9-yl}methyl)-phenoxy]-3-ethoxybenzoic acid hydrochloride,
(17) 4-[4-({(3R)-1-butyl-3-[(R)-cyclohexyl(hydroxy)methyl]-2,5-dioxo-1,4,9-triazaspiro[5.5]undec-9-yl}methyl)-phenoxy]benzoic acid hydrochloride, and the like.

The compound names described in the specification are the result of carrying out naming and numbering, using ACD/NAME (version 6.08/25) (trade name, by Advanced Chemistry Development) which is a computer program that mechanically produces IUPAC names.

For example, hydrochloride of a compound of formula (I) in which $R^1$ represents

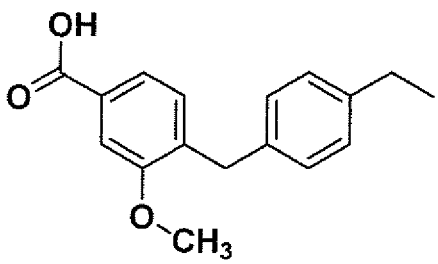

$R^2$ represents

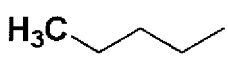

$R^3$ represents

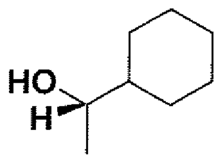

and $R^4$ represents a hydrogen atom, namely a compound represented by

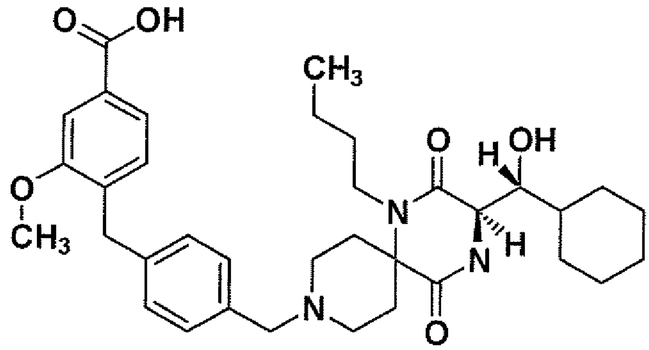

is named 4-[4-({(3R)-1-butyl-3-[(R)-cyclohexyl(hydroxy)methyl]-2,5-dioxo-1,4,9-triazaspiro[5.5]undec-9-yl}methyl)benzyl]-3-methoxybenzoic acid hydrochloride.

In addition, examples of the CCR5 antagonist according to the specification include compounds represented by formula (II), salts thereof or solvates thereof, or prodrugs thereof

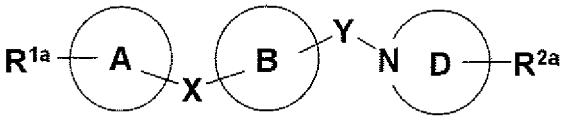 (II)

(in the formula, $R^{1a}$ represents a hydrogen atom or an acidic group which may be protected, X and Y each independently represents a linking bond or a spacer having from 1 to 3 atoms of the principal chain, ring A and ring B may be the same or different from each other and each represents a 3- to 15-membered homocyclic or heterocyclic ring which may further have a substituent, ring D represents a 3- to 15-membered nitrogen-containing heterocyclic ring which may further have a substituent, and $R^{2a}$ represents (1) a hydrogen atom, (2) a hydrocarbon group which may have a substituent(s), (3) cyano, (4) hydroxy group which may be protected, (5) amino which may have a substituent(s), (6) oxo, (7) a 3- to 15-membered heterocyclic ring which may have a substituent(s), or (8) = N-$OR^{6a}$ ($R^{6a}$ represents a hydrogen atom or C1-4 alkyl)).

The "acidic group which may be protected" represented by $R^{1a}$ represents an "acidic group" which may be protected by a "protecting group". Examples of the "acidic group" include various Brønsted acids such as hydroxy, alkoxy, carboxy (-COOH), sulfo ($-SO_3H$), sulfino ($-SO_2H$), sulfonamide ($-SO_2NH_2$ or $-NR^{101a}SO_3H$ ($R^{101a}$ represents a hydrogen atom or a hydrocarbon group which may have a substituent(s))), phosphono ($-PO(OH)_2$), phenol ($-C_6H_4OH$), a nitrogen-containing ring group having a hydrogen atom which can be deprotonated and the like. The "Brønsted acid" represents a substance which gives a hydrogen ion to other substance. Examples of the "nitrogen-containing ring group having a hydrogen atom which can be deprotonated" include:

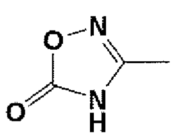 , 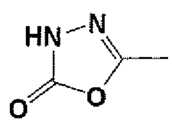 , 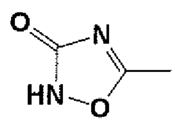 , 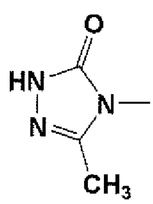 ,

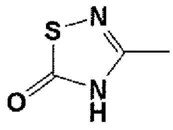 , 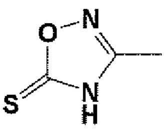 , 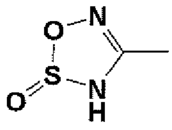 , 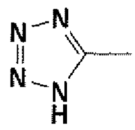 ,

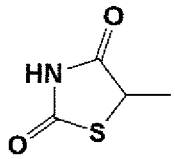 , 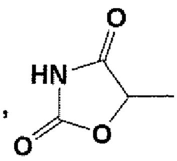 , 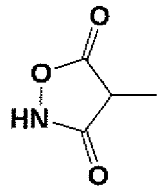 or 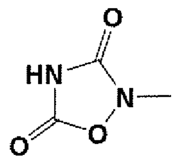

and the like. As preferred "acidic group", carboxy or sulfonamide can be cited. More preferably, sulfonamide can be cited.

In addition, examples of the "protecting group" include a hydrocarbon group which may have a substituent(s), alkoxy having from 1 to 6 carbon atoms, amino which may have a substituent(s),

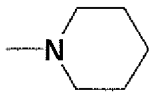

or

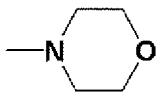

and the like.

Examples of the "hydrocarbon group" in the "hydrocarbon group which may have a substituent(s)" include alkyl having from 1 to 15 carbon atoms such as methyl, ethyl, propyl, isopropyl, butyl, isobutyl, s-butyl, t-butyl, pentyl, hexyl, heptyl, octyl, nonyl, decyl, undecyl, dodecyl, tridecyl, tetradecyl, or pentadecyl; cycloalkyl having from 3 to 8 carbon atoms such as cyclopropyl, cyclobutyl, cyclopentyl, or cyclohexyl; alkenyl having from 2 to 10 carbon atoms such as vinyl, allyl, 2-methylallyl, 2-butenyl, 3-butenyl, or 3-octenyl; alkynyl having from 2 to 10 carbon atoms such as ethynyl, 2-propynyl, or 3-hexynyl; cycloalkenyl having from 3 to 10 carbon atoms such as cyclopropenyl, cyclopentenyl, or cyclohexenyl; aryl having from 6 to 14 carbon atoms such as phenyl or naphthyl; aralkyl having from 7 to 16 carbon atoms such as benzyl or phenylethyl; and (cycloalkyl having from 3 to 8 carbon atoms)-(alkyl having from 1 to 4 carbon atoms) such as cyclohexylmethyl, cyclohexylethyl, cyclohexylpropyl, 1-methyl-1-cyclohexylmethyl or cyclopropylethyl. Also, examples of the "substituent" in the "hydrocarbon group which may have a substituent(s)" include (1) nitro, (2) hydroxy group, (3) oxo, (4) thioxo, (5) cyano, (6) carbamoyl, (7) aminocarbonyl substituted with a hydrocarbon having from 1 to 8 carbon atoms such as N-butylaminocarbonyl, N-cyclohexylmethylaminacarbonyl, N-butyl-N-cyclohexylmethylaminocarbonyl, N-cyclohexylaminocarbonyl, or phenylaminocarbonyl, (8) carboxy, (9) alkoxycarbonyl having from 1 to 4 carbon atoms such as methoxycarbonyl or ethoxycarbonyl, (10) sulfo, (11) halogen such as fluorine, chlorine, bromine or iodine, (12) lower alkoxy having from 1 to 4 carbon atoms which may be substituted with halogen, such as methoxy, ethoxy, propoxy, isopropoxy, butoxy, isobutoxy, s-butoxy, t-butoxy, difluoromethoxy, or trifluoromethoxy, (13) phenoxy, (14) halogenophenoxy such as o-, m- or p-chlorophenoxy or o-, m- or p-bromophenoxy, (15) lower alkylthio having from 1 to 4 carbon atoms such as methylthio, ethylthio, n-propylthio, isopropylthio, n-butylthio, or t-butylthio, (16) phenylthio, (17) lower alkylsulfinyl having from 1 to 4 carbon atoms such as methylsulfinyl or ethylsulfinyl, (18) lower alkylsulfonyl having from 1 to 4 carbon atoms such as methylsulfonyl or ethylsulfonyl, (19) amino, (20) lower acylamino having from 1 to 6 carbon atoms such as acetylamino or propionylamino, (21) primary or secondary amino substituted with a hydrocarbon group (this "hydrocarbon group" has the same meaning as the above-described "hydrocarbon group" and may be substituted with amino, carbamoyl, halogen, hydroxy group or the like which may be substituted with oxo or an optional substituent (e.g., a hydrocarbon group, *etc.*)), such as methylamino, ethylamino, n-propylamino, isopropylamino, n-butylamino, dimethylamino, diethylamino, cyclohexylamino, 1-carbamoyl-2-cyclohexylethylamino, N-butyl-N-cyclohexylmethylamino, or phenylamino, (22) lower acyl having from 1 to 4 carbon atoms such as formyl or acetyl, (23) benzoyl, (24) a 5- or 6-membered heterocyclic group containing 1 to 4 hetero atoms selected from oxygen, sulfur, nitrogen and the like in

addition to carbon, which may have 1 to 4 substituent(s) selected from (a) halogen such as bromine, chlorine, or fluorine, (b) hydrocarbon group which may be substituted with oxo, hydroxy or the like (this "hydrocarbon group" has the same meaning as the above-described "hydrocarbon group"), such as methyl, ethyl, propyl, isopropyl, benzyl, cyclohexyl, cyclohexylmethyl, or cyclohexylethyl, (c) halogenophenoxy such as o-, m- or p-chlorophenoxy or o-, m- or p-bromophenoxy, and (d) oxo or the like, such as 2- or 3-thienyl, 2- or 3-furyl, 3-, 4- or 5-pyrazolyl, 4-tetrahydropyranyl, 2-, 4- or 5-thiazolyl, 3-, 4- or 5-isothiazolyl, 2-, 4- or 5-oxazolyl, 3-, 4- or 5-isoxazolyl, 2-, 4- or 5-imidazolyl, 1,2,3- or 1,2,4-triazolyl, 1H- or 2H-tetrazolyl, 2-, 3- or 4-pyridyl, 2-, 4- or 5-pyrimidyl, 3- or 4-pyridazinyl, quinolyl, isoquinolyl, or indolyl, (25) haloalkyl having from 1 to 10 carbon atoms such as difluoromethyl, trifluoromethyl, trifluoroethyl, or trichloroethyl, (26) hydroxyimino, (27) alkyloxyimino such as methyloxyimino or ethyloxyimino, (28) alkylsulfonylamino such as methylsulfonylamino, ethylsulfonylamino, or benzylsulfonylamino, and (29) arylsulfonylamino such as phenylsulfonylamino or p-toluenesulfonylamino. The "hydrocarbon group which may have a substituent(s)" may have 1 to 10 substituent(s) selected from the above-described (1) to (29), and when the "hydrocarbon group" is cycloalkyl, cycloalkenyl, aryl or aralkyl, it may have 1 to 4 of lower alkyl having from 1 to 4 carbon atom(s) as the substituent(s), such as methyl, ethyl, propyl, isopropyl, and butyl. In addition, when the number of substituents is two or more, respective substituents may be the same or different from one another.

Examples of the substituent of amino in the "amino which may have a substituent(s)" according to the "protecting group" include the "hydrocarbon group which may have a substituent(s)" defined above.

Examples of the "alkoxy having from 1 to 6 carbon atoms" in the "protecting group" include methoxy, ethoxy, propoxy, butoxy, pentyloxy, hexyloxy and the like.

Preferred examples of the "protecting group" in $R^{1a}$ include a hydrocarbon group which may have a substituent(s), and more preferred examples include alkyl having from 1 to 4 carbon atoms or the like.

For example, ester such as methoxycarbonyl or ethoxycarbonyl and amide such as carbamoyl are also included in the "acidic group which may be protected" represented by $R^{1a}$.

Preferred examples of $R^{1a}$ include $-SO_2NR^{102a}R^{103a}$, $-NR^{101a}SO_2R^{104a}$, $-COOR^{105a}$, $-CONR^{106a}R^{107a}$ (in the formulae, $R^{102a}$ to $R^{107a}$ represent a hydrogen atom or the protecting groups defined above, and other symbols have the same meanings as the above-described counterparts) and the like. More preferred is $-SO_2NR^{102a}R^{103a}$ or $-NR^{101a}SO_2R^{104a}$.

The "spacer having from 1 to 3 atoms of the principal chain" shown by X and Y means an interval where 1 to 3 atoms of the principal chain are stood in a row. In this case, the "number of atoms of the principal chain" is counted in such a manner that atoms of the principal chain become minimum. Examples of the "spacer having from 1 to 3 atoms of the principal chain" include divalent groups and the like containing 1 to 3 groups selected from $-CR^{7a}R^{8a}-$, $-NR^{9a}-$ -CO-, -O-, -S-, -SO-, $-SO_2-$ and $-C(=NOR^{10a})-$ (in the formulae, $R^{7a}$ and $R^{8a}$ each independently represents a hydrogen atom, C1-4 alkyl, $-OR^{11a}$ or phenyl, $R^{9a}$ represents a hydrogen atom, C1-4 alkyl or phenyl, and $R^{10a}$ and $R^{11a}$ each independently represents a hydrogen atom or C1-4 alkyl). In this case, examples of the "C1-4 alkyl" includes methyl, ethyl, propyl, butyl and the like. Specific examples include $-CR^{7a}R^{8a}-$ $-NR^{9a}-$, -CO-, -O-, -S-, $-C(=N-OR^{10a})-$, $-NR^{9a}CO-$, $-CONR^{9a}-$, $-NR^{9a}COCR^{7a}R^{8a}-$, $-CONR^{9a}CR^{7a}R^{8a}-$ and the like (in the formulae, $R^{7a}$ to $R^{10a}$ have the same meanings as defined above). Examples of the preferred spacer according to the "spacer having from 1 to 3 atoms of the principal chain" represented by X include $-CR^{7a}R^{8a}-$ $-NR^{9a}-$, -CO-, -O-, -S-, -SO-, $-SO_2-$, $-C(=N-OR^{10a})-$ and the like (in the formulae, $R^{7a}$ and $R^{8a}$ each independently represents a hydrogen atom, C1-4 alkyl, $-OR^{11a}$ or phenyl, $R^{9a}$ represents a hydrogen atom, C1-4 alkyl or phenyl, and $R^{10a}$ and $R^{11a}$ each independently represents a hydrogen atom or C1-4 alkyl).

Preferred as X includes a linking bond, -O-, $-CH_2-$ and the like.

"C1-3 alkylene" can be preferably cited as the "spacer having from 1 to 3 atoms of the principal chain" represented by Y. Examples of the "C1-3 alkylene" include methylene, ethylene, propylene and the like. In addition, preferred as Y is methylene.

Examples of the "3- to 15-membered homocyclic ring" of the "3- to 15-membered homocyclic or heterocyclic ring which may further have a substituent" represented by ring A and ring B include "cyclic hydrocarbon having from 3 to 15 carbon atoms" and the like. Examples of the "cyclic hydrocarbon" of the "cyclic hydrocarbon having from 3 to 15 carbon atoms" include "unsaturated cyclic hydrocarbon" and "saturated cyclic hydrocarbon". Examples of the "saturated cyclic hydrocarbon" include cycloalkanes such as cyclopropane, cyclobutane, cyclopentane, cyclohexane, cycloheptane, cyclooctane, cyclononane, cyclodecane, cycloundecane, cyclododecane, cyclotridecane, cyclotetradecane and cyclopentadecane, as well as perhydropentalene, perhydroazulene, perhydroindene, perhydronaphthalene, perhydroheptalene, spiro[4.4]nonane, spiro[4.5]decane, spiro[5.5]undecane, bicyclo[2.2.1]heptane, bicyclo[3.1.1]heptane, bicyclo[2.2.2]octane, adamantane, noradamantane and the like. Examples of the "unsaturated cyclic hydrocarbon" include cycloalkenes such as cyclopentene, cylohexene, cycloheptene, cyclooctene, cyclopentadiene, cyclohexadiene, cycloheptadiene and cyclooctadiene, as well as benzene, pentalene, azulene, indene, indane, naphthalene, dihydronaphthalene, tetrahydronaphthalene, heptalene, biphenylene, as-indacene, s-indacene, acenaphthene, acenaphthylene, fluorene, phenalene, phenanthrene, anthracene, bicyclo[2.2.1]hept-2-ene, bicyclo[3.1.1]hept-2-ene, bicyclo[2.2.2]oct-2-ene and the like.

Examples of the "3- to 15-membered heterocyclic ring" of the "3- to 15-membered homocyclic or heterocyclic ring which may have a substituent(s)" represented by ring A and ring B include "3- to 15-membered unsaturated heterocyclic ring" and "3- to 15-membered saturated heterocyclic ring".

Examples of the "3- to 15-membered unsaturated heterocyclic ring" include pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzoazepine, benzodiazepine, benzofurazane, benzothiadiazole, benzotriazole, carbazole, β-carboline, acridine, phenazine, dibenzofuran, xanthene, dibenzothiophene, phenothiazine, phenoxazine, phenoxathiine, thianthrene, phenanthridine, phenanthroline, perimidine, pyrroline, imidazoline, triazoline, tetrazoline, pyrazoline, dihydropyridine, tetrahydropyridine, dihydropyrazine, tetrahydropyrazine, dihydropyrimidine, tetrahydropyrimidine, dihydropyridazine, tetrahydropyridazine, dihydroazepine, tetrahydroazepine, dihydrodiazepine, tetrahydrodiazepine, dihydrofuran, dihydropyran, dihydrooxepine, tetrahydrooxepine, dihydrothiophene, dihydrothiopyran, dihydrothiepine, tetrahydrothiepine, dihydrooxazole, dihyroisoxazole, dihydrothiazole, dihydroisothiazole, dihydrofurazane, dihydrooxadiazole, dihydrooxazine, dihydrooxadiazine, dihydrooxazepine, tetrahydrooxazepine, dihydrooxadiazepine, tetrahydrooxadiazepine, dihydrothiadiazole, dihydrothiazine, dihydrothiadiazine, dihydrothiazepine, tetrahydrothiazepine, dihydrothiadiazepine, tetrahydrothiadiazepine, indoline, isoindoline, dihydrobenzofuran, dihydroisobenzofuran, dihydrobenzothiophene, dihydroisobenzothiophene, dihydroindazole, dihydroquinoline, tetrahydroquinoline, dihydroisoquinoline, tetrahydroisoquinoline, dihydrophthalazine, tetrahydrophthalazine, dihydronaphthyridine, tetrahydronaphthyridine, dihydroquinoxaline, tetrahydroquinoxaline, dihydroquinazoline, tetrahydroquinazoline, dihydrocinnoline, tetrahydrocinnoline, benzoxathian, dihydrobenzoxazine, dihydrobenzothiazine, pyrazinomorpholine, dihydrobenzoxazole, dihydrobenzothiazole, dihydrobenzimidazole, dihydrobenzoazepine, tetrahydrobenzoazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, dihydroacridine, tetrahydroacridine, dihydrodibenzofuran, dihydrodibenzothiophene, tetrahydrodibenzofuran, tetrahydrodibenzothiophene, dioxaindane, benzodioxane, chroman, benzodithiolan, benzodithian ring and the like. Also, examples of the "3- to 15-membered saturated heterocyclic ring" include aziridine, azetidine, azocane, pyrrolidine, imidazolidine, triazolidine, tetrazolidine, pyrazolidine, piperidine, piperazine, perhydropyrimidine, perhydropyridazine, perhydroazepine, perhydrodiazepine, oxirane, oxetane, tetrahydrofuran, tetrahydropyran, perhydrooxepine, thiirane, thiethane, tetrahydrothiophene, tetrahydrothiopyran, perhydrothiepine, tetrahydrooxazole (oxazolidine), tetrahydroisoxazole (isoxazolidine), tetrahydrothiazole (thiazolidine), tetrahydroisothiazole (isothiazolidine), tetrahydrofurazane, tetrahydrooxadiazole (oxadiazolidine), tetrahydrooxazine, tetrahydrooxadiazine, perhydrooxazepine, perhydrooxadiazepine, tetrahydrothiadiazole (thiadiazolidine), tetrahydrothiazine, tetrahydrothiadiazine, perhydrothiazepine, perhydrothiadiazepine, morpholine, thiomorpholine, oxathian, perhydrobenzofuran, perhydroisobenzofuran, perhydrobenzothiophene, perhydroisobenzothiophene, perhydroindazole, perhydroquinoline, perhydroisoquinoline, perhydrophthalazine, perhydronaphthyridine, perhydroquinoxaline, perhydroquinazoline, perhydrocinnoline, perhydrobenzoxazole, perhydrobenzothiazole, perhydrobenzimidazole, perhydrocarbazole, perhydroacridine, perhydrodibenzofuran, perhydrodibenzothiophene, dioxolan, dioxane, dithiolan, dithian,

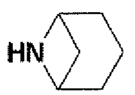 , 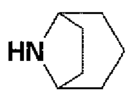 , 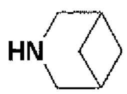 , 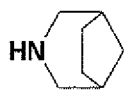 , or 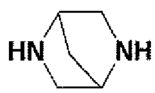

ring and the like.

Preferred examples of the "3- to 15-membered homocyclic or heterocyclic ring" represented by ring A and ring B include "5- to 10-membered homocyclic or heterocyclic ring". Specific examples of the 5- to 10-membered homocyclic ring include C5-10 saturated cyclic hydrocarbon, for example, C5-10 cycloalkane such as cyclopentane, cyclohexane, or cycloheptane, C5-10 cycloalkene such as cyclopentene, cyclohexene, cycloheptene, cyclooctene, cyclopentadiene, cyclohexadiene, cycloheptadiene, or cyclooctadiene, C5-10 unsaturated cyclic hydrocarbon such as benzene, naphthalene, or indene, and the like. Examples of the 5- to 10-membered heterocyclic ring include a 5- to 10-membered unsaturated heterocyclic ring such as pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzofurazane, benzothiadiazole, benzotriazole, pyrroline, imidazoline, triazoline, tetrazoline, pyrazoline, dihydropyridine, tetrahydropyridine, dihydropyrazine, tetrahydropyrazine, dihydropyrimidine, tetrahydropyrimidine, dihydropyridazine, tetrahydropyridazine, dihydroazepine, tetrahydroazepine, dihydrodiazepine, tetrahydrodiazepine, dihydrofuran, dihydropyran, dihydrooxepine, tetrahydrooxepine, dihydrothiophene, dihydrothiopyran, dihydrothiepine, tetrahydrothiepine, dihydrooxazole, dihyroisoxazole, dihydrothiazole, dihydroisothiazole, dihydrofurazane, dihydrooxadiazole, dihydrooxazine, dihydrooxadiazine, dihydrooxazepine, tetrahydrooxazepine, dihydrooxadiazepine, tetrahydrooxadiazepine, dihydrothiadiazole, dihydrothiazine, dihydrothiadiazine, dihydrothiazepine, tetrahydrothiazepine, dihydrothiadiazepine, tetrahydrothiadiazepine, indoline, isoindoline, dihydrobenzofuran, dihydroisobenzofuran, dihydrobenzothiophene, dihydroisobenzothiophene, dihydroindazole, dihydroquinoline, tetrahydroquinoline, dihydroisoquinoline, tetrahydroisoquinoline, dihydrophthalazine, tetrahydrophthalazine, dihydronaphthyridine, tetrahydronaphthyridine, dihydroquinoxaline, tetrahydroquinoxaline, dihydroquinazoline, tetrahydroquinazoline, dihydrocinnoline, tetrahydrocinnoline, benzoxathian, dihydrobenzoxazine, dihydrobenzothiazine, pyrazinomorpholine, dihydrobenzoxazole, dihydrobenzothiazole, dihydrobenzimidazole, dioxaindane, benzodioxane, chroman, benzodithiolan, or benzodithian, a 5- to 10-membered saturated heterocyclic ring such as pyrrolidine, imidazolidine, triazolidine, tetrazolidine, pyrazolidine, piperidine, piperazine, perhydropyrimidine, perhydropyridazine, perhydroazepine, perhydrodiazepine, tetrahydrofuran, tetrahydropyran, perhydrooxepine, tetrahydrothiophene, tetrahydrothiopyran, perhydrothiepine, tetrahydrooxazole (oxazolidine), tetrahydroisoxazole (isoxazolidine), tetrahydrothiazole (thiazolidine), tetrahydroisothiazole (isothiazolidine), tetrahydrofurazane, tetrahydrooxadiazole (oxadiazolidine), tetrahydrooxazine, tetrahydrooxadiazine, perhydrooxazepine, perhydrooxadiazepine, tetrahydrothiadiazole (thiadiazolidine), tetrahydrothiazine, tetrahydrothiadiazine, perhydrothiazepine, perhydrothiadiazepine, morpholine, thiomorpholine, oxathian, perhydrobenzofuran, perhydroisobenzofuran, perhydrobenzothiophene, perhydroisobenzothiophene, perhydroindazole, perhydroquinoline, perhydroisoquinoline, perhydrophthalazine, perhydronaphthyridine, perhydroquinoxaline, perhydroquinazoline, perhydrocinnoline, perhydrobenzoxazole, perhydrobenzothiazole, perhydrobenzimidazole, dioxolan, dioxane, dithiolan, dithian,

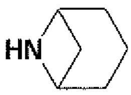 , 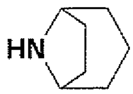 , 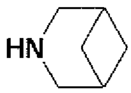 , 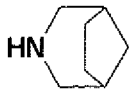 , or 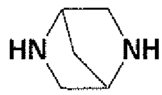

ring, and the like.

More preferably, a "5- to 10-membered unsaturated homocyclic or heterocyclic ring" can be exemplified as the ring A or ring B. The "5- to 10-membered unsaturated homocyclic or heterocyclic ring" means a "5- to 10-membered unsaturated cyclic hydrocarbon" or a "5- to 10-membered unsaturated heterocyclic ring". More preferred examples include a 5- or 6-membered aromatic ring such as benzene, pyrrole, imidazole, triazole, tetrazole, pyrazole, pyridine, pyrazine, pyrimidine, pyridazine, triazine, furan, thiophene, oxazole, isoxazole, thiazole, isothiazole, furazane, oxadiazole, or thiadiazole ring.

Regarding the "substituent" in the "3- to 15-membered homocyclic or heterocyclic ring which may have a substituent(s)" shown by the ring A and ring B, its examples include (1) a hydrocarbon group which may have a substituent(s) (this "hydrocarbon group which may have a substituent(s)" has the same meaning as the above-described "hydrocarbon group which may have a substituent(s)"), (2) alkoxy having from 1 to 6 carbon atoms which may be substituted with halogen, such as methoxy, ethoxy, propoxy, iso-propoxy, n-butoxy, iso-butoxy, sec-butoxy, tert-butoxy, or trifluoromethoxy, (3) (alkoxy having from 1 to 4 carbon atoms)-(alkyl having from 1 to 4 carbon atoms) such as methoxyethyl, (4) phenoxy, (5) alkanoyl having from 1 to 8 carbon atoms such as formyl, acetyl, propionyl, n-butyryl, iso-butyryl, or cyclohexylcarbonyl, (6) benzoyl, (7) alkanoyloxy having from 1 to 8 carbon atoms such as formyloxy, acetyloxy, propionyloxy, n-butyryloxy, iso-butyryloxy, or cyclohexylcarbonyloxy, or benzoyloxy, (8) carboxy, (9) alkoxycarbonyl having from 2 to 7 carbon atoms such as methoxycarbonyl, ethoxycarbonyl, n-propoxycarbonyl, isopropoxycarbonyl, n-butoxycarbonyl, iso-butoxycarbonyl, or tert-butoxycarbonyl, (10) carbamoyl, (11) N-mono-C1-4 alkylcarbamoyl such as N-methylcarbamoyl, N-ethylcarbamoyl, N-propylcarbamoyl, N-isopropylcarbarnoyl, or N-butylcarbamoyl, (12) N,N-di-C1-4 alkylcarbamoyl such as N,N-dimethylcarbamoyl, N,N-diethylcarbamoyl, N,N-dipropylcarbamoyl, or N,N-dibutylcarbamoyl, (13) cyclic aminocarbonyl such as 1-atiridinylcarbonyl, 1-azetidinylcarbonyl, 1-pyrrolidinylcarbonyl, 1-piperidinylcarbanyl, N-methylpiperazinylcarbonyl, or morpholinocarbonyl, (14) halogen such as fluorine, chlorine, bromine, or iodine, (15) mono-, di- or tri-halogeno-C1-4 alkyl such as chloromethyl, dichloromethyl, trifluoromethyl, or trifluoroethyl, (16) oxo, (17) amidino, (18) imino, (19) amino, (20) mono-C1-4 alkylamino such as methyl amino, ethylamino, propylamino, isopropylamino, or butylamino, (21) di-C1-4 alkylamino such as dimethylamino, diethylamino, dipropylamino, diisopropylamino, or dibutylamino, (22) 3- to 6-membered cyclic amino which may contain 1 to 3 hetero atoms selected from oxygen atom, sulfur atom, nitrogen atom and the like other than carbons and one nitrogen atom, such as atiridinyl, azetidinyl, pyrrolidinyl, pyrrolinyl, pyrrolyl, imidazolyl, pyrazolyl, imidazolidinyl, piperidino, morpholino, dihydropyridyl, pyridyl, N-methylpiperazinyl, or N-ethylpiperazinyl, (23) alkanoylamido having from 1 to 8 carbon atoms such as formamido, acetamido, trifluoroacetamido, propionylamido, butyrylamido, isobutyrylamido, or cyclohexylcarbonylamido, (24) benzamido, (25) carbamoylamino, (26) N-C1-4 alkylcarbamoylamino such as N-methylcarbamoylamino, N-ethylcarbamoylamino, N-propylcarbamoylamino, N-isopropylcarbamoylamino, or N-butylcarbamoylamino, (27) N,N-di-C1-4 alkylcarbamoylamino such as N,N-dimethylcarbamoylamino, N,N-diethylcarbamaylamino, N,N-dipropylcarbamoylamino, or N,N-dibutylcarbamoylamino, (28) alkylenedioxy having from 1 to 3 carbon atoms such as methylenedioxy or ethylenedioxy, (29) -B(OH)$_2$, (30) hydroxy, (31) epoxy, (32) nitro, (33) cyano, (34) mercapto, (35) sulfo, (36) sulfino, (37) phosphono, (38) sulfamoyl, (39) monoalkyl sulfamoyl having from 1 to 6 carbon atoms such as N-methylsulfamoyl, N-ethylsulfamoyl, N-propylsulfamoyl, N-isopropylsulfaanoyl, or N-butylsulfamoyl, (40) di-C1-4 alkyl sulfamoyl such as N,N-dimethylsulfamoyl, N,N-diethylsulfamoyl, N,N-dipropylsulfamoyl, or N,N-dibutylsulfamoyl, (41) alkylthio having from 1 to 6 carbon atoms such as methylthio, ethylthio, propylthio, isopropylthio, n-butylthio, sec-butylthio, or tert-butylthio, (42) phenylthio, (43) alkylsulfinyl having from 1 to 6 carbon atoms such as methylsulfinyl, ethylsulfinyl, propylsulfinyl, or butylsulfinyl, (44) phenylsulfinyl, (45) alkylsulfonyl having from 1 to 6 carbon atoms such as methylsulfonyl, ethylsulfonyl, propylsulfonyl, or butylsulfonyl, (46) phenylsulfonyl, (47) azido and the like. The ring A and ring B may have from 1 to 10 of the above-described substituents at substitutable positions on the cyclic groups. In addition, when the number of substituents is two or more, respective substituents may be the same or different from one another. Preferred substituents on the ring A and ring B include a hydrocarbon group which may have a substituent(s), alkoxy, carboxy, alkanoylamido and the like. A hydrocarbon group and alkoxy can be cited as more preferred ones.

The "nitrogen-containing heterocyclic ring " according to the "3- to 15-membered nitrogen-containing heterocyclic ring which may have a substituent(s)" shown by the ring D represents a heterocyclic ring which contains at least one nitrogen atom other than carbons and may further contain 1 to 3 hetero atoms selected from nitrogen atom, oxygen atom and sulfur atom. Examples of the "3- to 15-membered nitrogen-containing heterocyclic ring" include a "3- to 15-membered nitrogen-containing unsaturated heterocyclic ring" and a "3- to 15-membered nitrogen-containing saturated heterocyclic ring".

Examples of the "3- to 15-membered nitrogen-containing unsaturated heterocyclic ring" include pyrrole, imidazole, triazole, tetrazole, pyrazole, indole, isoindole, indazole, purine, benzimidazole, benzoazepine, benzodiazepine, benzotriazole, carbazole, β-carboline, phenothiazine, phenoxazine, perimidine, pyrroline, imidazoline, triazoline, tetrazoline, pyrazoline, dihydropyridine, tetrahydropyridine, dihydropyrazine, tetrahydropyrazine, dihydropyrimidine, tetrahydropyrimidine, dihydropyridazine, tetrahydropyridazine, dihydroazepine, tetrahydroazepine, dihydrodiazepine, tetrahydrodiazepine, dihydrooxazole, dihyroisoxazole, dihydrothiazole, dihydroisothiazole, dihydrofurazane, dihydrooxadiazole, dihydrooxazine, dihydrooxadiazine, dihydrooxazepine, tetrahydrooxazepine, dihydrooxadiazepine, tetrahydrooxadiazepine, dihydrothiadiazole, dihydrothiazine, dihydrothiadiazine, dihydrothiazepine, dihydrothiadiazepine, tetrahydrothiadiazepine, indoline, isoindoline, dihydroindazole, dihydroquinoline, tetrahydroquinoline, dihydroisoquinoline, tetrahydroisoquinoline, dihydrophthalazine, tetrahydrophthalazine, dihydronaphthyridine, tetrahydronaphthyridine, dihydroquinoxaline, tetrahydroquinoxaline, dihydroquinazoline, tetrahydroquinazoline, dihydrocinnoline, tetrahydrocinnoline, dihydrobenzoxazine, dihydrobenzothiazine, pyrazinomorpholine, dihydrobenzoxazole, dihydrobenzothiazole, dihydrobenzimidazole, dihydrobenzoazepine, tetrahydrobenzoazepine, dihydrobenzodiazepine, tetrahydrobenzodiazepine, dihydrobenzoxazepine, tetrahydrobenzoxazepine, dihydrocarbazole, tetrahydrocarbazole, dihydroacridine, tetrahydroacridine and the like. Also, examples of the "3- to 15-membered nitrogen-containing saturated heterocyclic ring" include aziridine, azetidine, azocane, pyrrolidine, imidazolidine, triazolidine, tetrazolidine, pyrazolidine, piperidine, piperazine, perhydropyrimidine, perhydropyridazine, perhydroazepine, perhydrodiazepine, tetrahydrooxazole (oxazolidine), tetrahydroisoxazole (isoxazolidine), tetrahydrothiazole (thiazolidine), tetrahydroisothiazole (isothiazolidine), tetrahydrofurazane, tetrahydrooxadiazole (oxadiazolidine), tetrahydrooxazine, tetrahydrooxadiazine, perhydrooxazepine, perhydrooxadiazepine, tetrahydrothiadiazole (thiadiazolidine), tetrahydrothiazine, tetrahydrothiadiazine, tetrahydrothiazepine, perhydrothiazepine, perhydrothiadiazepine, morpholine, thiomorpholine, perhydroindazole, perhydroquinoline, perhydroisoquinoline, perhydrophthalazine, perhydronaphthyridine, perhydroquinoxaline, perhydroquinazoline, perhydrocinnoline, perhydrobenzoxazole, perhydrobenzothiazole, perhydrobenzimidazole, perhydrocarbazole, perhydroacridine,

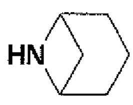 , 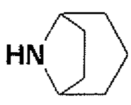 , 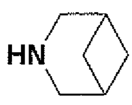 , 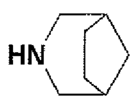 , or 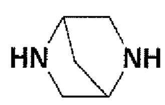

and the like.

Preferably, a "5- to 10-membered nitrogen-containing heterocyclic ring" can be cited as the "3- to 15-membered nitrogen-containing heterocyclic ring" shown by the ring D. Specific examples of the "5- to 10-membered nitrogen-containing unsaturated heterocyclic ring" include pyrrole, imidazole, triazole, tetrazole, pyrazole, indole, isoindole, indazole,

purine, benzimidazole, benzotriazole, pyrroline, imidazoline, triazoline, tetrazoline, pyrazoline, dihydropyridine, tetrahydropyridine, dihydropyrazine, tetrahydropyrazine, dihydropyrimidine, tetrahydropyrimidine, dihydropyridazine, tetrahydropyridazine, dihydroazepine, tetrahydroazepine, dihydrodiazepine, tetrahydrodiazepine, dihydrooxazole, dihydroisoxazole, dihydrothiazole, dihydroisothiazole, dihydrofurazane, dihydrooxadiazole, dihydrooxazine, dihydrooxadiazine, dihydrooxazepine, tetrahydrooxazepine, dihydrooxadiazepine, tetrahydrooxadiazepine, dihydrothiadiazole, dihydrothiazine, dihydrothiadiazine, dihydrothiazepine, tetrahydrothiazepine, dihydrothiadiazepine, tetrahydrothiadiazepine, indoline, isoindoline, dihydroindazole, dihydroquinoline, tetrahydroquinoline, dihydroisoquinoline, tetrahydroisoquinoline, dihydrophthalazine, tetrahydrophthalazine, dihydronaphthyridine, tetrahydronaphthyridine, dihydroquinoxaline, tetrahydroquinoxaline, dihydroquinazoline, tetrahydroquinazoline, dihydrocinnoline, tetrahydrocinnoline, dihydrobenzoxazine, dihydrobenzothiazine, pyrazinomorpholine, dihydrobenzoxazole, dihydrobenzothiazole, dihydrobenzimidazole and the like. Also, examples of the "5- to 10-membered nitrogen-containing saturated heterocyclic ring" include azocane, pyrrolidine, imidazolidine, triazolidine, tetrazolidine, pyrazolidine, piperidine, piperazine, perhydropyrimidine, perhydropyridazine, perhydroazepine, perhydrodiazepine, tetrahydrooxazole (oxazolidine), tetrahydroisoxazole (isoxazolidine), tetrahydrothiazole (thiazolidine), tetrahydroisothiazole (isothiazolidine), tetrahydrofurazane, tetrahydrooxadiazole (oxadiazolidine), tetrahydrooxazine, tetrahydrooxadiazine, perhydrooxazepine, perhydrooxadiazepine, tetrahydrothiadiazole (thiadiazolidine), tetrahydrothiazine, tetrahydrothiadiazine, perhydrothiazepine, perhydrothiadiazepine, morpholine, thiomorpholine, perhydroindazole, perhydroquinoline, perhydroisoquinoline, perhydrophthalazine, perhydronaphthyridine, perhydroquinoxaline, perhydroquinazoline, perhydrocinnoline, perhydrobenzoxazole, perhydrobenzothiazole, perhydrobenzimidazole,

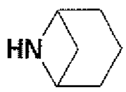 , 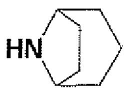 , 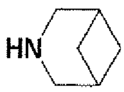 , 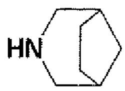 , or 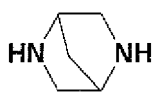

and the like.

In addition, as the "nitrogen-containing heterocyclic ring" shown by the ring D, piperidine or piperazine can be preferably cited.

The "substituent" in the "3- to 15-membered nitrogen-containing heterocyclic ring which may have a substituent(s)" shown by the ring D has the same meaning as the above-described "substituent" in the "3- to 15-membered nitrogen-containing heterocyclic ring which may have a substituent(s)" shown by the ring A and ring B.

The ring D is preferably unsubstituted, or substituted with a hydrocarbon group which may have a substituent(s), mona-C1-4 alkylamino, di-C1-4 alkylamino or the like. More preferred is an unsubstituted one.

The "hydrocarbon group" in the "hydrocarbon group which may have a substituent(s)" represented by $R^{2a}$ has the same meaning as the "hydrocarbon group which may have a substituent(s)" defined by the "protecting group" in the "acidic group which may be protected" represented by $R^{1a}$. Preferred as the "hydrocarbon group which may have a substituent(s)" represented by $R^{2a}$ is alkyl substituted with oxo or (cycloalkyl having from 3 to 8 carbon atoms)-(alkyl having from 1 to 4 carbon atoms) substituted with oxo.

Among $R^{2a}$, the "hydroxy which may have a substituent(s)" represents "hydroxy" which may be protected by a "protecting group", and as the "protecting group" of hydroxy, for example, (1) alkyl having from 1 to 6 carbon atoms such as methyl, ethyl, n-propyl, iso-propyl, n-butyl, or tert-butyl, which may have 1 to 4 substituent(s) selected from a halogen atom such as chlorine, bromine, or fluorine, aryl having from 6 to 10 carbon atoms such as phenyl or naphthyl, aralkyl having from 7 to 12 carbon atoms such as benzyl or phenylethyl, nitro and the like, (2) aryl having from 6 to 10 carbon atoms such as phenyl or naphthyl, which may have 1 to 4 substituent(s) selected from a halogen atom such as chlorine, bromine or fluorine, alkyl having from 1 to 6 carbon atoms such as methyl, ethyl, or n-propyl, aryl having from 6 to 10 carbon atoms such as phenyl or naphthyl, aralkyl having from 7 to 12 carbon atoms such as benzyl or phenylethyl, nitro and the like, (3) aralkyl having from 7 to 12 carbon atoms such as benzyl, phenylethyl, or naphthylmethyl, which may have 1 to 4 substituent(s) selected from a halogen atom such as chlorine, bromine, or fluorine, alkyl having from 1 to 6 carbon atoms such as methyl, ethyl, or n-propyl, aryl having from 6 to 10 carbon atoms such as phenyl or naphthyl, aralkyl having from 7 to 12 carbon atoms such as benzyl or phenylethyl, nitro and the like, (4) formyl, (5) alkylcarbonyl having from 1 to 6 carbon atoms such as acetyl or propionyl, which may have 1 to 3 substituent(s) selected from a halogen atom such as chlorine, bromine, or fluorine, alkyl having from 1 to 6 carbon atoms such as methyl, ethyl, or n-propyl, aryl having from 6 to 10 carbon atoms such as phenyl or naphthyl, aralkyl having from 7 to 12 carbon atoms such as benzyl or phenylethyl, nitro and the like, (6) aryloxycarbonyl having from 6 to 10 carbon atoms such as phenyloxycarbonyl or naphthyloxycarbonyl, which may have 1 to 4 substituent(s) selected from a halogen atom such as chlorine, bromine, or fluorine, alkyl having from 1 to 6 carbon atoms such as methyl, ethyl, or n-propyl, aryl having from 6 to 10 carbon atoms such as phenyl or naphthyl, aralkyl having from 7 to 12 carbon atoms such as benzyl or phenylethyl, nitro and the like, (7) arylcarbonyl having from 6 to 10 carbon atoms such as benzoyl or naphthylcarbonyl, which may

have 1 to 4 substituent(s) selected from a halogen atom such as chlorine, bromine, or fluorine, alkyl having from 1 to 6 carbon atoms such as methyl, ethyl, or n-propyl, aryl having from 6 to 10 carbon atoms such as phenyl or naphthyl, aralkyl having from 7 to 12 carbon atoms such as benzyl or phenylethyl, nitro and the like, (8) aralkyl-carbonyl having from 7 to 12 carbon atoms such as benzylcarbonyl or phenethylcarbonyl, which may have 1 to 4 substituent(s) selected from a halogen atom such as chlorine, bromine, or fluorine, alkyl having from 1 to 6 carbon atoms such as methyl, ethyl, or n-propyl, aryl having from 6 to 10 carbon atoms such as phenyl or naphthyl, aralkyl having from 7 to 12 carbon atoms such as benzyl or phenylethyl, nitro and the like, (9) pyranyl or furanyl, which may have 1 to 4 substituent(s) selected from a halogen atom such as chlorine, bromine, or fluorine, alkyl having from 1 to 6 carbon atoms such as methyl, ethyl, or n-propyl, aryl having from 6 to 10 carbon atoms such as phenyl or naphthyl, aralkyl having from 7 to 12 carbon atoms such as benzyl or phenylethyl, nitro and the like, (10) tri-C1-4 alkylsilyl such as trimethylsilyl or triethylsilyl, and the like.

Examples of the "substituent" in the "amino which may have a substituent(s)" represented by $R^{2a}$ include a hydrocarbon group which may have a substituent(s), $-SO_2R^{201a}$, $=NR^{202a}$, $-OR^{203a}$ (in the formulae, each of $R^{201a}$ to $R^{303a}$ is a hydrocarbon group which may have a substituent(s)) and the like. In this connection, the "hydrocarbon group which may have a substituent(s)" has the same meaning as the "hydrocarbon group which may have a substituent(s)" defined by the "protecting group" in the "acidic group which may be protected" represented by $R^{1a}$. Preferred as the "substituent" of the "amino which may have a substituent(s)" represented by $R^{2a}$ is a hydrocarbon group which may have a substituent(s).

The "3- to 15-membered heterocyclic group which may have a substituent(s)" represented by $R^{2a}$ has the same meaning as the "3- to 15-membered heterocyclic group which may have a substituent(s)" represented by the ring A and ring B. Preferred examples of the "3- to 15-membered heterocyclic group which may have a substituent(s)" represented by $R^{2a}$ include a piperidine or piperazine ring which may have a substituent(s), and more preferred examples include

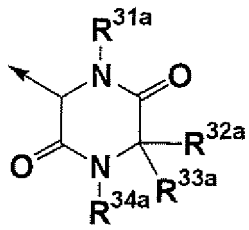

(in the formula, the arrow shows the binding position with ring D, and $R^{31a}$, $R^{32a}$, $R^{33a}$ and $R^{34a}$ each independently has the same meaning as the "substituent" of the "3- to 15-membered heterocyclic group which may have a substituent(s)" represented by the ring A and ring B), or the like.

A hydrocarbon group which may have a substituent(s), amino which may have a substituent(s) and the like can, for example, be exemplified as preferred $R^{2a}$. More preferably,

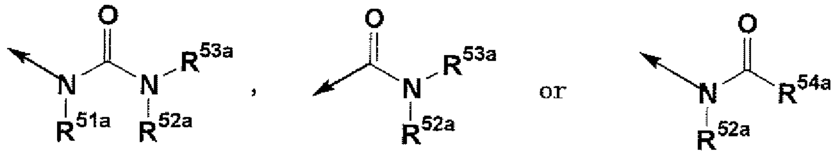

(in the formula, the arrow shows binding position with ring D, and $R^{51a}$, $R^{52a}$, $R^{53a}$ and $R^{54a}$ each independently represents a hydrogen atom, a hydrocarbon group which may have a substituent(s), a 3- to 15-membered heterocyclic group which may have a substituent(s), a C1-4 alkoxy which may have a substituent(s), phenoxy which may have a substituent(s) or benzyloxy which may have a substituent(s)) and the like can be exemplified. In this connection, the "hydrocarbon group which may have a substituent(s)" and "3- to 15-membered heterocyclic group which may have a substituent(s)" have the same respective meanings as described in the foregoing. Examples of the C1-4 alkoxy include methoxy, ethoxy, propoxy, isopropoxy, butoxy, isobutoxy, s-butoxy, t- butoxy and the like. In this connection, the C1-4 alkoxy, phenoxy or benzyloxy may have an optional substituent. The above-described "substituent" in the "hydrocarbon group which may have a substituent(s)" and the like can be exemplified as the substituent of C1-4 alkoxy, phenoxy or benzyloxy.

Preferred examples of the $R^{51a}$, $R^{52a}$, $R^{53a}$ and $R^{54a}$ include a hydrogen atom, a hydrocarbon group which may have a substituent(s), a 3- to 15-membered heterocyclic group which may have a substituent(s) and the like. Also, a compound in which either one of $R^{52a}$ and $R^{53a}$ is a hydrogen atom is desirable.

According to the present invention, a compound of formula (II) which comprises a combination of the above-described preferred groups and preferred rings is desirable. Examples include a compound in which ring D is piperidine or piperazine and Y is methylene, namely a compound represented by formula (Ia)

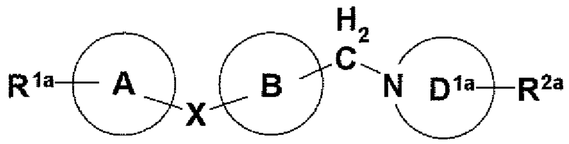

(Ia)

(in the formula, ring $D^{1a}$ represents piperidine or piperazine which may have a substituent(s), and other symbols have the same meanings as defined above), a compound in which ring D is piperidine or piperazine and $R^{2a}$ is

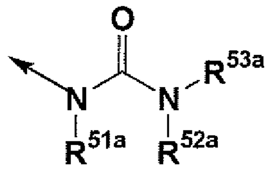

namely a compound represented by formula (Ib)

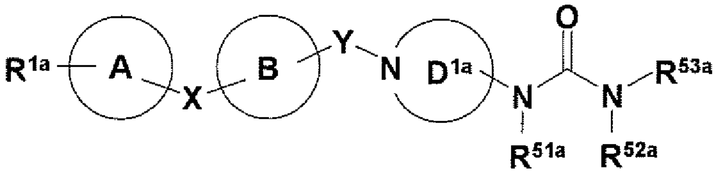

(Ib)

(in the formula, all symbols have the same meanings as defined above), a compound in which $R^{1a}$ is $-SO_2NR^{102a}R^{103a}$ or $-NR^{101a}SO_2R^{104a}$, X is a single bond, $-CR^{7a}R^{8a}$- $-NR^{9a}$-, -CO-, -O-, -S-, -SO-, $-SO_2$- or $-C(=N-OR^{10a})$- (in the formulae, $R^{7a}$ and $R^{8a}$ each independently represents a hydrogen atom, C1-4 alkyl, $-OR^{11a}$ or phenyl, $R^{9a}$ represents a hydrogen atom, C1-4 alkyl or phenyl, and $R^{10a}$ and $R^{11a}$ each independently represents a hydrogen atom or C1-4 alkyl), Y is methylene, ring A and ring B are each independently a benzene ring which may be substituted, ring D is piperidine, and $R^{2a}$ is

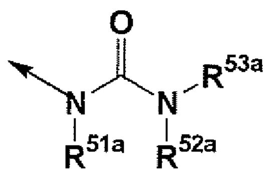

namely a compound represented by a formula (Ic)

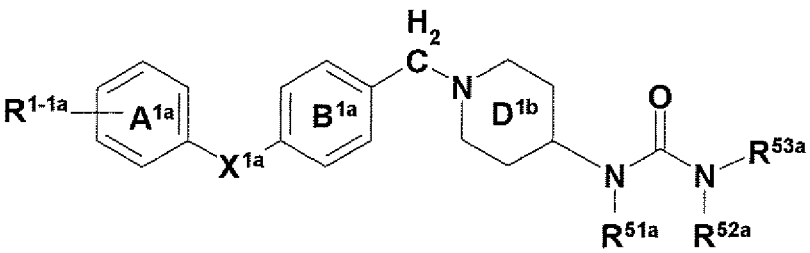

(Ic)

(in the formula, $R^{1-1a}$ is $-SO_2NR^{102a}R^{103a}$ or $NR^{101a}SO_2R^{104a}$, $X^{1a}$ is a single bond, $-CR^{7a}R^{8a}$-, $-NR^{9a}$-, -CO-, -O-, -S-,

-SO-, $-SO_2-$ or $-C(=N-OR^{10a})-$ (in the formulae, $R^{7a}$ and $R^{8a}$ each independently represents a hydrogen atom, C1-4 alkyl, $-OR^{11a}$ or phenyl, $R^{9a}$ represents a hydrogen atom, C1-4 alkyl or phenyl, and $R^{10a}$ and $R^{11a}$ each independently represents a hydrogen atom or C1-4 alkyl), ring $A^{1a}$ and ring $B^{1a}$ each independently represents benzene ring which may have a substituent(s), ring $D^{1b}$ represents piperidine which may have a substituent(s), and other symbols have the same meanings as defined above), a compound in which $R^{1a}$ is $-SO_2NR^{10a}R^{103a}$ or $-NR^{101a}SO_2R^{104a}$, X is a single bond, $-CR^{7a}R^{8a}-$ $-NR^{9a}-$, -CO-, -O-, -S-, -SO-, $-SO_2-$ or $-C(=N-OR^{10a})-$ (in the formulae, $R^{7a}$ and $R^{8a}$ each independently represents a hydrogen atom, C1-4 alkyl, $-OR^{11a}$ or phenyl, $R^{9a}$ represents a hydrogen atom, C1-4 alkyl or phenyl, and $R^{10a}$ and $R^{11a}$ each independently represents a hydrogen atom or C1-4 alkyl), Y is methylene, ring A and ring B each independently represents benzene ring or an unsaturated monocyclic hetero ring, which may be substituted, ring D is piperidine or piperazine, and $R^{2a}$ is

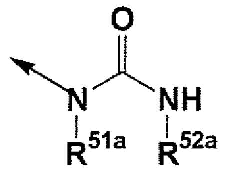

namely a compound represented by a formula (Id)

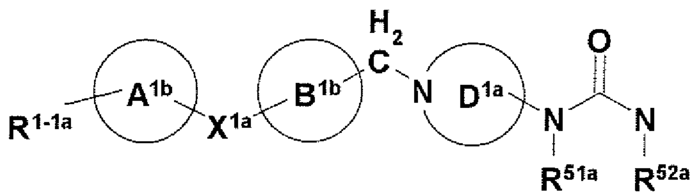

(Id)

(in the formula, ring $A^{1b}$ and ring $B^{1b}$ each independently represents a benzene ring or a 5- or 6-membered aromatic ring, which may be substituted, and other symbols have the same meanings as defined above) and the like.

Among compounds represented by formula (II) to be used as CCR5 antagonists, preferred compounds include:

(1) 2-[3-methyl-4-(4-{4-[(methylsulfonyl)amino]phenoxy}benzyl)piperazin-1-yl]-N-phenylhexaneamide,
(2) N-{4-[4-({4-[(anilinocarbonyl)(butyl)amino]-4'-methyl-1,4'-bipiperidin-1'-yl}methyl)phenoxy]phenyl}methanesulfonamide,
(3) N-[4-(4-{[3-[(anilinocarbonyl)(butyl)amino]-4-(3-fluorophenyl)pyrrolidin-1-yl]methyl}phenoxy)phenyl]methanesulfonamide,
(4) N-[4-(4-{[3-(butylamino)-4-(3-fluorophenyl)pyrrolidin-1-yl]methyl}phenoxy)phenyl]methanesulfonamide,
(5) N-butyl-N-(1-{3-ethyl-1-[4-(methylsulfonyl)benzyl]-1H-pyrazol-4-yl}piperidin-4-yl)-N'-phenylurea,
(6) N-butyl-N-[1-({4-methyl-2-[4-(trifluoromethyl)phenyl]-1H-imidazol-5-yl}methyl)piperidin-4-yl]-N'-phenylurea,
(7) N-{4-[4-({3-[(anilinocarbonyl)(butyl)amino]-8-azabicyclo[3.2.1]octan-8-yl}methyl)phenoxy]phenyl}methanesulfonamide,
(8) N-[4-(4-{[4-(3-isopropyl-5-methyl-4H-1,2,4-triazol-4-yl)piperidin-1-yl]methyl}phenoxy)phenyl]methanesulfonamide,
(9) N-[4-(4-{[4-(2-methyl-1H-benzimidazol-1-yl)piperidin-1-yl]methyl}phenoxy)phenyl]methanesulfonamide,
(10) N-[4-(4-{[4-[(anilinocarbonyl)(butyl)amino]-3,4-dihydroquinoline-1(2H)-yl]methyl}phenoxy)phenyl]methanesulfonamide,
(11) N-[4-(4-{[4-(2-oxo-3-phenyl-6-propyltetrahydropyrimidine-1(2H)-yl)piperidin-1-yl]methyl}phenoxy)phenyl]methanesulfonamide,
(12) N-(4-{4-[(3-butyl-2-oxo-1,2,3,3a,4,5-hexahydro-6H-pyrido[4,3,2-de]quinazolin-6-yl)methyl]phenoxy}phenyl)-methanesulfonamide,
(13) N-(4-{4-[(1-butyl-2-oxo-4-phenyloctahydropyrido[4,3-d]pyrimidine-6(2H)-yl)methyl]phenoxy}phenyl)methanesulfonamide,
(14) N-(4-[4-({8-[(anilinocarbonyl)(butyl)amino]-3-azabicyclo[3.2.1]octan-3-yl}methyl)phenoxy]phenyl)methanesulfonamide,
(15) N-[4-(4-{[(22)-1-butyl-2-(phenylimino)hexahydro-2H-pyrido[4,3-d][1,3]oxazine-s(4H)-yl]methyl}phenoxy)phenyl]methanesulfonamide, and

(16) N-[7-({4-[(anilinocarbonyl)(butyl)amino]piperidin-1-yl}methyl)-9H-xanthen-2-yl]methanesulfonamide, and more preferred examples include:

(1) N-[4-(4-{[4-(butyl{[(2,4-difluorophenyl)amino]carbonyl}amino)-1-piperidinyl]methyl}phenoxy)phenyl]methanesulfonamide,
(2) N-[4-(4-{[4-(butyl{[(6-methyl-3-pyridinyl)amino]carbonyl}amino)-1-piperidinyl]methyl}phenoxy)phenyl]methanesulfonamide,
(3) N-[4-(4-{[4-(butyl{[(2,4-difluorophenyl)amino]carbonyl}amino)piperidin-1-yl]methyl}-3,5-dimethyl-1H-pyrazol-1-yl)phenyl]methanesulfonamide,
(4) N-[4-(4-{[4-(butyl{[(1-methyl-1H-pyrazol-4-yl)amino]carbonyl}amino)piperidin-1-yl]methyl}phenoxy)phenyl]methanesulfonamide,
(5) 3-[({butyl[1-(4-{4-[(methylsulfonyl)amino]phenoxy}benzyl)piperidin-4-yl]amino}carbonyl)amino]benzamide,
(6) N-{4-[4-({4-[{[(4-fluorophenyl)amino]carbonyl}(phenyl)amino]piperidin-1-yl}methyl)phenoxy]phenyl}methanesulfonamide,
(7) 5-[({butyl[1-(4-{4-[(methylsulfonyl)amino]phenoxy}benzyl)piperidin-4-yl]amino}carbonyl)amino]-2-fluorobenzamide,
(8) 5-[({butyl[1-(4-{4-[(methylsulfanyl)amino]phenoxy}benzyl)piperidin-4-yl]amino}carbonyl)amino]-2,4-difluorobenzamide,
(9) N-[4-(4-{[4-(butyl{[(3-cyano-4-fluorophenyl)amino]carbonyl}amino)piperidin-1-yl]methyl}phenoxy)phenyl]-methanesulfonamide,
(10) N-[4-(4-{[4-(butyl{[(3-hydroxycyclohexyl)amino]carbonyl}amino)piperidin-1-yl]methyl}phenoxy)phenyl]-methanesulfonamide, and
(11) N-14-[4-({4-[{[(4-fluorophenyl)amino]carbonyl}(1,3-thiazol-4-ylmethyl)amino]piperidin-1-yl}methyl)phenoxy]phenyl}methanesulfonamide.

Pharmacological activity:

As a pharmacological test other than those described in Examples, for example, there is a method shown below. By the method shown below, efficacy of the compound of the present invention in in vivo transplantation model can be proved. Immunosuppression action of CCR5 antagonist in monkey kidney transplantation model:
Cynomolgus monkey individuals (body weight: 3 to 4.5 kg) having the same ABO blood type and different major histocompatibility complex (MHC) (MLR miss-matched) are used in combination of donor (male) and recipient (either male or female). Both kidneys of a recipient are extracted, and one kidney derived from a donor is transplanted into the recipient. Administration of the compound to be tested (CCR5 antagonist and/or immunosuppressant) is started on the preceding day (Day -1) of the day of transplantation (Day 1) and continued every day for a maximum of 30 days until the rejection is confirmed, and the number of days of adhesion of the transplanted kidney is used as the object of evaluation.
Regarding the evaluation method, for example, there is a method in which a CCR5 antagonist is administered in combination with an already known immunosuppressant (cyclosporin, sirolimus and/or tacrolimus) and compared with a group of immunosuppressant alone.
Regarding the administration mode of each CCR5 antagonist, for example, there is a method in which 30 mg/kg of the CCR5 antagonist is orally administered twice a day. Also, regarding the administration mode of each immunosuppressant, for example, there is a method in which it is administered by intramuscular injection, and the dose is gradually reduced. For example, a method of Day -1: 12.5 (mg/kg), Day 1 to 6: 10.0 (mg/kg), Day 7 to 13: 5.0 (mg/kg), Day 14 to 20: 2.5 (mg/kg), Day 21 to 28: 0 (mg/kg) in the case of cyclosporin, or Day -1: 0.1 (mg/kg), Day 1 to 6: 0.075 (mg/kg), Day 7 to 13: 0.05 (mg/kg), Day 14 to 20: 0.025 (mg/kg), Day 21 to 28: 0 (mg/kg) in the case, for example, of sirolimus can be exemplified.
In addition, as the judging standard, the blood creatinine value can, for example, be used as the index. Specifically, when the blood creatinine value exceeded 8 mg/dl for example, it can be judged that the kidney was rejected.

Application to medicaments:

Since the CCR5 antagonist of the present invention inhibits the function of effector cells, it is effective for the prevention and/or treatment of, for example, a transplant rejection (e.g., rejection of a solid organ graft, rejection of islet cell transplantation in diabetes mellitus, graft-versus-host disease (GVHD), *etc.),* an autoimmune disease (e.g., arthritis, rheumatoid arthritis, multiple sclerosis, ulcerative colitis, *etc.),* an allergic disease (e.g., asthma, *etc.*), an ischemic disease (e.g., ischemia-reperfusion injury, *etc.)* and the like in animals including human, particularly in human.
Since the CCR5 antagonist of the present invention is safe and has low toxicity, it can be administered, for

example, to human and mammals (e.g., rat, mouse, rabbit, sheep, pig, cattle, cat, dog, monkey, *etc.*).

When the CCR5 antagonist of the present invention, or a combination preparation of the CCR5 antagonist of the present invention and other concomitant drug, is used for the above-described purpose, generally, it is systemically or topically administered orally or parenterally.

In addition, according to the present invention, the CCR5 antagonist may be administered as a combination drug in combination with other drug for the purpose of;

1) complementing and/or reinforcing preventive and/or therapeutic effect of the compound,
2) improving dynamics and absorption of the compound and reducing the dose, and/or
3) reducing side effects of the compound.

In addition, for the purpose of (1) complementing and/or reinforcing preventive and/or therapeutic effect, (2) improving dynamics and absorption, and/or (3) reducing side effects, of the other drug to be jointly used, it may be administered as a combination drug in combination with the compound of the present invention.

The combination drug of the CCR5 antagonist with other drug may be administered in the form of a combination drug in which both components are formulated in one pharmaceutical preparation, or in another form in which they are administered as separate preparations. When administered as separate preparations, simultaneous administration and differential time administration are included. In addition, in the case of the differential time administration, the CCR5 antagonist may be firstly administered followed by the administration of the other drug, or the other drug may be firstly administered followed by the administration of the CCR5 antagonist, and respective administration methods may be the same or different.

The diseases in which their preventive and/or therapeutic effects are exerted by the above-described combination drug are not particularly limited, and they may be the diseases in which preventive and/or therapeutic effect of the CCR5 antagonist is complemented and/or reinforced.

The present invention includes those which reinforce preventive and/or therapeutic effect of the disease of interest, in comparison with a single preparation, through the combination of the CCR5 antagonist with a compound that does not have the preventive and/or therapeutic effect of the disease of interest.

As examples of the other drug to be used in combination with the CCR5 antagonist of the present invention, immunosuppressants can, for example, be cited as the drugs to be used in the prevention and/or treatment of transplant rejection. Examples of those which are used in the prevention and/or treatment of autoimmune diseases include non-steroidal anti-inflammatory drugs, disease modifying anti-rheumatoid drugs (DMARDs, delayed effect anti-rheumatoid drugs), steroid drugs, immunosuppressants, anti-inflammatory enzyme preparations, cartilage protectors, T cell inhibitors, TNF$\alpha$ inhibitors (including protein preparations such as anti-TNF$\alpha$ antibody, *etc.*), prostaglandin synthase inhibitors, IL-6 inhibitors (including protein preparations such as anti-IL-6 receptor antibody, *etc.*), interferon y agonists, IL-1 inhibitors, prostaglandins, phosphodiesterase inhibitors, metalloproteinases and the like.

Examples of those which are used in the prevention and/or treatment of ischemic diseases include radical scavengers, astrocyte modulators, N-methyl-D-aspartate (NMDA) antagonists, alpha-amino-3-hydroxy-5-methylisoxazole-4-propionate (AMPA) antagonists, anti-thrombotic agents, thrombolytic agents, immunosuppressants, intercellular adhesion molecule inhibitors, nitric oxide synthase (NOS) inhibitors, neurotrophic factors, interleukin-8 antagonists and the like.

Examples of those which are used in the prevention and/or treatment of allergic diseases, in the case of asthma, include steroid drugs, $\beta_2$ adrenalin receptor stimulants, leukotriene receptor antagonists, thromboxane synthase inhibitors, thromboxane $A_2$ receptor antagonists, mediator release inhibitors, antihistaminics, xanthine derivatives, anticholinergics, cytokine inhibitors, prostaglandins, forskolin preparations, phosphodiesterase inhibitors, elastase inhibitors, metalloproteinase inhibitors, expectorants, antibiotics and the like.

Examples of the immunosuppressants include tacrolimus (FK506), cyclosporin, sirolimus (rapamycin), corticosteroid, azathioprine, mycophenolate mofetil, FTY-720, cyclophosphamide and the like.

Regarding the steroid drugs, in the case of external preparations, examples include clobetasol propionate, diflorasone diacetate, fluocinonide, mometasone furancarboxylate, betamethasone dipropionate, betamethasone butyrate propionate, betamethasone valerate, difluprednate, budesonide, diflucortolone valerate, amcinonide, halcinonide, dexamethasone, dexamethasone acetate, hydrocortisone acetate, hydrocortisone butyrate, hydrocortisone butyrate propionate, deprodone propionate, prednisolone valerate acetate, fluocinolone acetonide, beclometasone propionate, triamcinolone acetonide, flumetasone pivalate, alclometasone dipropionate, clobetasone butyrate, prednisolone, beclomethasone dipropionate, fludroxycortide and the like. Examples of internal medicines and injections include cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone diacetate, triamcinolone acetonide, dexamethasone, dexamethasone acetate, dexamethasone sodium phosphate, dexamethasone palmitate, paramethasone acetate, betamethasone and the like. Examples of inhalations include beclometasone dipropionate, fluticasone propionate, budesonide, flunisolide, triamcinolone, ST-126P, ciclesonide, dexamethasone palmitate, mometasone furoate, sodium prasterone sulfate, deflazacort, methylprednisolone suleptanate, methylprednisolone sodium succinate and the like.

Examples of the $\beta_2$ adrenaline receptor stimulants include fenoterol hydrobromide, salbutamol sulfate, terbutaline sulfate, formoterol fumarate, salmeterol xinafoate, isoproterenol sulfate, orciprenaline sulfate, chlorprenaline sulfate, epinephrine, trimetoquinol hydrochloride, hexoprenaline mesyl sulfate, procaterol hydrochloride, tulobuterol hydrochloride, tulobuterol, pirbuterol hydrochloride, clenbuterol hydrochloride, mabuterol hydrochloride, ritodrine hydrochloride, bambuteral, dopexamine hydrochloride, meruadrine tartarate, AR-C68397, levosalbutamol, R,R-formoterol, KUR-1246, KUL-7211, AR-C89855, S-1319 and the like.

Examples of the leukotriene receptor antagonists include planlukast hydrate, montelukast, zafirlukast, seratrodast, MCC-847, KCA-757, CS-615, YM-158, L-740515, CP-195494, LM-1484, RS-635, A-93178, S-36496, BIIL-284, ONO-4057 and the like.

Examples of the thromboxane synthase inhibitors include ozagrel hydrochloride, imitrodast sodium and the like.

Examples of the thromboxane A2 receptor antagonist include seratrodast, ramatroban, domitroban calcium hydrate, KT-2-962 and the like.

Examples of the mediator release inhibitors include tranilast, cromoglicic acid sodium, amlexanox, repirinast, ibudilast, tazanolast, pemirolast potassium and the like.

Examples of the antihistaminics include ketotifen fumarate, mequitazine, azelastine hydrochloride, oxatomide, terfenadine, emedastine difumarate, epinastine hydrochloride, astemizole, ebastine, cetirizine hydrochloride, bepotastine, fexofenadine, loratadine, desloratadine, olopatadin hydrochloride, TAK-427, ZCR-2060, NIP-530, mometasone furoate, mizolastine, BP-294, andlast, auranofin, acrivastine and the like.

Examples of the xanthine derivatives include aminophylline, theophylline, doxofylline, sipamphylline, diprophylline and the like.

Examples of the anticholinergics include ipratropium bromide, oxitropium bromide, flutropium bromide, cimetropium bromide, temibelline, thiotropium bromide, revatropate (UK-112166) and the like.

Examples of the cytokine inhibitors include suplatast tosylate (trade name IPD) and the like.

Examples of the prostaglandins (to be referred to as PG hereinafter) include PG receptor agonists, PG receptor antagonists and the like. Examples of the PG receptor include PGE receptors (EP1, EP2, EP3, EP4), PGD receptors (DP, CRTH2), PGF receptor (FP), PGI receptor (IP), TX receptor (TP) and the like.

Examples of the phosphodiesterase inhibitors include PDE4 inhibitors rolipram, cilomilast (trade name Ariflo), Bay19-8004, NIK-616, roflumilast (BY-217), cipamfylline (BRL-61063), atizoram (CP-80633), SCH-351591, YM-976, V-11294A, PD-168787, D-4396, IC-485, ONO-6125 and the like.

Examples of the elastase inhibitors, ONO-5046, ONO-6818, MR-889, PBI-1101, EPI-HNE-4, R-665, ZD-0892, ZD-8321, GW-311616, AE-3763 and the like.

Examples of the expectorants include foeniculated ammonia spirit, sodium bicarbonate, bromhexine hydrochloride, carbocysteine, ambroxol hydrochloride, ambroxol sustained release preparation, methylcysteine hydrochloride, acetylcysteine, ethyl L-cysteine hydrochloride, tyloxapol and the like.

Examples of the non-steroidal anti-inflammatory drugs include sasapyrine, sodium salicylate, aspirin, aspirin dialuminate combinations, diflunisal, indomethacin, suprofen, ufenamate, dimethyl-isopropyl-azulene, bufexamac, felbinac, diclofenac, tolmetin sodium, clinoril, fenbufen, napumetone, proglumetacin, indomethacin, farnesil, acemetacin, proglumetacin maleate, amfenac sodium, mofezolac, etodolac, ibuprofen, ibuprofen piconol, naproxen, flurbiprofen, flurbiprofen axetil, ketoprofen, fenoprofen calcium, tiaprofen, oxaprozin, planoprofen, loxoprofen sodium, alminoprofen, zaltoprofen, mefenamic acid, aluminum mefenamate, tolfenamic acid, floctafenine, ketophenylbutazone, oxyphenbutazone, pyroxicam, tenoxicam, ampiroxicam, Napageln ointment, epirizole, tiaramide hydrochloride, tinoridine hydrochloride, emorfazone, sulpyrine, migrenin, Saridon, Sedes G, Amipylo-N, sorbon, pyrine cold remedies, acataminophen, phenacetin, dimethotiazine mesilate, simetride combinations, non-pilin cold remedies and the like.

Examples of the disease modifying anti-rheumatoid drugs (DMARDs, delayed effect anti-rheumatoid drugs) include aurothioglucose, sodium aurothiomalate, auranofin, actarit, D-penicillamine preparation, lobenzarit disodium, bucillamine, hydroxychloroquine, salazosulfapyridine, methotrexate, leflunomide and the like.

Examples of the cartilage protectors include sodium hyaluronate, glucosamine, chondroitin sulfate, glycosaminoglycan polysulfate and the like.

Examples of the prostaglandin synthase inhibitors include salazosulfapyridine, mesalazine, osalazine, 4-aminosalicylic acid, JTE-522, auranofin, carprofen, difenpiramide, flunoxaprofen, flurbiprofen, indomethacin, ketoprofen, lornoxicam, meloxicam, oxaprozin, parsalmide, piproxen, piroxicam, piroxicam betadex, piroxicam cinnamate, tropine indomethacinate, zaltoprofen, pranoprofen and the like.

Examples of the radical scavengers include radicut and the like.

Examples of the astrocyte modulators include ONO-2506 and the like.

Examples of the anti-thrombus agents include cataclot, argatroban, aspirin and the like.

Examples of the thrombolytic agents include human tissue plasminogen activation factor (t-PA), urokinase, heparin and the like.

Examples of the anti-inflammatory enzyme preparations include, lysozyme chloride, bromelain, pronase, serrapeptase, streptokinase streptodornase combination preparation and the like.

Examples of the TNF$\alpha$ inhibitors (including protein preparations such as anti-TNF$\alpha$ antibody, *etc.*) include infliximab, adalimumab, etanercept and the like.

Examples of the IL-6 inhibitors (including protein preparations such as anti-IL-6 receptor antibody, *etc.*) include MRA and the like.

Examples of the LL-1 inhibitors (including protein preparations such as human Il-1 receptor antagonist, *etc.*) include anakinra and the like.

Examples of the antibiotics include cefuroxime sodium, meropenem trihydrate, netilmicin sulfate, sisomicin sulfate, ceftibuten, PA-1806, IB-367, tobramycin, PA-1420, doxorubicin, astromicin sulfate, cefetamet pivoxil hydrochoride and the like. Examples of the inhalation antibiotics include PA-1806, IB-367, tobramycin, PA-1420, doxorubicin, astromicin sulfate, cefetamet pivoxil hydrochoride and the like.

Mass ratio of the jointly used CCR5 antagonist and other drug is not particularly limited.

The other drug may be administered as a combination of optional two or more species.

Also, the other drug which complement and/or reinforce the preventive and/or therapeutic effect of CCR5 antagonist is not limited to those exemplified in the above. In addition, not only those which have so far been found but also will be found based on the above-described mechanism are included in the other drug which complement and/or reinforce the preventive and/or therapeutic effect of CCR5 antagonist.

Clinical dose of the compound represented by formula (I) varies dependent on the age, body weight, symptoms, therapeutic effect, administration method, treating period and the like, but in general, it is orally administered once in several days, once in 3 days, once in 2 days or once or several times a day, within the range of 1 ng to 1000 mg per once per one adult, or parenterally administered (preferably by intravenous administration) once in several days, once in 3 days, once in 2 days or once or several times a day, within the range of 1 ng to 100 mg per once per one adult, or continuously administered through a vein within the range of 1 hour to 24 hours a day.

Since the dose varies under various conditions as a matter of course as described in the foregoing, a smaller dose than the above range may be sufficient enough in some cases, or administration exceeding the above range may be necessary in some cases.

When the CCR5 antagonist of the present invention or a combination drug of the CCR5 antagonist of the present invention and other drug is administered, it is used as solid compositions for internal use, liquid compositions for internal use, and injections, external preparations, suppositories, eye drops, inhalations and the like for parenteral administration.

Tablets, pills, capsules, powders, granules and the like are included in the solid compositions for internal use for use in the oral administration. Hard capsules and soft capsules are included in the capsules. Also, sublingual tablets, buccal adhesive tablets, buccal quick disintegration tablets and the like are included in the tablets.

In such solid compositions for internal use, one or more active substance(s) are directly used or after making into pharmaceutical preparations by the law of the art by mixing with an excipient (lactose, mannitol, glucose, microcrystalline cellulose, starch, *etc.*), a binder (hydroxypropylcellulose, polyvinylpyrrolidone, magnesium aluminometasilicate, *etc.*), a disintegrant (cellulose calcium glycolate, *etc.*), a lubricant (magnesium stearate, *etc.*), a stabilizing agent, a solubilizing agent (glutamic acid, aspartic acid, *etc.*) and the like. If necessary, they may be coated with a coating agent (sucrose, gelatin, hydroxypropylcellulose, hydroxypropylmethylcellulose phthalate, *etc.*), or coated with two or more layers. In addition, a capsule of an absorbable substance such as gelatin is also included therein.

The sublingual tablets are produced in accordance with a conventionally known method. For example, one, two or more active substances are used after making into pharmaceutical preparations by the law of the art by mixing with an excipient (lactose, mannitol, glucose, microcrystalline cellulose, colloidal silica, starch, *etc.),* a binder (hydroxypropylcellulose, polyvinylpyrrolidone, magnesium aluminometasilicate, *etc.),* a disintegrant (starch, L-hydroxypropylcellulose, carboxymethylcellulose, croscarmellose sodium, cellulose calcium glycolate, *etc.),* a lubricant (magnesium stearate, *etc.),* a swelling agent (hydroxypropylcellulose, hydroxypropylmethylcellulose, carbopol, carboxymethylcellulose, polyvinyl alcohol, xanthan gum, guar gum, *etc.),* a swelling adjuvant (glucose, fructose, mannitol, xylitol, erythritol, maltose, trehalose, phosphate, citrate, silicate, glycine, glutamic acid, arginine, *etc.*), a stabilizing agent, a solubilizing agent (polyethylene glycol, propylene glycol, glutamic acid, aspartic acid, *etc.*), a flavoring agent (orange, strawberry, mint, lemon, vanilla, *etc.*) and the like. Also, if necessary, they may be coated with a coating agent (sucrose, gelatin, hydroxypropylcellulose, hydroxypropylmethylcellulose phthalate, *etc.*), or coated with two or more layers. In addition, if necessary, a preservative, an antioxidant, a colorant, a sweetening agent and the like generally used additive agents can also be added thereto. The buccal adhesive tablets are produced in accordance with a conventionally known method. For example, one, two or more active substances are used after making into pharmaceutical preparations by the law of

the art by mixing with an excipient (lactose, mannitol, glucose, microcrystalline cellulose, colloidal silica, starch, *etc.*), a binder (hydroxypropylcellulose, polyvinylpyrrolidone, magnesium aluminometasilicate, *etc.),* a disintegrant (starch, L-hydroxypropylcellulose, carboxymethylcellulose, croscarmellose sodium, cellulose calcium glycolate, *etc.),* a lubricant (magnesium stearate, *etc.*), an adhesive agent (hydroxypropylcellulose, hydroxypropylmethylcellulose, carbopol, carboxymethylcellulose, polyvinyl alcohol, xanthan gum, guar gum, *etc.),* an adhesive adjuvant (glucose, fructose, mannitol, xylitol, erythritol, maltose, trehalose, phosphate, citrate, silicate, glycine, glutamic acid, arginine, *etc.*), a stabilizing agent, a solubilizing agent (polyethylene glycol, propylene glycol, glutamic acid, aspartic acid, *etc.*), a flavoring agent (orange, strawberry, mint, lemon, vanilla, *etc.*) and the like. Also, if necessary, they may be coated with a coating agent (sucrose, gelatin, hydroxypropylcellulose, hydroxypropylmethylcellulose phthalate, *etc.*), or coated with two or more layers. In addition, if necessary, a preservative, an antioxidant, a colorant, a sweetening agent and the like generally used additive agents can also be added thereto. The buccal quick disintegration tablets are produced in accordance with a conventionally known method. For example, one, two or more active substance(s) are used as such or after making into pharmaceutical preparations by the law of the art by mixing the active substances, prepared by coating the material powder or granulated material particles with an appropriate coating agent (ethyl cellulose, hydroxypropylcellulose, hydroxypropylmethylcellulose, acrylate-methacrylate copolymer, *etc.*) and a plasticizer (polyethylene glycol, triethyl citrate, *etc.*), with an excipient (lactose, mannitol, glucose, microcrystalline cellulose, colloidal silica, starch, *etc.),* a binder (hydroxypropylcellulose, polyvinylpyrrolidone, magnesium aluminometasilicate, *etc.),* a disintegrant (starch, L-hydroxypropylcellulose, carboxymethylcellulose, croscarmellose sodium, cellulose calcium glycolate, *etc.),* a lubricant (magnesium stearate, *etc.),* a dispersing adjuvant (glucose, fructose, mannitol, xylitol, erythritol, maltose, trehalose, phosphate, citrate, silicate, glycine, glutamic acid, arginine, *etc.*), a stabilizing agent, a solubilizing agent (polyethylene glycol, propylene glycol, glutamic acid, aspartic acid, *etc.),* a flavoring agent (orange, strawberry, mint, lemon, vanilla, *etc.*) and the like. Also, if necessary, they may be coated with a coating agent (sucrose, gelatin, hydroxypropylcellulose, hydroxypropylmethylcellulose phthalate, *etc.*), or coated with two or more layers. In addition, if necessary, a preservative, an antioxidant, a colorant, a sweetening agent and the like generally used additive agents can also be added thereto.

The liquid compositions for internal use for use in oral administration includes pharmaceutically acceptable solutions, suspensions, emulsions, syrups, elixirs and the like. In such solutions, one or more active substance(s) are dissolved, suspended or emulsified in a generally used inert diluent (purified water, ethanol or a mixed liquid thereof In addition, these liquid compositions may contain humectants, suspending agents emulsifying agents, sweetening agents, flavoring agents, aromas, preservatives, buffering agents and the like.

Dosage forms of the external preparations for parenteral administration include, for example, ointments, gels, creams, fomentations, adhesive preparations, liniments, air sprays, inhalations, splays, aerosols, eye drops, nasal drops and the like. These contain one or more active substance(s) and are prepared by conventionally known methods or generally used recipes.

The ointments are prepared by conventionally known or generally used recipes. For example, these are produced and prepared by mixing or melting one or more active substance(s) in the base. The ointment base is selected from those which are conventionally known or generally used. For example, those which are selected from higher fatty acids or higher fatty acid esters (adipic acid, myristic acid, palmitic acid, stearic acid, oleic acid, adipic acid ester, myristic acid ester, palmitic acid ester, stearic acid ester, oleic acid ester, *etc.),* waxes (beeswax, spermaceti, ceresin, *etc.),* surfactants (polyoxyethylene alkyl ether phosphoric acid ester, *etc.*), higher alcohols (cetanol, stearyl alcohol, cetostearyl alcohol, *etc.*), silicon oils (dimethyl polysiloxane, *etc.*), hydrocarbons (hydrophilic petrolatum, white petrolatum, purified lanolin, liquid paraffin, *etc.*), glycols (ethylene glycol, diethylene glycol, propylene glycol, polyethylene glycol, macrogol, *etc.*), plant oils (castor oil, olive oil, sesame oil, turpentine oil, *etc.*), animal oils (minke whale oil, yolk oil, squalane, squalene, *etc.),* water, absorption accelerators and rash preventing agents are used alone or by mixing two or more thereof. In addition, these may contain a humectant, a preservative, a stabilizing agent, an antioxidant, a flavoring agent and the like.

The gels are prepared by conventionally known or generally used recipes. For example, these are prepared by melting one or more active substance(s) in a base. The gel base is selected from those which are conventionally known or generally used. For example, those which are selected from lower alcohols (ethanol, isopropyl alcohol, *etc.),* gelling agents (carboxymethylcellulose, hydroxyethylcellulose, hydroxypropylcellulose, ethylcellulose, *etc.*), neutralizing agents (triethanolamine, diisopropanolamine, *etc.*), surfactants (polyethylene glycol monostearate, *etc.),* gums, water, absorption accelerators and rash preventing agents are used alone or by mixing two or more thereof In addition, these may contain a preservative, an antioxidant, a flavoring agent and the like.

The creams are prepared by conventionally known or generally used recipes. For example, these are prepared by melting or emulsifying one or more active substance(s) in a base. The cream base is selected from those which are conventionally known or generally used. For example, those which are selected from higher fatty acid esters, lower alcohols, hydrocarbons, polyhydric alcohols (propylene glycol, 1,3-butylene glycol, *etc.),* higher alcohols (2-hexyldecanol, cetanol, *etc.*), emulsifying agents (polyoxyethylene alkyl ethers, fatty acid esters, *etc.*), water, absorption accelerators and rash preventing agents are used alone or by mixing two or more thereof In addition, these may contain a preservative,

an antioxidant, a flavoring agent and the like.

The fomentations are prepared by conventionally known or generally used recipes. For example, these are prepared by melting one or more active substance(s) in a base, and spreading and coating the kneaded material on a support. The fomentation base is selected from those which are conventionally known or generally used. For example, those which are selected from viscosity-icreasing agents (polyacrylic acid, polyvinylpyrrolidone, gum arabic, starch, gelatin, methylcellulose, *etc.*), humectants (urea, glycerol, propylene glycol, *etc.*), excipients (kaolin, zinc oxide, talc, calcium, magnesium, *etc.*), water, solution adjuvants, adhesiveness providing agents and rash preventing agents are used alone or by mixing two or more thereof In addition, these may contain a preservative, an antioxidant, a flavoring agent and the like.

The adhesive preparations are prepared by conventionally known or generally used recipes. For example, these are prepared by melting one or more active substance(s) in a base, and spreading and coating this on a support. The base for adhesive preparations is selected from those which are conventionally known or generally used. For example, those which are selected from polymer bases, oils and fats, higher fatty acids, adhesiveness providing agents and rash preventing agents are used alone or by mixing two or more thereof In addition, these may contain a preservative, an antioxidant, a flavoring agent and the like.

The liniments are prepared by conventionally known or generally used recipes. For example, these are prepared by dissolving, suspending or emulsifying one or more active substance(s) in one or two or more substance(s) selected from water, alcohols (ethanol, polyethylene glycol, *etc.*), higher fatty acids, glycerol, soap, emulsifying agents, suspending agents and the like. In addition, these may contain a preservative, an antioxidant, a flavoring agent and the like.

The air sprays, inhalations and sprays may contain a stabilizing agent such as sodium hydrogen sulfite and a buffering agent which provides tonicity, such as sodium chloride, sodium citrate, citric acid or the like tonicity agent, in addition to a generally used diluent. The production method of sprays are described in detail, for example, in US Patent 2,868,691 and US Patent 3,095,355.

As the injections for parenteral administration, all injections are included, and drip infusions are also included. For example, injections for intramuscular use, injections for subcutaneous use, injections for intradermal use, injections for intraarterial use, injections for intravenous use, injections for intraperitoneal use, injections for spinal cavity use, drip infusions for intravenous use and the like are included.

As the injections for parenteral administration, solutions, suspensions, emulsions and solid injections which are used by dissolving or suspending in a solvent when used are included. The injections are used by dissolving, suspending or emulsifying one or more active substance(s) in a solvent. As the solvent, for example, distilled water for injection, physiological saline, plant oil, propylene glycol, polyethylene glycol, alcohol such as ethanol and a combination thereof are used. These injections may further contain a stabilizing agent, a solubilizing adjuvant (glutamic acid, aspartic acid, polysorbate 80 (trade name), *etc.*), a suspending agent, an emulsifying agent, a soothing agent, buffering agent, a preservative and the like. These are prepared by sterilizing in the final process or by a sterile operation method. Alternatively, they may be used by firstly producing sterile solid preparations such as freeze-dried preparations and dissolving them in sterilized or sterile distilled water or other solvent for injection prior to their use.

Eye lotions, suspension type eye lotions, emulsion type eye lotions, before use dissolving type eye lotions and eye ointments are included in the eye drops for parenteral administration use.

These eye drops are produced in accordance with a conventionally known method. For example, one or more active substance(s) are used by dissolving, suspending or emulsifying in a solvent. As the solvent of the eye drops, for example, sterile purified water, physiological saline, other aqueous solvent or non-aqueous solvent for injection (e.g., a plant oil, *etc.*) and the like and a combination thereof are used. The eye drops may contain a tonicity agent (sodium chloride, concentrated glycerol, *etc.*), a buffering agent (sodium phosphate, sodium acetate, *etc.*), a surfactant (Polysorbate 80 (trade name), Polyoxyl stearate 40, hydrogenated polyoxyethylene castor oil, *etc.),* a stabilizing agent (sodium citrate, sodium edetate, *etc.*), a preservative (benzalkonium chloride, paraben, *etc.*) and the like, by optionally selecting them, if necessary. These are prepared by sterilizing in the final process or by a sterile operation method. Alternatively, they may be used by firstly producing sterile solid preparations such as freeze-dried preparations and dissolving them in sterilized or sterile purified water or other solvent prior to their use.

As the inhalations for parenteral administration use, aerosols, powders for inhalation use or solutions for inhalation use are included, and said solutions for inhalation use may be in such a form that they are used by dissolving or suspending in water or other appropriate medium prior to use.

These inhalations are produced in accordance with a conventionally known method.

For example, in the case of solutions for inhalation use, they are prepared by optionally selecting a preservative (benzalkonium chloride, paraben, *etc.*), a colorant, a buffering agent (sodium phosphate, sodium acetate, *etc.*), a tonicity agent (sodium chloride, concentrated glycerol, *etc.),* a viscosity-increasing agent (carboxy vinyl polymer, *etc.),* an absorption accelerating agent and the like, if necessary.

In the case of powders for inhalation use, they are prepared by optionally selecting a lubricant (stearic acid and a salt thereof, *etc.),* a binder (starch, dextrin, *etc.),* an excipient (lactose, cellulose, *etc.*), a colorant, a preservative

(benzalkonium chloride, paraben, *etc.*), an absorption accelerating agent and the like, if necessary.

When solutions for inhalation use are administered, a sprayer (atomizer or nebulizer) is generally used, and an inhalation administration device for powder preparation use is generally used when powders for inhalation is used.

As other compositions for parenteral administration use, suppositories for rectal administration, pessaries for vaginal administration and the like, which contain one mo more active substance(s) and are formulated in the usual way, are included.

Effect of the invention:

By finding that CCR5 antagonists inhibit the function of effector cells, their usefulness as preventive and/or therapeutic agents for diseases in which said cells are concerned, such as a transplant rejection, an autoimmune disease, an ischemic disease, an allergic disease, a cancer, a metastatic disease and the like, was shown.

BEST MODE FOR CARRYING OUT THE INVENTION

The following describes the present invention in detail based on reference examples, Examples, formulation examples and test examples, though the present invention is not limited thereto.

Biological Examples

The action of CCR5 antagonist to inhibit function of effector cell was confirmed by the following tests. The general operations were carried out based on basic genetic engineering techniques by preparing gene high expression cells and applying conventional methods. In addition, the measuring methods for the evaluation of the compounds of the present invention were modified for improving measuring accuracy and/or improving measuring sensitivity as described below. Details of the test methods are shown in the following.

Example 1

Migration test of human CCR5 expression cell (hCCR5-Ba/F3 cell):

(1-1) Establishment of human CCR5 expression cell

<1-1-A> Isolation of human CCR5 gene

Human placental cDNA was prepared using Marathon cDNA Amplification Kit (Clontech). PCR primers hCCR5XbaI-F1: 5'-AGCTAGTCTA GATCCGTTCC CCTACAAGAA ACTCTCC-3' (SEQ ID NO:1) and hCCR5XbaI-R1:

5'-AGCTAGTCTA GAGTGCACAA CTCTGACTGG GTCACCA-3' (SEQ ID NO:2)

were designed based on the sequence of GenBank U54994.

Using the human placental cDNA as the template and using Ex Taq (Takara), PCR reaction (2 minutes at 95°C → [30 seconds at 95°C, 45 seconds at 60°C, 1 minute at 72°C] × 35 times) was carried out. The thus amplified PCR product was subjected to 1% agarose gel electrophoresis and then purified using QIAquick Gel Extraction Kit (QIAGEN) and digested with a restriction enzyme *XbaI*. The thus digested fragment was ligated with an expression vector pEF-BOS-bsr using DNA Ligation Kit Ver. 2 (Takara) and transformed into *Escherichia coli* DH5a. By preparing this plasmid pEF-BOS-bsr[1]/hCCR5, the DNA sequence was confirmed.

<1-1-B> Culturing of Ba/F3 cell

The Ba/F3 cell was statically cultured in a $CO_2$ incubator (temperature: 37°C, $CO_2$ concentration: 5%, humidity: 100%) using RPMI-1640 medium (Gibeo BRL) containing an antibiotic preparation (Antibiotic-Antimycotic) (final concentration: penicillin G sodium (100 U/ml), streptomycin sulfate (100 μg/ml), amphotericin B (0.25 μg/ml)) (Gibco BRL), fetal bovine serum (FBS) (10%) and interleukin 3 (IL-3) (5 ng/ml) (Pepro Tech, Inc). For the culturing of an external gene stably over-expressing cell, blasticidin (Kaken Pharmaceutical) was added to the above-described medium to a final concentration of 10 μg/ml.

<1-1-C> Transduction into Ba/F3 cell

The plasmid for human CCR5 expression (pEF-BOS-bsr[1]/hCCR5) was made into linear chain by digesting with *Aat*II. The linear-chained plasmid was purified using QIA Quick PCR Purification Kit (QIAGEN) and then transferred into Ba/F3 cell by electroporation (Gene Pulser (BIO RAD) 960 μF/250 V). The cells were inoculated into a 96 well culture plate at a cell density of 1000, 100 or 10 cells/100 μl/well, and 48 hours thereafter, blasticidin was added thereto to a final concentration of 10 μg/ml to carry out cloning of blasticidin resistant strains, thereby establishing a stable over-expression clone capable of expressing the transferred external gene (hCCR5-Ba/F3 cell).

<1-1-D> CCR5 expression analysis

Human CCR5 expression strength of the clone obtained by the method described in the above-described <1-1-C> was analyzed by detecting the cells by a fluorescein isothiocyanate (FITC)-labeled anti-human CCR5 antibody (BD Pharmingen) and measuring using FACSort (trade name, manufactured by Becton Dickinson). In this case, FITC-labeled mouse IgG2aκ (BD Pharmingen) was used as the isotype control.

(1-2) Cell migration test

A medium (0.5 ml) containing MIP-1α (3 nM, Pepro Tech, Inc), MIP-1β (3 nM, Pepro Tech, Inc) or RANTES (3 nM, Pepro Tech, Inc) was added to the lower chamber of a 24 well trans-well plate, and a medium (0.05 ml) containing 2 times concentration of a solution of each drug to be tested (containing 0.02% dimethyl sulfoxide (DMSO)) and a medium (0.05 ml) in which the hCCR5-Ba/F3 cells were suspended were added to the upper chamber. The test was started by superposing the upper chamber on the lower chamber, and the cells were cultured for 3 hours in a $CO_2$ incubator (5% $CO_2$, humidity 95%) kept at 37°C. Outside bottom moiety of the upper chamber was washed out into the lower chamber using 0.5 ml of a washing buffer (phosphate buffered saline (PBS) containing sodium ethylenediaminetetraacetate (EDTA) (2 mM(and FBS (0.1%)), and the number of cells in the lower chamber was counted using a flow cytometer (Becton Dickinson).

The migration inhibition activity by the compounds of the present invention was calculated as the inhibition ratio (%) in accordance with the following formula, in which the value of well to which the medium containing DMSO but not containing the drug to be tested was added was used as the control value (A), and the value of well to which the DMSO-containing medium containing the drug to be tested was added as the value (B).

$$\text{Inhibition ratio (\%)} = [(A\text{-}B)/A] \times 100$$

By calculating the inhibition ratio of each compound of respective concentration, a value which shows 50% inhibition ratio ($IC_{50}$ value) was determined from a calibration curve.

Results:

It was found that the compounds of the present invention have the activity to inhibit migration of the human CCR5 expression cell. For example, $IC_{50}$ values of 4-[4-({(3R)-1-butyl-3-[(R)-cyclohexyl(hydroxy)methyl]-2,5-dioxo-1,4,9-triazaspiro[5.5]undec-9-yl}methyl)phenoxy]benzoic acid hydrochloride (hereinafter referred to as "Compound (a)"), 4-[4-({(3R)-1-butyl-3-[(R)-cyclohexyl(hydroxy)methyl] -2,5 -dioxo- 1, 4,9-triazaspiro [5.5]undec-9-yl}methyl)benzyl]-3-methoxybenzoic acid (hereinafter referred to as "Compound (b)") and 4-[4-({(3R)-1-butyl-3-[(R)-cyclohexyl(hydroxy)methyl]-2,5-dioxo-1,4,9-triazaspiro[5.5]undec-9-yl}methyl)phenoxy]-3-ethoxybenzoic acid hydrochloride (hereinafter referred to as "Compound (c)") were as shown in Table 1.

Table 1

| $IC_{50}$ (nM) | Chemokine | | |
|---|---|---|---|
| | MIP-1α | MIP-1β | RANTES |
| Compound (a) | 10.6 | 22.5 | 12.9 |
| Compound (b) | 9.8 | 11.1 | 12.4 |
| Compound (c) | 17.3 | 18.4 | 22.5 |

Example 2

Cell growth test of human CD8-positive memory T cell:

(2-1) Cell preparation

A heparinized blood sample was collected from a human, healthy volunteer, and peripheral blood mononuclear cell (PBMC) was isolated by a density gradient centrifugation. Specifically, a blood sample (33 ml) 2-fold diluted with physiological saline was overlaid on a medium for hemocyte separation use (10 ml) having a specific gravity of 1.077 $\pm$ 0.001 g/ml contained in a centrifugation tube (LymphoPrep tube (Nycomed Pharma)) and centrifuged (3000 $\times$ g, 10 minutes) at room temperature. The layer containing PBMC was recovered and washed twice with PBS to isolate PBMC.

<2-1-A> Preparation of CD8-positive T cell

CD8-positive T cell was isolated from PBMC by a negative selection method using Human T Cell CD8 Subset Column Kit (R & D Systems, Inc). Specifically, the cells to be removed such as B cell, CD4-positive T cell, and monocyte were labeled with antibodies by adding an antibody cocktail (1 ml) (attached to the kit) to PBMC (up to 2 $\times$ $10^8$ cells) and incubating at room temperature for 15 minutes. The cells were washed twice with a column buffer (10 ml) (attached to the kit) and suspended in said column buffer (2 ml). The cell suspension was added to Human T Cell Subset Enrichment Column (attached to the kit) which had been equilibrated in advance using the column buffer (10 ml). By eluting with the column buffer (10 ml) after incubation at room temperature for 10 minutes, the CD8-positive T cell was obtained.

<2-1-B> Preparation of CD8-positive naive T cell and CD8-positive memory T cell

CD8-positive naive T cell and CD8-positive memory T cell were obtained by fractionating the CD8-positive T cell obtained by the above-described method using VarioMACS (Miltenyl Biotec). Specifically, a marker of naive T cell, CD45RA antigen, was labeled with antibody magnetic beads by adding 20 $\mu$l of CD45RA MicroBeads (Miltenyl Biotec) to 1 $\times$ $10^7$ cells of the CD8-positive T cell, mixing them and incubating at 6 to 12°C for 15 minutes. The cells were washed once with MACS buffer (PBS containing 2 mM EDTA and 0.5% bovine serum albumin (BSA)) (20 ml) (attached to the kit) and re-suspended in MACS buffer (0.5 ml). The cell suspension was added to an MS column (Miltenyl Biotec) which had been set to VarioMACS and equilibrated with MACS buffer (0.5 ml) in advance, and further washed with MACS buffer (0.5 ml $\times$ 3 times). This washing solutions were recovered and used in the following test as the CD45RA-negative cell, namely CD8-positive memory T cell. Also, since the CD45RA-positive cell can be recovered by taking off the MS column from VarioMACS and eluting it with MACS buffer (1.5 ml), this was used in the following test as the CD8-positive naive T cell.

(2-2) CCR5 expression analysis

Expression of CCR5 in the cells fractionated by the method described in the above-described (2-1) was analyzed by detecting the fluorescein isothiocyanate (FITC)-labeled anti-human CCR5 antibody (BD Pharmingen) and RO antigen by a phycoerythrin (PE)-labeled anti-CD45RO antibody (BD Pharmingen) and measuring using FACSort (trade name, manufactured by Becton Dickinson). In this case, FITC-labeled mouse IgG2aκ (BD Pharmingen) and PE-labeled mouse IgG2a (BD Pharmingen) were used as the isotype control antibodies.

Results:

CCR5 was expressed in 63% of the CD8-positive memory T cells, and its expression in the CD8-positive naive T cells was less (about 10%).

(2-3) Growth test

The cells fractionated by the method described in the above-described (2-1) were inoculated into a 96 well plate coated with an anti-human CD3 antibody, at a cell density of 2 $\times$ $10^5$ cells/200 $\mu$l/well. The anti-human CD3 antibody-coated 96 well plate used herein was prepared by coating it with an anti-human CD3 antibody OKT-3 (0.1 $\mu$g/ml) overnight at 4°C. The cells were cultured for 48 hours in a $CO_2$ incubator (5% $CO_2$, humidity 95%) kept at 37°C. The cell growth was measured using Cell Proliferation ELISA, BrdU (Colorimetric) (Roche), using uptake of 5-bromo-2-deoxyuridine (BrdU) as the index. The operation method is shown below.

(2-4) Measurement of cell growth activity

After treating the cells with BrdU for a predetermined period of time, the plate was centrifuged (300 × g, 10 minutes), and then turned upside down and lightly shaken to remove the labeled medium. The plate was dried (for about 15 minutes) using a hair dryer. A cell fixing and DNA denaturing solution (200 μl, attached to the kit) was added to each well and left alone for 30 minutes. After removing the cell fixing and DNA denaturing solution and further removing water, a peroxidase-labeled anti-BrdU antibody (anti-BrdU-POD reaction solution) (100 μl) (attached to the kit) was added thereto and left alone at room temperature for 90 minutes. The antibody solution was discarded, each well was washed three times with PBS (200 μl), water was discarded, and then the substrate solution (100 μl) (attached to the kit) was added thereto. After carrying out the enzyme reaction for a period of 5 to 30 minutes, 1 N sulfuric acid solution (25 μl) was added thereto and stirred on a shaker. The absorbance of samples was measured by a microplate reader (SPECTRAMAX-PRO) at 450 nm (control wavelength: 690 nm).

Results:

It was found that, by the anti-CD3 antibody stimulation, the CD8-positive memory T cell shows a 10 times or more higher growth activity, calculated as the absorbance, than that of the CD8-positive naive T cell (absorbance of the CD8-positive naive T cell: 0.041, absorbance of the CD8-positive memory T cell: 0.714). In addition, it was found that the proliferation reaction is dependent on the concentration of anti-CD3 antibody. When the anti-CD3 antibody concentration was changed to 0.003, 0.01, 0.03 or 0.1 (μg/ml), absorbance of the CD8-positive memory T cell increased to 0.146, 0.229, 0.430 or 0.442.

(2-5) Chemokine production test

In the cell growth test described in the above-described (2-3), the culture medium at the time of the completion of culturing of the CD8-positive memory T cell was collected to carry out measurement of the amount of chemokine production. Measurement of the chemokine production was carried out by ELISA method, and amounts of RANTES, MIP-1α and MIP-1β were measured. Specifically, they were measured using Quantikine Human RANTES Immunoassay, Quantikine Human MIP-1α Immunoassay and Quantikine Human MIP-1β Immunoassay (all manufactured by R & D Systems, Inc).

Results:

It was found that the CD8-positive memory T cell produces RANTES, MIP-1α and MIP-1β when anti-CD3 antibody stimulation is carried out. By 48 hours of culturing, 1454 pg/ml of RANTES, 15.3 ng/ml of MIP-1α and 31.0 ng/ml of MIP-1β were produced.

(2-6) Cell growth inhibition test by compounds of the present invention

The cells fractionated by the method described in the above-described (2-1) were inoculated into a 96 well plate coated with the anti-human CD3 antibody, at a cell density of $2 \times 10^5$ cells/100 μl/well, a medium (100 μl) containing 2 times concentration solution of each drug to be tested (contains 0.2% DMSO) was added thereto, and the cells were cultured for 48 hours in a $CO_2$ incubator (5% $CO_2$, humidity 95%) kept at 37°C. The BrdU uptake activity was measured by the method described in the above-described (2-4).

The cell growth inhibition activity of human CD8-positive memory T cell by the compounds of the inhibition was calculated as the inhibition ratio (%) in accordance with the following formula, in which the value of well to which the medium containing DMSO but not containing the drug to be tested was added was used as the control value (A), and the value of well to which the DMSO-containing medium containing the drug to be tested was added as the value (B).

$$\text{Inhibition ratio (\%)} = [(A-B)/A] \times 100$$

Results:

It was found that Compound (a), Compound (b) and Compound (c) concentration-dependently inhibit cell growth of CD8-positive memory T cell by anti-CD3 antibody stimulation. Each drug to be tested at a concentration of 0.01, 0.1, 1 or 10 μM inhibited 16.1%, 26.2%, 36,3% or 66.4% (inhibition ratio of Compound (a)), -10.6%, 5.6%, 18.3% or 53.6%

(inhibition ratio of Compound (b)) or 9.2%, 13.4%, 18.8% or 39.6% (inhibition ratio of Compound (c)), respectively, of the cell growth in the absence of the compounds.

Example 3

Growth test of human CD4-positive Th1 differentiation cell:

(3-1) Preparation of cells

A heparinized blood sample was collected from a human, healthy volunteer, and peripheral blood mononuclear cell (PBMC) was isolated by the density gradient centrifugation described in the above-described (2-1). CD4-positive T cell was isolated from PBMC by a negative selection method using Human T Cell CD4 Subset Column Kit (R & D Systems, Inc). Specifically, the cells to be removed such as B cell, CD8-positive T cell, monocyte and the like were labeled with antibodies by adding an antibody cocktail (1 ml) (attached to the kit) to PBMC (up to $2 \times 10^8$ cells) and incubating at room temperature for 15 minutes. The cells were washed twice with a column buffer (10 ml) (attached to the kit) and suspended in the column buffer (2 ml). The cell suspension was added to Human T Cell Subset Enrichment Column (attached to the kit) which had been equilibrated in advance using the column buffer (10 ml). By eluting with the column buffer (10 ml) after incubation at room temperature for 10 minutes, the CD4-positive T cell was obtained.

The CD4-positive T cell obtained by the above-described method was inoculated into wells coated with the anti-CD3 antibody (2 μg/ml) and cultured for 4 days in a $CO_2$ incubator (5% $CO_2$, humidity 95%) kept at 37°C, in the presence of interleukin 12 (IL-12) (5 ng/ml) (BD Pharmingen) and anti-interleukin 4 (IL-4) antibody (1 μg/ml) (BD Pharmingen). The anti-human CD3 antibody-coated 24 well plate used herein was prepared by coating it with an anti-human CD3 antibody OKT-3 (0.1 μg/ml) overnight at 4°C. Thereafter, the cells were transferred into wells uncoated with the anti-CD3 antibody and cultured for 3 days in the $CO_2$ incubator (5% $CO_2$, humidity 95%) kept at 37°C, thereby preparing the Th1 cell under resting state.

(3-2) CCR5 expression analysis

Expression of CCR5 in the Th1 cell prepared by the method described in the above-described (3-1) and expression of RO antigen as a cell surface marker of memory T cell were analyzed using the method described in the above-described (2-2).

Results:

It was found that 95.5% of Th1 cells are positive for the RO antigen which is a cell surface marker of memory T cell, and CCR5 is expressed in 45.5% thereof

(3-3) Growth test

Using the human CD4-positive Th1 differentiation cell prepared by the method described in the above-described (3-1), the growth test described in the above-described (2-3) was carried out.

Results:

It was found that the human CD4-positive Th1 differentiation cell shows the proliferation reaction dependently on the concentration of anti-CD3 antibody. When the anti-CD3 antibody concentration was changed to 0.01, 0.03 or 0.1 (μg/ml), absorbance of the CD8-positive memory T cell increased to 0.47, 0.76 or 1.58.

(3-4) Cell growth inhibition test by compounds of the present invention

Using the human CD4-positive Th1 differentiation cell prepared by the method described in the above-described (3-1), the cell growth inhibition test by compounds of the present invention described in the above-described (2-6) was carried out.

Results:

It was found that Compound (a), Compound (b) and Compound (c) concentration-dependently inhibit cell growth of CD4-positive Th1 differentiation cell by anti-CD3 antibody stimulation. Each drug to be tested at a concentration of

0.1, or 10 μM inhibited 23.0% or 27.0% (inhibition ratio of Compound (a)), -2.3% or 21.8% (inhibition ratio of Compound (b)) or 48.3% or 47.7% (inhibition ratio of Compound (c)), respectively, of the cell growth in the absence of the compounds.

Example 4

Inhibition of allo-reactive growth (class I-MLR) of human CD8-positive T cell:

(4-1) Preparation of cells

A heparinized blood sample was collected from a human, healthy volunteer, and peripheral blood mononuclear cell (PBMC) was isolated by the density gradient centrifugation described in the above-described (2-1). CD8-positive T cell was isolated from this PBMC by the negative selection method described in the above-described (2-1).

CD14-positive cell was obtained by fractionating the PBMC obtained by the above-described method using VarioMACS (Miltenyl Biotec). Specifically, CD14 antigen was labeled with antibody magnetic beads by adding 20 μl of CD14 MicroBeads (Miltenyl Biotec) to $1 \times 10^7$ cells of the PBMC, mixing them and incubating at 6 to 12°C for 15 minutes. The cells were washed once with an MACS buffer (PBS containing 2 mM EDTA and 0.5% bovine serum albumin (BSA)) (20 ml) (attached to the kit) and re-suspended in the MACS buffer (2 ml). The cell suspension was added to an LS column (Miltenyl Biotec) which had been set to the VarioMACS and equilibrated with the MACS buffer (3 ml) in advance, and further washed with the MACS buffer (3 ml $\times$ 3 times). The CD14-positive cell was obtained by taking off the LS column from VarioMACS and eluting it with MACS buffer (10 ml).

The CD14-positive cells obtained in this manner were suspended at a cell density of $1 \times 10^6$ cells/ml in a culture medium for dendritic cell use (RPMI-1640 medium containing FBS (10%) (Gibco BRL), IL-4 (0.3 μg/ml) (Ono Pharmaceutical), granulocyte macrophage colony-stimulating factor (GM-CSF) (50 ng/ml) (Pepro Tech, Inc), and antibiotic preparation (Antibiotic-Antimycotic) (final concentration: penicillin G sodium (100 U/ml), streptomycin sulfate (100 μg/ml), amphotericin B (0.25 μg/ml)) (Gibco BRL)) and cultured for 5 days in a $CO_2$ incubator (5% $CO_2$ humidity 95%) which was kept at 37°C. Thereafter, a lipopolysaccharide (LPS) (Sigma) was added thereto to a final concentration of 1 μg/ml, and the culturing was further continued for 2 days. After completion of the culturing, the recovered cells were used as dendritic cells in the following test.

(4-2) Activation marker expression analysis

Expression of activation markers (HLA-DR, HLA-ABC, CD11c, CD83, CD80 and CD86) in the dendritic cell isolated by the method described in the above-described (4-1) was detected by fluorescence-labeled antibodies (antibodies of HLA-DR, HLA-ABC, CD83, CD80 and CD86 are FITC-labeled, and antibody of CD11c is PE-labeled) (BD Pharmingen) and analyzed using FACSort (trade name, manufactured by Becton Dickinson). In this case, FITC-labeled mouse IgG2b, FITC-labeled mouse IgG1, PE-labeled mouse IgG1 and FITC-labeled mouse IgM (BD Pharmingen) were used as the isotype control antibodies.

Results:

It was found that HLA-DR, HLA-ABC, CD11c, CD83, CD80 and CD86 are expressed in the prepared dendritic cell.

(4-3) Allo-reaction growth test

An allo-reaction growth test (class I-MLR) was carried out in the following manner, using the human CD8-positive T cell and dendritic cell isolated by the method described in the above-described (4-1) as a responder and a stimulator, respectively.

In the presence or absence of a compound to be tested, the CD8-positive T cell was inoculated into a 96 well plate, by fixing the number of cells thereof ($1 \times 10^5$ cells/well), together with the dendritic cell such that the stimulator/responder ratio of the number of cells thereof became from 1/10 to 1/100. The cells were cultured for 5 days in a $CO_2$ incubator (5% $CO_2$, humidity 95%) which was kept at 37°C, and then subjected to the same operation of the cell growth activity measurement described in the above-described (2-4).

Results:

It was found that Compound (a) and Compound (b) have the effect to inhibit cell growth in the allo-reaction growth test. Each drug to be tested at a concentration of 0.1, 1 or 10 μM inhibited 15.8%, 14.7% or 84.0% (inhibition

ratio of Compound (a)) or 7.8%, 17.6% or 44.1% (inhibition ratio of Compound (b)), respectively, of the allo-reaction growth in the absence of the compounds.

Formulation Example 1

The following respective components were admixed in a conventional method, punched out to give 100 tablets each containing 50 mg of the active ingredient.

| | |
|---|---|
| 4-[4-({(3R)-1-Butyl-3-[(R)-cyclohexyl(hydroxy)methyl]-2,5-dioxo-1,4,9-triazaspiro[5.5]undec-9-yl}methyl)benzyl]-3-methoxybenzoic acid hydrochloride | 5.0 g |
| Carboxymethylcellulose calcium (disintegrator) | 0.2 g |
| Magnesium stearate (lubricant) | 0.1 g |
| Microcrystalline cellulose | 4.7 g |

Formulation Example 2:

The following respective components were admixed in a conventional method. The solution was sterilized in a conventional method, filled in 5 ml portions into ampoules and freeze-dried in a conventional method to give 100 ampoules each containing 20 mg of the active ingredient.

| | |
|---|---|
| 4-[4-({(3R)-1-Butyl-3-[(R)-cyclohexyl(hydroxy)methyl]-2,5-dioxo-1,4,9-triazaspiro[5.5]undec-9-yl}methyl)benzyl]-3-methoxybenzoic acid hydrochloride | 2.0 g |
| Mannitol | 20 g |
| Distilled water | 500 ml |

INDUSTRIAL APPLICABILITY

Since the CCR5 antagonists inhibit functions of effector cells, they are useful for the prevention and/or treatment of, for example, a transplant rejection (e.g., rejection of a solid organ graft, rejection of islet cell transplantation in diabetes mellitus, graft-versus-host disease (GVHD), *etc.),* an autoimmune disease (e.g., arthritis, rheumatoid arthritis, multiple sclerosis, ulcerative colitis, *etc.),* an allergic disease (e.g., asthma, *etc.),* an ischemic disease (e.g., ischemia-reperfusion injury, *etc.*) and the like in animals including human, particularly in human.

## Claims

1. A function inhibitor of an effector cell, which comprises a CCR5 antagonist.

2. The function inhibitor of an effector cell according to claim 1, wherein the function is cell migration, cell proliferation or cell activation.

3. The function inhibitor of an effector cell according to claim 1, wherein the effector cell is a CCR5-positive effector cell.

4. The function inhibitor of an effector cell according to claim 1, which is an agent for prevention and/or treatment of a disease caused by effector cell function.

5. The function inhibitor of an effector cell according to claim 1, which is an agent for prevention and/or treatment of a T cell-mediated disease.

6. The function inhibitor of an effector cell according to claim 1, which is an agent for prevention and/or treatment of a myeloid cell-mediated disease.

7. The function inhibitor of an effector cell according to claim 5, wherein the T cell-mediated disease is transplant rejection, autoimmune disease, allergic disease or ischemic disease.

8. The function inhibitor of an effector cell according to claim 6, wherein the myeloid cell-mediated disease is cancer or cancer metastasis.

9. The function inhibitor of an effector cell according to claim 1, wherein the CCR5 antagonist is a non-peptide substance.

10. The function inhibitor of an effector cell according to claim 1, wherein the CCR5 antagonist is a compound of formula (I)

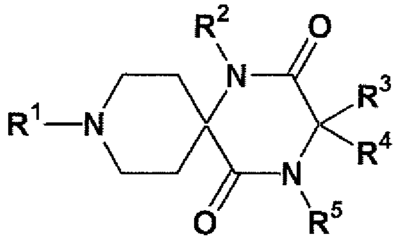

(I)

wherein $R^1$ represents (1) a hydrogen atom, (2) C1-18 alkyl, (3) C2-18 alkenyl, (4) C2-18 alkynyl, (5) $-COR^6$, (6) $-CONR^7R^8$, (7) $-COOR^9$, (8) $-SO_2R^{10}$, (9) $-COCOOR^{11}$, (10) $-CONR^{12}COR^{13}$, (11) Cyc1 or (12) C1-18 alkyl, C2-18 alkenyl or C2-18 alkynyl substituted with 1-5 substituent(s) selected from (a) halogen, (b) $-CONR^7R^8$, (c) $-COOR^9$, (d) $-OR^{14}$, (e) $-SR^{15}$, (f) $-NR^{16}R^{17}$, (g) $-NR^{18}COR^{19}$, (h) $-SO_2NR^{20}R^{21}$, (i) $-OCOR^{22}$, (j) $-NR^{23}SO_2R^{24}$, (k) $-NR^{25}COOR^{26}$, (1) $-NR^{27}CONR^{28}R^{29}$, (m) Cycl, (n) keto and (o) $-N(SO_2R^{24})_2$;
$R^6$-$R^9$, $R^{11}$-$R^{21}$, $R^{23}$, $R^{25}$ and $R^{27}$-$R^{29}$ each independently represents (1) a hydrogen atom, (2) C1-8 alkyl, (3) C2-8 alkenyl, (4) C2-8 alkynyl, (5) Cyc1 or (6) C1-8 alkyl, C2-8 alkenyl or C2-8 alkynyl substituted with 1-5 substituent(s) selected from (a) Cyc1, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}COR^{38}$, (i) $-NR^{39}SO_2R^{40}$ and (j) $-N(SO_2R^{40})_2$, or
$R^7$ and $R^8$, $R^{20}$ and $R^{21}$, or $R^{28}$ and $R^{29}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{195}$-(C2-6 alkylene)-, wherein $R^{195}$ is a hydrogen atom, C1-8 alkyl, phenyl, or C1-8 alkyl substituted with phenyl;
$R^{10}$, $R^{22}$, $R^{24}$ and $R^{26}$ each independently represents (1) C1-8 alkyl, (2) C2-8 alkenyl, (3) C2-8 alkynyl, (4) Cycl or (5) C1-8 alkyl, C2-8 alkenyl or C2-8 alkynyl substituted with 1-5 substituent(s) selected from (a) Cycl, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}COR^{38}$, (i) $-NR^{39}SO_2R^{40}$ and (j) $-N(SO_2R^{40})_2$;
$R^{30}$-$R^{37}$ and $R^{39}$ each independently represents a hydrogen atom, C1-8 alkyl, Cycl or C1-8 alkyl substituted with Cycl, or
$R^{35}$ and $R^{36}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{196}$-(C2-6 alkylene)-, wherein $R^{196}$ represents a

hydrogen atom, C1-8 alkyl, phenyl or C1-8 alkyl substituted with phenyl;

$R^{38}$ and $R^{40}$ each independently represents C1-8 alkyl, Cycl or C1-8 alkyl substituted with Cycl;

Cycl represents a C3-15 mono-, bi- or tri-(fused or spiro)carbocyclic ring or a 3-15 membered mono-, bi- or tri-(fused or spiro)cyclic hetero ring containing 1-4 nitrogen atom(s), 1-3 oxygen atom(s) and/or 1-3 sulfur atom(s), and Cycl may be substituted with 1-5 of $R^{51}$;

$R^{51}$ represents (1) C1-8 alkyl, (2) C2-8 alkenyl, (3) C2-8 alkynyl, (4) halogen, (5) nitro, (6) trifluoromethyl, (7) trifluoromethoxy, (8) nitrile, (9) keto, (10) Cyc2, (11) $-OR^{52}$, (12) $-SR^{53}$, (13) $-NR^{54}R^{55}$, (14) $-COOR^{56}$, (15) $-CONR^{57}R^{58}$, (16) $-NR^{59}COR^{60}$, (17) $-SO_2NR^{61}R^{62}$, (18) $-OCOR^{63}$, (19) $-NR^{64}SO_2R^{65}$, (20) $-NR^{66}COOR^{67}$, (21) $-NR^{68}CONR^{69}R^{70}$, (22) $-B(OR^{71})_2$, (23) $-SO_2R^{72}$, (24) $-N(SO_2R^{72})_2$, or (25) C1-8 alkyl, C2-8 alkenyl or C2-8 alkynyl substituted with 1-5 substituent(s) selected from (a) halogen, (b) Cyc2, (c) $-OR^{52}$, (d) $-SR^{53}$, (e) $-NR^{54}R^{55}$, (f) $-COOR^{56}$, (g) $-CONR^{57}R^{58}$, (h) $-NR^{59}COR^{60}$, (i) $-SO_2NR^{61}R^{62}$, (j) $-OCOR^{63}$, (k) $-NR^{64}SO_2R^{65}$, (l) $-NR^{66}COOR^{67}$, (m) $-NR^{68}CONR^{69}R^{70}$, (n) $-B(OR^{71})_2$, (o) $-SO_2R^{72}$ and (p) $-N(SO_2R^{72})_2$;

$R^{52}$-$R^{62}$, $R^{64}$, $R^{66}$ and $R^{68}$-$R^{71}$ each independently represents (1) a hydrogen atom, (2) C1-8 alkyl, (3) C2-8 alkenyl, (4) C2-8 alkynyl, (5) Cyc2 or (6) C1-8 alkyl, C2-8 alkenyl or C2-8 alkynyl substituted with Cyc2, $-OR^{73}$, $-COOR^{74}$ or $-NR^{75}R^{76}$, or

$R^{57}$ and $R^{58}$, $R^{61}$ and $R^{62}$, or $R^{69}$ and $R^{70}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{197}$-(C2-6 alkylene)-, wherein $R^{197}$ represents a hydrogen atom, C1-8 alkyl, phenyl or C1-8 alkyl substituted with phenyl;

$R^{63}$, $R^{65}$, $R^{67}$ and $R^{72}$ each independently represents (1) C1-8 alkyl, (2) C2-8 alkenyl, (3) C2-8 alkynyl, (4) Cyc2 or (5) C1-8 alkyl, C2-8 alkenyl or C2-8 alkynyl substituted with Cyc2, $-OR^{73}$, $-COOR^{74}$ or $-NR^{75}R^{76}$;

$R^{73}$-$R^{76}$ each independently represents a hydrogen atom, C1-8 alkyl, Cyc2 or C1-8 alkyl substituted with Cyc2;

Cyc2 has the same meaning as Cyc1, and Cyc2 may be substituted with 1-5 of $R^{77}$;

$R^{77}$ represents (1) C1-8 alkyl, (2) halogen, (3) nitro, (4) trifluoromethyl, (5) trifluoromethoxy, (6) nitrile, (7) $-OR^{78}$, (8) $-NR^{79}R^{80}$, (9) $-COOR^{81}$, (10) $-SR^{82}$, (11) $-CONR^{83}R^{84}$, (12) C2-8 alkenyl, (13) C2-8 alkynyl, (14) keto, (15) Cyc6, (16) $-NR^{161}COR^{162}$, (17) $-SO_2NR^{163}R^{164}$, (18) $-OCOR^{165}$, (19) $-NR^{166}SO_2R^{167}$, (20) $-NR^{168}COOR^{169}$, (21) $-NR^{170}CONR^{171}R^{172}$, (22) $-SO_2R^{173}$, (23) $-N(SO_2R^{167})_2$ or (24) C1-8 alkyl, C2-8 alkenyl or C2-8 alkynyl substituted with 1-5 substituent(s) selected from (a) halogen, (b) $-OR^{78}$, (c) $-NR^{79}R^{80}$, (d) $-COOR^{81}$, (e) $-SR^{82}$, (f) $-CONR^{83}R^{84}$, (g) keto, (h) Cyc6, (i) $-NR^{161}COR^{162}$, (j) $-SO_2NR^{163}R^{164}$, (k) $-OCOR^{165}$, (1) $-NR^{166}SO_2R^{167}$, (m) $-NR^{168}COOR^{169}$, (n) $-NR^{170}CONR^{171}R^{172}$, (o) $-SO_2R^{173}$, and (p) $-N(SO_2R^{167})_2$;

$R^{78}$-$R^{84}$, $R^{161}$-$R^{164}$, $R^{166}$, $R^{168}$ and $R^{170}$-$R^{172}$ each independently represents (a) a hydrogen atom, (b) C1-8 alkyl, (c) C2-8 alkenyl, (d) C2-8 alkynyl, (e) Cyc6, (f) C1-8 alkyl, C2-8 alkenyl or C2-8 alkynyl substituted with Cyc6, $-OR^{174}$, $-COOR^{175}$, $-NR^{176}R^{177}$ or $-CONR^{178}R^{179}$, or

$R^{83}$ and $R^{84}$ $R^{163}$ and $R^{164}$, or $R^{171}$ and $R^{172}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{198}$-(C2-6 alkylene)-, wherein $R^{198}$ represents a hydrogen atom, C1-8 alkyl, phenyl or C1-8 alkyl substituted with phenyl;

$R^{165}$, $R^{167}$, $R^{169}$ and $R^{173}$ each independently represents (a) C1-8 alkyl, (b) C2-8 alkenyl, (c) C2-8 alkynyl, (d) Cyc6 or (e) C1-8 alkyl, C2-8 alkenyl or C2-8 alkynyl substituted with Cyc6, $-OR^{174}$, $-COOR^{175}$, $-NR^{176}R^{177}$ or $-CONR^{178}R^{179}$;

$R^{174}$-$R^{177}$ each independently represents (1) a hydrogen atom, (2) C1-8 alkyl, (3) Cyc6 or (4) C1-8 alkyl substituted with Cyc6, or

$R^{178}$ and $R^{179}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{199}$-(C2-6 alkylene)-, wherein $R^{199}$ represents a hydrogen atom, C1-8 alkyl, phenyl or C1-8 alkyl substituted with phenyl;

Cyc6 represents a C3-8 mono-carbocyclic ring or a 3-8 membered monocyclic hetero ring containing 1-4 nitrogen atom(s), 1-2 oxygen atom(s) and/or 1-2 sulfur atom(s), with the proviso that, Cyc6 may be substituted with 1-5 of $R^{180}$;

$R^{180}$ represents (1) C1-8 alkyl, (2) halogen, (3) nitro, (4) trifluoromethyl, (5) trifluoromethoxy, (6) nitrile, (7) $-OR^{181}$, (8) $-NR^{182}R^{183}$, (9) $-COOR^{184}$, (10) $-SR^{185}$ or (11) $-CONR^{186}R^{187}$;

$R^{181}$-$R^{187}$ each independently represents (1) a hydrogen atom, (2) C1-8 alkyl, (3) phenyl or (4) C1-8 alkyl substituted with phenyl, or

$R^{182}$ and $R^{183}$ or $R^{186}$ and $R^{187}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{200}$-(C2-6 alkylene)-, wherein $R^{200}$ represents a hydrogen atom, C1-8 alkyl, phenyl, C1-8 alkyl substituted with phenyl;

$R^2$ represents (1) a hydrogen atom, (2) C1-8 alkyl, (3) C2-8 alkenyl, (4) C2-8 alkynyl, (5) $-OR^{90}$, (6) Cyc3 or (7) C1-8 alkyl, C2-8 alkenyl or C2-8 alkynyl substituted with 1-5 substituent(s) selected from (a) halogen, (b) $-OR^{90}$, (c) $-SR^{91}$, (d) $-NR^{92}R^{93}$, (e) $-COOR^{94}$, (f) $-CONR^{95}R^{96}$, (g) $-NR^{97}COR^{98}$, (h) $-SO_2NR^{99}R^{100}$, (i) $-OCOR^{101}$, (j) $-NR^{102}SO_2R^{103}$, (k) $-NR^{104}COOR^{105}$, (1) $-NR^{106}CONR^{107}R^{108}$, (m) Cyc3, (n) keto and (o) $-N(SO_2R^{103})_2$;

$R^{90}$-$R^{100}$, $R^{102}$, $R^{104}$ and $R^{106}$-$R^{108}$ each independently represents (1) a hydrogen atom, (2) C1-8 alkyl, (3)

C2-8 alkenyl, (4) C2-8 alkynyl, (5) Cyc3 or (6) C1-8 alkyl, C2-8 alkenyl or C2-8 alkynyl substituted with Cyc3, or $R^{95}$ and $R^{96}$, $R^{99}$ and $R^{100}$, or $R^{107}$ and $R^{108}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{201}$-(C2-6 alkylene)-, wherein $R^{201}$ is a hydrogen atom, C1-8 alkyl, phenyl or C1-8 alkyl substituted with phenyl;
$R^{101}$, $R^{103}$ and $R^{105}$ are each independently (1) C1-8 alkyl, (2) C2-8 alkenyl, (3) C2-8 alkynyl or (4) Cyc3, or C1-8 alkyl, C2-8 alkenyl or C2-8 alkynyl substituted with Cyc3;
Cyc3 has the same meaning as Cyc1, and Cyc3 may be substituted with 1-5 of $R^{109}$;
$R^{109}$ has the same meaning as $R^{51}$;
$R^3$ and $R^4$ each independently represents (1) a hydrogen atom, (2) C1-8 alkyl, (3) C2-8 alkenyl, (4) C2-8 alkynyl, (5) $-COOR^{120}$, (6) $-CONR^{121}R^{122}$, (7) Cyc4 or (8) C1-8 alkyl, C2-8 alkenyl or C2-8 alkynyl substituted with 1-5 substituent(s) selected from (a) halogen, (b) nitrile, (c) Cyc4, (d) $-COOR^{120}$, (e) $-CONR^{121}R^{122}$, (f) $-OR^{123}$, (g) $-SR^{124}$, (h) $-NR^{125}R^{126}$, (i) $-NR^{127}COR^{128}$, (j) $-SO_2NR^{129}R^{130}$, (k) $-OCOR^{131}$, (l) $-NR^{132}SO_2R^{133}$, (m) $-NR^{134}COOR^{135}$, (n) $-NR^{136}CONR^{137}R^{138}$, (o) $-S-SR^{139}$, (p) $-NHC(=NH)NHR^{140}$, (q) keto, (r) $-NR^{145}CONR^{146}COR^{147}$ and (s) $-N(SO_2R^{133})_2$;
$R^{120}$-$R^{130}$, $R^{132}$, $R^{134}$, $R^{136}$-$R^{138}$, $R^{145}$ and $R^{146}$ each independently represents (1) a hydrogen atom, (2) C1-8 alkyl, (3) C2-8 alkenyl, (4) C2-8 alkynyl, (5) Cyc4 or (6) C1-8 alkyl, C2-8 alkenyl or C2-8 alkynyl substituted with Cyc4, halogen, $-OR^{148}$, $-SR^{149}$, $-COOR^{150}$ or $-NHCOR^{141}$, or
$R^{121}$ and $R^{122}$, $R^{129}$ and $R^{130}$, or $R^{137}$ and $R^{138}$ are taken together to represent (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{201}$-(C2-6 alkylene)-, wherein $R^{201}$ represents a hydrogen atom, C1-8 alkyl, phenyl, C1-8 alkyl substituted with phenyl;
$R^{131}$, $R^{133}$, $R^{135}$, $R^{139}$ and $R^{147}$ each independently represents (1) C1-8 alkyl, (2) C2-8 alkenyl, (3) C2-8 alkynyl, (4) Cyc4 or (5) C1-8 alkyl, C2-8 alkenyl or C2-8 alkynyl substituted with Cyc4, halogen, $-OR^{148}$, $-SR^{149}$, $-COOR^{150}$ or $-NHCOR^{141}$;
$R^{140}$ represents a hydrogen atom, $COOR^{142}$ or $-SO_2R^{143}$;
$R^{141}$-$R^{143}$ each independently represents (1) C1-8 alkyl, (2) C2-8 alkenyl, (3) C2-8 alkynyl, (4) Cyc4 or (5) C1-8 alkyl, C2-8 alkenyl or C2-8 alkynyl substituted with Cyc4;
$R^{148}$-$R^{150}$ each independently represents (1) a hydrogen atom, (2) C1-8 alkyl, (3) C2-8 alkenyl, (4) C2-8 alkynyl, (5) Cyc4 or (6) C1-8 alkyl, C2-8 alkenyl or C2-8 alkynyl substituted with Cyc4;
Cyc4 has the same meaning as Cyc1, and Cyc4 may be substituted with 1-5 of $R^{144}$;
$R^{144}$ has the same meaning as $R^{51}$, or
$R^3$ and $R^4$ are taken together to represent

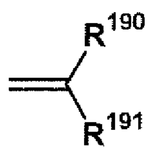

wherein $R^{190}$ and $R^{191}$ each independently represents (1) a hydrogen atom, (2) C1-8 alkyl, (3) C2-8 alkenyl, (4) C2-8 alkynyl, (5) $-COOR^{120}$, (6) $-CONR^{121}R^{122}$, (7) Cyc4 or (8) C1-8 alkyl, C2-8 alkenyl or C2-8 alkynyl substituted with 1-5 substituent(s) selected from (a) halogen, (b) nitrile, (c) Cyc4, (d) $-COOR^{120}$, (e) $-CONR^{121}R^{122}$ , (f) $-OR^{123}$, (g) $-SR^{124}$, (h) $-NR^{125}R^{126}$, (i) $-NR^{127}COR^{128}$, (j) $-SO_2NR^{129}R^{130}$, (k) $-OCOR^{131}$, (l) $-NR^{132}SO_2R^{133}$, (m) $-NR^{134}COOR^{135}$, (n) $-NR^{136}CONR^{137}R^{138}$, (o) $-S-SR^{139}$, (p) $-NHC(=NH)NHR^{140}$, (q) keto, (r) $-NR^{145}CONR^{146}COR^{147}$ and (s) $-N(SO_2R^{133})_2$;
$R^{120}$-$R^{140}$ and $R^{145}$-$R^{147}$ have the same meanings as described above;
$R^5$ represents (1) a hydrogen atom, (2) C1-8 alkyl, (3) Cyc5 or (4) C1-8 alkyl substituted with Cyc5;
Cyc5 has the same meaning as Cyc1, and Cyc5 may be substituted with 1-5 of $R^{150}$; of $R^{150}$;
$R^{150}$ has the same meaning as $R^{51}$;
an N-oxide thereof, a salt thereof, or a prodrug thereof.

**11.** A medicament which comprises a function inhibitor of an effector cell comprising a CCR5 antagonist, in combination with one, two or more immunosuppressive drug(s).

**12.** The medicament according to claim 11, wherein the one, two or more immunosuppressive drug(s) are selected from the group of tacrolimus, cyclosporine, sirolimus, corticosteroid, azathioprine, mycophenolate mofetil, FTY-720 and cyclophosphamide.

**13.** A method for prevention and/or treatment of a disease caused by effector cell function, which comprises administering to a mammal an effective amount of a compound of formula (I)

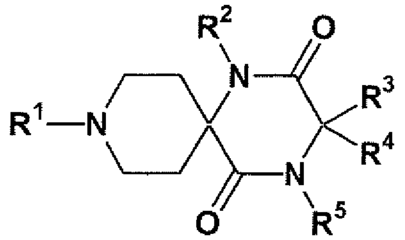

(I)

wherein all symbols have the same meanings as those defined in claim 10, an N-oxide thereof, a salt thereof, or a prodrug thereof

**14.** Use of a compound of formula (I)

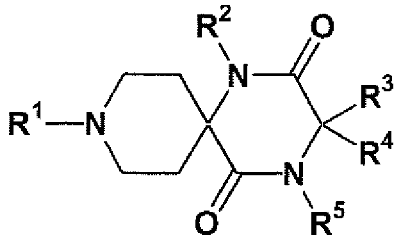

(I)

wherein all symbols have the same meanings as those defined in claim 10, an N-oxide thereof, a salt thereof, or a prodrug thereof for the manufacture of an agent for prevention and/or treatment of a disease caused by effector cell function.

**INTERNATIONAL SEARCH REPORT**

International application No. PCT/JP2004/006197

A. CLASSIFICATION OF SUBJECT MATTER
Int.Cl[7] A61K45/00, 45/06, 31/499, A61P37/06, 37/08, 9/10, 35/00, C07D487/10

According to International Patent Classification (IPC) or to both national classification and IPC

B. FIELDS SEARCHED

Minimum documentation searched (classification system followed by classification symbols)
Int.Cl[7] A61K45/00, 45/06, 31/499, A61P37/06, 37/08, 9/10, 35/00, C07D487/10

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)
CAPLUS(STN), MEDLINE(STN), BIOSIS(STN), REGISTRY(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| X | WO 02/74770 A1 (Ono Pharmaceutical Co., Ltd.), 26 September, 2002 (26.09.02), Full text; particularly, pages 75, 77 & EP 1378510 A1 | 1-12,14 |
| Y | GAO, P. et al., The unique target specificity of a non-peptide chemokine receptor selective blockade of two Th1 chemokine receptors CCR antagonist, J. Leukocyte Biol., 2003 February, Vol.73, No.2, pages 273 to 280, full text, particularly, page 273, Abstract | 1-12,14 |

☒ Further documents are listed in the continuation of Box C. ☐ See patent family annex.

* Special categories of cited documents:
"A" document defining the general state of the art which is notconsidered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>24 May, 2004 (24.05.04) | Date of mailing of the international search report<br>31 August, 2004 (31.08.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/006197

C (Continuation). DOCUMENTS CONSIDERED TO BE RELEVANT

| Category* | Citation of document, with indication, where appropriate, of the relevant passages | Relevant to claim No. |
|---|---|---|
| Y | TAKAMI, Shinya et al., TAK-779, a nonpeptide CC chemokine receptor antagonist, protects the brain against focal cerebral ischemia in mice, Journal of cerebral Blood Flow and Metabolism, 2002, Vol.27, No.7, pages 780 to 784, full text, particularly, page 780, Abstract | 1-12,14 |

Form PCT/ISA/210 (continuation of second sheet) (January 2004)

**INTERNATIONAL SEARCH REPORT**

International application No.
PCT/JP2004/006197

**Box No. II Observations where certain claims were found unsearchable (Continuation of item 2 of first sheet)**

This international search report has not been established in respect of certain claims under Article 17(2)(a) for the following reasons:

1. ☒ Claim No.: 13
because they relate to subject matter not required to be searched by this Authority, namely:
Claim 13 relates to methods for treatment of a human body by therapy.

2. ☐ Claims Nos.:
because they relate to parts of the international application that do not comply with the prescribed requirements to such an
extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

See extra sheet.

1. ☐ As all required additional search fees were timely paid by the applicant, this international search report covers all searchable
claims.

2. ☐ As all searchable claims could be searched without effort justifying an additional fee, this Authority did not invite payment of any additional fee.

3. ☐ As only some of the required additional search fees were timely paid by the applicant, this international search report covers
only those claims for which fees were paid, specifically claims Nos.:

4. ☒ No required additional search fees were timely paid by the applicant. Consequently, this international search report is restricted to the invention first mentioned in the claims; it is covered by claims Nos.: 1-12 and 14 specifically parts thereof wherein the CCR5-antagonist is a compound represented by the formula (I).

**Remark on Protest** ☐ The additional search fees were accompanied by the applicant's protest.
☐ No protest accompanied the payment of additional search fees.

Form PCT/ISA/210 (continuation of first sheet (2)) (January 2004)

**INTERNATIONAL SEARCH REPORT**

International application No.
PCT/JP2004/006197

Continuation of Box No.III of continuation of first sheet(2)

Providing that the invention claimed in claim 1 of this application, "effector cell function inhibitor comprised of CCR5-antagonist", is the specified invention, it appears that the matter common to the invention claimed in claim 10 "effector cell function inhibitor according to claim 1 wherein the CCR5-antagonist is a compound represented by the general formula (I)" is the "effector cell function inhibitor comprised of CCR5-antagonist".

However, this feature is not a novel matter and cannot be recognized as a principal element of invention because that "the CCR5-antagonist inhibits the ambulato of effector cells" is publicly known as described in the following reference.

Further, it does not appear that both have technical common task having been unsolved until the time of filing of this application.

Therefore, the invention of claims 1-9, 11 and 12 and the invention of claims 10 and 14 comprising specified compound of the general formula (I) as an active ingredient do not constitute a group of inventions linked with each other so as to form a single general inventive concept.

Reference: GAO, P et al., The unique target specificity of a nonpeptide chemokinereceptor selective blockade of two Th1 chemokine receptors CCR antagonist, J. Leukocyte Biol., 2003 Feb, Vol.73, No.2, pp273-280.